(19) **Europäisches Patentamt**
**European Patent Office**
**Office européen des brevets**

(11) **EP 1 887 079 A1**

(12) **EUROPÄISCHE PATENTANMELDUNG**

(43) Veröffentlichungstag:
**13.02.2008 Patentblatt 2008/07**

(51) Int Cl.:
*C12N 5/10* (2006.01)     *C12N 15/29* (2006.01)
*A01H 5/00* (2006.01)     *C08B 30/04* (2006.01)
*A23L 1/0522* (2006.01)   *C12N 9/12* (2006.01)
*C12N 9/10* (2006.01)

(21) Anmeldenummer: 06090134.5

(22) Anmeldetag: **09.08.2006**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(71) Anmelder: **Bayer CropScience AG**
**40789 Monheim (DE)**

(72) Erfinder: **Claus Frohberg**
**14532 Kleinmachnow (DE)**

(74) Vertreter: **Beyer, Andreas et al**
**Bayer BioScience GmbH**
**Intellectual Property Department**
**Hermannswerder 20a**
**14473 Potsdam (DE)**

(54) **Genetisch modifizierte Pflanzen, die eine Stärke mit erhöhtem Quellvermögen synthetisieren**

(57)     Die vorliegende Erfindung betrifft genetisch modifizierte Pflanzenzellen und Pflanzen, Verfahren zur Herstellung von genetisch modifizierten Pflanzenzellen und Pflanzen, die eine erhöhte Aktivität eines Proteins mit der Aktivität einer Stärkesynthase II und eine erhöhte Aktivität eines Proteins mit der Aktivität einer Glucan-Wasser-Dikinase aufweisen. Solche Pflanzen synthetisieren Stärke mit erhöhtem Heißwasser Quellvermögen. Stärken mit erhöhtem Heißwasser Quellvermögen, sowie Verfahren zu deren Herstellung sind ebenfalls Gegenstand der vorliegenden Erfindung.

Bestimmung von SS2 Aktivität in transgenen Linien

Fig. 1

EP 1 887 079 A1

**Beschreibung**

Die vorliegende Erfindung betrifft genetisch modifizierte Pflanzenzellen und Pflanzen, Verfahren zur Herstellung von genetisch modifizierten Pflanzenzellen und Pflanzen, die eine erhöhte Aktivität eines Proteins mit der Aktivität einer Stärkesynthase II und eine erhöhte Aktivität eines Proteins mit der Aktivität einer Glucan-Wasser-Dikinase aufweisen. Solche Pflanzen synthetisieren Stärke mit erhöhtem Heißwasser Quellvermögen. Stärken mit erhöhtem Heißwasser Quellvermögen, sowie Verfahren zu deren Herstellung sind ebenfalls Gegenstand der vorliegenden Erfindung.

Neben Ölen, Fetten und Proteinen stellen Polysaccharide die wesentlichen nachwachsenden Rohstoffe aus Pflanzen dar. Eine zentrale Stellung bei den Polysacchariden nimmt neben Cellulose die Stärke ein, die einer der wichtigsten Speicherstoffe in Höheren Pflanzen ist.

Weiterhin stellt Stärke einen ernährungsphysiologisch wesentlichen Bestandteil der menschlichen und tierischen Nahrung dar. Die strukturellen Merkmale der in Nahrungsmitteln enthaltenden Stärke können die funktionellen (z.B. Wasserbindungsvermögen, Quellvermögen), ernährungsphysiologischen (z.B. Verdaubarkeit, Einfluß des Nahrungsmittels auf den Glykämischen Index) oder strukturgebenden (z.B. Schnittfestigkeit, Textur, Klebrigkeit, Verarbeitbarkeit) Eigenschaften von verschiedensten Nahrungsmitteln beeinflussen. Nahrungsmittelzusammensetzungen enthalten daher häufig eine Stärke mit bestimmten strukturellen Merkmalen, die die gewünschten Eigenschaften des betreffenden Nahrungsmittels bedingen. Auch die Eigenschaften von Nahrungsmitteln, enthaltend Stärke speichernde Pflanzengewebe (z.B. Körner, Früchte, Mehle), können von der in den Pflanzengeweben enthaltenden Stärke beeinflusst werden.

Das Polysaccharid Stärke ist ein Polymer aus chemisch einheitlichen Grundbausteinen, den Glucosemolekülen. Es handelt sich dabei jedoch um ein sehr komplexes Gemisch aus unterschiedlichen Molekülformen, die sich hinsichtlich ihres Polymerisationsgrades, des Auftretens von Verzweigungen der Glucoseketten und deren Kettenlängen unterscheiden, die darüber hinaus modifiziert, z.B. phosphoryliert sein können. Daher stellt Stärke keinen einheitlichen Rohstoff dar. Man unterscheidet insbesondere die Amylose, ein im wesentlichen unverzweigtes Polymer aus alpha-1,4-glycosidisch verknüpften Glucosemolekülen, vom Amylopektin, das ein komplexes Gemisch aus unterschiedlich verzweigten Glucoseketten darstellt. Die Verzweigungen kommen dabei durch das Auftreten von zusätzlichen alpha-1,6-glycosidischen Verknüpfungen zustande. In typischen, für die industrielle Stärkeproduktion oder als Nahrungsmittel verwendeten Pflanzen, wie z.B. Mais, Reis, Weizen oder Kartoffel, besteht die synthetisierte Stärke zu ca. 20% - 25% aus Amylose und zu ca. 70% - 75% aus Amylopektin.

Die funktionellen, ernährungsphysiologischen oder strukturgebenden Eigenschaften der Stärke, wie z.B. die Löslichkeit, das Retrogradationsverhalten, das Wasserbindevermögen, die Filmbildungseigenschaften, die Viskosität, die Verkleisterungseigenschaften, die Gefrier-Tau-Stabilität, die Säurestabilität, die Gelfestigkeit, das Quellvermögen, die Verdaubarkeit, die Stärkekorngröße von Stärken werden u.a. durch die strukturellen Merkmale der Stärke wie das Amylose/Amylopektin Verhältnis, das Molekulargewicht der Glucosepolymere, das Muster der Seitenkettenverteilung, den Gehalt an Ionen, den Lipid- und Proteingehalt und/oder die Stärkekornmorphologie etc. beeinflusst.

Durch auf Züchtung basierende Verfahren können ausgewählte strukturelle Merkmale der Stärke und damit auch funktionelle, ernährungsphysiologische oder strukturgebende Eigenschaften von Stärke in pflanzlichen Zellen verändert werden. Jedoch ist dieses heute nur für ausgewählte strukturelle Merkmale von Stärke (z.B. Amylopektin- / Amylosegehalt, US 5,300,145) möglich. Derzeit ist es z.B. nicht möglich, den Gehalt an Phosphat in pflanzlicher Stärke allein durch züchterische Maßnahmen zu beeinflussen.

Eine Alternative zu züchterischen Verfahren besteht in der gezielten Modifikation Stärke produzierender Pflanzen durch gentechnische Methoden. Voraussetzung hierfür ist jedoch die Identifizierung und Charakterisierung der an der Stärkesynthese und/oder Stärkemodifikation beteiligten Enzyme und deren anschließende funktionelle Analyse in transgenen Pflanzen.

An der Stärkesynthese in pflanzlichen Zellen sind verschiedene Enzyme, die unterschiedliche Reaktionen katalysieren, beteiligt. Stärkesynthasen (EC2.4.1.21, ADP-glucose:1,4-alpha-D-glucan 4-alpha-D-glucosyltransferase) katalysieren eine Polymerisierungsreaktion durch Übertragung eines Glucosylrestes von ADP-Glucose auf alpha-1,4-Glucane, wobei der übertragene Glucosylrest mit dem alpha-1,4-Glucan durch Erzeugung einer alpha-1,4-Bindung verknüpft wird. In fast allen bisher untersuchten Pflanzen konnten jeweils mehrere Isoformen von Stärkesynthasen nachgewiesen werden. Stärkesynthasen können in zwei unterschiedliche Gruppen eingeteilt werden: Stärkekorn gebundene Stärkesynthasen ("granule-bound starch synthases"; GBSS) und lösliche Stärkesynthasen ("soluble starch synthases"; im Zusammenhang mit der vorliegenden Erfindung auch als "SS" abgekürzt). Stärkekorn gebundene Stärkesynthasen katalysieren die Synthese von Amylose, wohingegen lösliche Stärkesynthasen an der Synthese von Amylopektin beteiligt sind (Ball und Morell, 2003, Annu. Rev, Plant Biol. 54, 207-233; Teltow et al., 2004, J. Expt. Bot. 55 (406), 2131-2145). Die Gruppe der löslichen Stärkesynthasen weist mehrere Isoformen auf, die in der Fachliteratur als SSI, SSII, SSIII, SSIV bezeichnet werden. Die Zuordnung von Stärkesynthasen zu den einzelnen Gruppen (SSI, SSII, SSIII, SSIV) erfolgt anhand von Sequenzhomologien der betreffenden Proteinsequenzen der jeweiligen Enzyme (Ball und Morell, 2003, Annu. Rev, Plant Biol. 54, 207-233). Jeder einzelnen Isoform der löslichen Stärkesynthasen wird nach momentaner Lehrmeinung eine spezifische Funktion bei der Stärkesynthese zugewiesen. In dicotylen Pflanzen konnte bisher nur eine Isoform von SSII Proteinen nachgewiesen werden, während in manchen monocotylen Pflanzen (z.B. Mais) zwei unterschiedliche Klassen von SSII Proteinen nachgewiesen wurden, die mit SSIIa bzw. SSIIb bezeichnet werden. In monocotylen Pflanzen wird SSIIa präferentiell im Endosperm und SSIIb präferentiell in den Blattgewebe exprimiert (Teltow et al., 2004, J. Expt. Bot. 55(406), 2131-2145). Die spezifische Funktion insbesondere der einzelnen löslichen Stärkesynthasen bei der Synthese der Stärke ist zurzeit noch nicht abschließend geklärt (Ball und Morell, 2003, Annu. Rev, Plant Biol. 54, 207-233).

Die funktionellen, ernährungsphysiologischen oder strukturgebenden Eigenschaften von Stärke werden auch vom Phosphatgehalt, einer nicht-Kohlenstoffkomponente von Stärke, beeinflusst. Dabei ist zwischen Phosphat, welches in Form von Monoestern kovalent an die Glucosemoleküle der Stärke gebunden ist (im Zusammenhang mit der vorliegenden Erfindung als Stärkephosphat bezeichnet) und Phosphat in Form von mit der Stärke assoziierten Phospholipiden zu unterscheiden.

Der Gehalt an Stärkephosphat variiert je nach Pflanzensorte. So synthetisieren z.B. bestimmte Maismutanten eine Stärke mit erhöhtem Gehalt an Stärkephosphat (waxy-Mais 0,002% und Hoch-Amylose-Mais 0,013%), während herkömmliche Maissorten nur Spuren von Stärkephosphat aufweisen. Ebenfalls geringe Mengen an Stärkephosphat findet man in Weizen (0,001 %) während in Hafer und *Sorghum* kein Stärkephosphat nachgewiesen werden konnte. In Reis-Mutanten wurde ebenfalls weniger Stärkephosphat gefunden (waxy-Reis 0,003%) als in herkömmlichen Reissorten (0,013%). Signifikante Mengen von Stärkephosphat wurden in Knollen-oder Wurzelspeicherstärke synthetisierenden Pflanzen wie z.B. Tapioca (0,008%), Süßkartoffel (0,011%), Pfeilwurz (0,021%) oder Kartoffel (0,089%) nachgewiesen. Die im vorangegangenen zitierten prozentualen Werte für den Stärkephosphatgehalt beziehen sich jeweils auf das Trockengewicht der Stärke und sind von Jane et al. (1996, Cereal Foods World 41 (11), 827-832) ermittelt worden.

Stärkephosphat kann in Form von Monoestern an der C2, C3 oder C6 Position der polymerisierten Glucosemonomere vorliegen (Takeda und Hizukuri, 1971, Starch/Stärke 23, 267-272). Die Phosphatverteilung des Phosphates in von Pflanzen synthetisierter Stärke zeichnet sich im Allgemeinen dadurch aus, dass etwa 30% bis 40% der Phosphatreste in C3-Position und etwa 60% bis 70% der Phosphatreste in C6-Position der Glucosemoleküle kovalent gebunden sind (Blennow et al., 2000, Int. J. of Biological Macromolecules 27, 211-218). Blennow et al. (2000, Carbohydrate Polymers 41, 163-174) ermittelten einen Gehalt an Stärkephosphat, der in C6-Position der Glukosemoleküle gebunden ist, für verschiedene Stärken, wie z.B. Kartoffelstärke (zwischen 7,8 und 33,5 nMol pro mg Stärke, je nach Sorte), Stärke aus verschiedenen *Curcuma* Spezies (zwischen 1,8 und 63 nMol pro mg), Tapiocastärke (2,5 nMol pro mg Stärke), Reisstärke (1,0 nMol pro mg Stärke), Mungbohnenstärke (3,5 nMol pro mg Stärke) und Sorghumstärke (0,9 nMol pro mg Stärke). In Gerstenstärke und Stärke aus verschiedenen waxy-Mutanten von Mais konnten diese Autoren kein an der C6-Position gebundenes Stärkephosphat nachweisen. Bisher konnte kein Zusammenhang zwischen dem Genotyp einer Pflanze und dem Gehalt von Stärkephosphat hergestellt werden (Jane et al., 1996, Cereal Foods World 41 (11), 827-832). Daher ist es zurzeit nicht möglich, den Gehalt an Stärkephosphat in Pflanzen durch züchterische Maßnahmen zu beeinflussen.

Bisher sind zwei Proteine beschrieben, welche die Einführung von kovalenten Bindungen von Phosphatresten an die Glucosemoleküle der Stärke vermitteln. Das erste Protein besitzt die enzymatische Aktivität einer alpha-Glucan-Wasser-Dikinase (GWD, E.C.: 2.7.9.4) (Ritte et al., 2002, PNAS 99,7166-7171), wird insbesondere in der älteren wissenschaftlichen Literatur häufig als R1 bezeichnet und ist an die Stärkekörner der Speicherstärke in Kartoffelknollen gebunden (Lorberth et al., 1998, Nature Biotechnology 16, 473-477). Das zweite, in der Literatur beschriebene Protein, das die Einführung von Stärkephosphat in Stärke katalysiert, besitzt die enzymatische Aktivität einer Phospho-Glucan-Wasser-Dikinase (PWD, E.C.: 2.7.9.5) (Kötting et al., 2005, Plant Physiol. 137, 2424-252, Baunsgaard et al., 2005, Plant Journal 41, 595-605).

Ein wesentlicher Unterschied zwischen GWD und PWD bestehet darin, dass GWD nicht phosphorylierte Stärke als Substrat verwenden kann, d.h. eine *de novo* Phosphorylierung von nicht phosphorylierter Stärke durch GWD katalysiert werden kann, während PWD bereits phosphorylierte Stärke als Substrat benötigt, d.h. zusätzlich Phosphat in bereits phosphorylierte Stärke einführt (Kötting et al., 2005, Plant Physiol. 137, 2424-252, Baunsgaard et al., 2005, Plant Journal 41, 595-605). Ein weiterer wesentlicher Unterschied zwischen GWD und PWD besteht darin, dass GWD Phosphatgruppen ausschließlich in C6-Position der Glucosemoleküle von Stärke einführt, während PWD ausschließlich die C3-Position der Glucosemoleküle von phosphorylierter Stärke phosphoryliert (Ritte et al., Manuskript eingereicht).

In der von GWD bzw. PWD katalysierten Reaktion werden die Edukte alpha-1,4-Glucan (für GWD) bzw. phosphoryliertes alpha-1,4-Glucan (für PWD), Adenosintriphosphat (ATP) und Wasser zu den Produkten Glucan-Phosphat (Stärkephosphat), Monophosphat und Adenosinmonophosphat umgesetzt (Kötting et al., 2005, Plant Physiol. 137, 2424-252, Ritte et al., 2002, PNAS 99, 7166-7171).

Weizenpflanzen, welche durch Expression eines GWD codierenden Gens aus Kartoffel eine erhöhte Aktivität von GWD Proteinen aufweisen, sind in WO 02 34923 beschrieben. Diese Pflanzen synthetisieren im Vergleich zu entsprechenden Wildtyp-Pflanzen, in welchen kein Stärkephosphat detektiert werden konnte, eine Stärke mit signifikanten Mengen an Stärkephosphat in der C6-Position der Glucosemoleküle.

WO 05 2359 beschreibt die Überexpression einer bezüglich von Maispflanzen verwendeten Codons optimierten GWD aus Kartoffel in Maispflanzen. Mittels $^{31}$P NMR wurde ein Gesamtphosphatgehalt (gebunden in C6-, C3- und C2-Position der Glucosemoleküle) der betreffenden Maisstärke von 0,0736% Phosphat bezogen auf die Menge Glucose ermittelt. Legt man für Phosphat ($H_3PO_4$) ein Molekulargewicht von 98 zu Grunde, so ergibt sich für den in WO 05 2359 ermittelten Gesamtphosphatgehalt von 0,0736% für Stärke, isoliert aus transgenen Maispflanzen, ein Gesamtphosphatgehalt von ca. 7,5 nmol Phosphat pro mg Stärke.

Pflanzen, welche durch Überexpression eines PWD codierenden Gens aus *Arabidopsis thaliana* eine erhöhte Aktivität eines PWD Proteins aufweisen, sind in WO 05 095617 beschrieben. Diese Pflanzen weisen im Vergleich zu entsprechenden nicht transformierten Wildtyp-Pflanzen einen erhöhten Gehalt an Stärkephosphat auf.

Eine wichtige funktionelle Eigenschaft, z.B. bei der Verarbeitung von Stärken in der Nahrungsmittelindustrie, stellt das Quellvermögen dar. Verschiedene strukturelle Eigenschaften von Stärken, wie das Amylose / Amylopektinverhältnis, die Seitenkettenlänge, das Molekulargewicht, die Anzahl der Verzweigungen, haben einen Einfluss auf das Quellvermögen der betreffenden Stärken (Narayana und Moorthy, 2002, Starch/Stärke 54, 559-592).

Der Fachliteratur kann der Hinweis entnommen werden, dass neben dem Amylose/Amylopektin Verhältnis, der Seitenkettenverteilung des Amylopektins und der Molekulargewichtsverteilung der Stärkepolymere auch die Menge an Stärkephosphat einen Einfluss auf funktionelle Eigenschaften, insbesondere auf das Quellvermögen (swelling power) der Stärke hat (Narayana und Moorthy, 2002, Starch/Stärke 54, 559-592).

Es ist darauf hinzuweisen, dass betreffend des Quellvermögens von Stärke zwischen Quellvermögen in kaltem Wasser (z.B. Raumtemperatur) und Quellvermögen in warmem bzw. heißem Wasser zu unterscheiden ist. Native Stärken weisen, wenn überhaupt, in kaltem Wasser ein vernachlässigbares Quellvermögen auf, während physikalisch modifizierte (vorverkleisterte, getrocknete) Stärken bereits in kaltem Wasser quellen können. Herstellungsverfahren für in kaltem Wasser quellende Stärken sind z.B. beschrieben in US 4,280,851. Im Zusammenhang mit der vorliegenden Erfindung bezieht sich der Begriff "Quellvermögen" auf das Verhalten von Stärke in warmen/heißen wässrigen Suspensionen. Das Quellvermögen wird standardmäßig ermittelt, indem Stärkekörner in Anwesenheit eines Wasserüberschusses erwärmt, nicht gebundenes Wasser nach Zentrifugation der Suspension entfernt und der Quotient aus dem Gewicht des erhaltenen Rückstandes und des Gewichts der eingewogenen Menge an Stärke gebildet wird. Bei der Durchführung dieses Verfahrens werden bei Erwärmung der Stärkesuspension kristalline Bereiche der Stärkekörner aufgelöst und Wassermoleküle in die Stärkekörner eingelagert, jedoch ohne dass dabei die Struktur des Stärkekorns selbst aufgelöst wird, d.h. es findet lediglich eine Quellung der einzelnen Stärkekörner, bedingt durch die Aufnahme von Wassermolekülen, statt.

Im Vergleich zu Cerealienstärken weisen Stärken isoliert aus Knollen oder knollenartigen Geweben ein wesentlich höheres Heißwasser Quellvermögen auf.

Für Kartoffelstärken, isoliert aus verschiedenen Varietäten, wurde nach der Methode von Leach et al. (1959, Cereal Chemistry 36, 534-544) bei 85°C ein maximales Quellvermögen von 74,15 g/g (Varietät Kufri Jyoti) ermittelt (Singh et al.,2002, Journal of the Science of Food and Agriculture 82, 1376-1383). Takizawa et al. (2004, Brazilian Archives of Biology and Technology 47(6), 921-931) ermittelten für Kartoffelstärke ein Quellvermögen von 100 g/g (90°C, nach der Methode von Leach et al. (1959, Cereal Chemistry 36, 534-544)). Weizenstärke, isoliert aus verschiedenen Kultivaren, weist ein Quellvermögen von 16,6 g/g bis 26,0 g/g (Temperatur: kochende wässrige 0,1% $AgNO_3$ Suspension) auf (Yamamori und Quynh, 2000, Theor Appl Genet 100, 23-38). Stärke, isoliert aus verschiedenen Kultivaren spelzenfreier (hull-less) Gerste, weist ein Quellvermögen von 16,5 g/g bzw. 19,3 g/g und waxy bzw. Amylose freie Stärke verschiedener Kultivare besagter Gerste weist ein Quellvermögen von 36,0 g/g bis 55,7 g/g auf (Temperatur: 70°C wässrige 0,1% $AgNO_3$, Yasui et al., 2002, Starch/Stärke 54, 179-184). Für Maisstärke wurde ein Quellvermögen von 22,3 g/g und für Hoch-Amylose Maisstärken ein Quellvermögen von 9,6 g/g (Hylon V), 6,1 g/g (Hylon VII) bzw. 3,9 g/g (LAPS = Low AmyloPectin Starch) ermittelt (90°C, Shi et al., 1998, J. Cereal Sci. 27, 289-299). In US 6,299,907 wurde für waxy Maisstärke ein Quellvermögen von 35,4 g/g angegeben. Für Stärke, isoliert aus verschiedenen Reiskultivaren wurde nach der Methode von Leach et al. (1959, Cereal Chemistry 36, 534-544) ein Quellvermögen von 26,0 g/g bis 33,2 g/g ermittelt (Sodhi und Singh, 2003, Food Chemistry 80, 99-108).Chen et al. (2003, Starch/Stärke 55, 203-212) ermittelten für verschiedene Mischungen von waxy Reisstärken mit Hoch-Amylose Reisstärken ein Quellvermögen von etwa 25 g/g bis etwa 49 g/g (95°C, wässrige Suspension). Yasui et al. (2002, Starch/Stärke 54, 179-184) ermittelten für eine Amylose freie Reisstärke ein Quellvermögen von 55,7 g/g (gemessen in kochendem Wasser in 0,1 % wässriger Silbernitrat Lösung).

Durch die Herstellung von Derivaten nativer Stärken können funktionelle Eigenschaften der Stärken verändert werden. Kreuzvernetzte ("cross-linked") Weizenstärken weisen je nach Grad der Kreuzvernetzung ein Quellvermögen von 6,8 g/g bis 8,9 g/g, acetylierte Weizenstärken ein Quellvermögen von maximal 10,3 g/g und gleichzeitig kreuzvernetzte und acetylierte Weizenstärken ein Quellvermögen von 9,4 g/g auf, während die entsprechenden nicht derivatisierten Stärken ein Quellvermögen von 8,8 g/g aufwiesen (gemessen bei 90°C; Van Hung und Morita, 2005, Starch/Stärke 57, 413-420). Für acetylierte Waxy-Reisstärken wurde ein Quellvermögen von ca. 30 g/g und für kreuzvernetzte Waxy-Reisstärke ein Quellvermögen von ca. 15 g/g ermittelt, während entsprechende nicht derivatisierte Waxy-Reisstärke ein Quellvermögen von ca. 41 g/g aufwies. Acetylierte Reisstärke wies ein Quellvermögen von ca. 20 g/g und kreuzvernetzte Reisstärke ein Quellvermögen von ca. 13 g/g auf, während entsprechende nicht derivatisierte Reisstärke ein Quellvermögen von ca. 14 g/g aufwies (gemessen bei 90°C, Liu et al., 1999, Starch/Stärke 52, 249-252). US 6,299,907 beschreibt kreuzvernetzte Stärken, wobei die Kreuzvernetzungsreaktion nach Vorquellung der betreffenden Stärken in einer Natriumhydroxid/Sulfat Lösung durchgeführt wurde. Je nach Grad der Kreuzvernetzung wurde für Weizenstärke ein Quellvermögen von 6,8 g/g bis 7,3 g/g (entsprechende nicht derivatisierte Weizenstärke 14,7 g/g), für Hydroxypropylweizenstärke ein Quellvermögen von 9,7 g/g (entsprechende nicht derivatisierte Weizenstärke 22,9 g/g), für kreuzvernetzte Maisstärke ein Quellvermögen von 5,9 g/g (entsprechende nicht derivatisierte Maisstärke 16,7 g/g), für kreuzvernetzte Waxy-Maisstärke ein Quellvermögen von 8,3 g/g (entsprechende nicht derivatisierte Waxy-Maisstärke 35,4 g/g) und für kreuzvernetzte Kartoffelstärke ein Quellvermögen von 6,7 g/g (entsprechende nicht derivatisierte Kartoffelstärke wurde nicht genau spezifiziert) ermittelt (gemessen bei 95°C). Daraus ergibt sich, dass das Quellvermögen von Stärke durch heutzutage gängige Methoden der Derivatisierung wenn überhaupt, nicht wesentlich gesteigert werden kann.

Der vorliegenden Erfindung liegt die Aufgabe zu Grunde, modifizierte Stärken mit veränderten funktionellen Eigenschaften, sowie Pflanzenzellen und Pflanzen, die eine Stärke mit veränderten funktionellen Eigenschaften synthetisieren, als auch Verfahren und Mittel zur Erzeugung besagter Pflanzen und/oder Pflanzenzellen zur Verfügung zu stellen.

Somit betrifft die vorliegende Erfindung genetisch modifizierte Pflanzenzellen und genetisch modifizierte Pflanzen, dadurch gekennzeichnet, dass sie eine erhöhte Aktivität eines Proteins mit der Aktivität einer Stärkesynthase II und eine erhöhte Aktivität eines Proteins mit der Aktivität einer Glucan-Wasser-Dikinase aufweisen, im Vergleich zu entsprechenden genetisch nicht modifizierten Wildtyp-Pflanzenzellen bzw. Wildtyp-Pflanzen.

Ein erster Aspekt der vorliegenden Erfindung betrifft eine Pflanzenzelle oder eine Pflanze, die genetisch modifiziert ist, wobei die genetische Modifikation zur Erhöhung der Aktivität mindestens eines Proteins mit der Aktivität einer Stärkesynthase II und gleichzeitig mindestens eines Proteins mit der Aktivität einer Glucan-Wasser-Dikinase führt, im Vergleich zu entsprechenden nicht genetisch modifizierten Wildtyp-Pflanzenzellen bzw. Wildtyp-Pflanzen.

Die genetische Modifikation kann dabei jede genetische Modifikation sein, die zu einer Erhöhung der Aktivität mindestens eines Proteins mit der Aktivität einer Stärkesynthase II und (gleichzeitig) mindestens eines Proteins mit der Aktivität einer Glucan-Wasser-Dikinase in genetisch modifizierten Pflanzenzellen oder genetisch modifizierten Pflanzen führt, im Vergleich zu entsprechenden nicht genetisch modifizierten Wildtyp-Pflanzenzellen oder Wildtyp-Pflanzen.

Der Begriff "Wildtyp-Pflanzenzelle" bedeutet im Zusammenhang mit der vorliegenden Erfindung, dass es sich um Pflanzenzellen handelt, die als Ausgangsmaterial für die Herstellung der erfindungsgemäßen Pflanzenzellen dienten, d.h. deren genetische Information, abgesehen von der eingeführten genetischen Modifikation, der einer erfindungsgemäßen Pflanzenzelle entspricht.

Der Begriff "Wildtyp-Pflanze" bedeutet im Zusammenhang mit der vorliegenden Erfindung, dass es sich um Pflanzen handelt, die als Ausgangsmaterial für die Herstellung der erfindungsgemäßen Pflanzen dienten, d.h. deren genetische Information, abgesehen von der eingeführten genetischen Modifikation, der einer erfindungsgemäßen Pflanze entspricht.

Der Begriff "entsprechend" bedeutet im Zusammenhang mit der vorliegenden Erfindung, dass beim Vergleich von mehreren Gegenständen die betreffenden Gegenstände, die miteinander verglichen werden, unter gleichen Bedingungen gehalten wurden. Im Zusammenhang mit der vorliegenden Erfindung bedeutet der Begriff "entsprechend" im Zusammenhang mit Wildtyp-Pflanzenzelle oder Wildtyp-Pflanze, dass die Pflanzenzellen oder Pflanzen, die miteinander verglichen werden, unter gleichen Kulturbedingungen aufgezogen wurden und dass sie ein gleiches (Kultur-) Alter aufweisen.

Der Begriff "erhöhte Aktivität mindestens eines Proteins mit der Aktivität einer Stärkesynthase II" bedeutet dabei im Rahmen der vorliegenden Erfindung eine Erhöhung der Expression endogener Gene, die Proteine mit der Aktivität einer Stärkesynthase II codieren und/oder eine Erhöhung der Menge an Proteinen mit der Aktivität einer Stärkesynthase II in den Zellen und/oder eine Erhöhung der enzymatischen Aktivität von Proteinen mit der Aktivität einer Stärkesynthase II in den Zellen.

Der Begriff "erhöhte Aktivität eines Proteins mit der Aktivität einer Glucan-Wasser-Dikinase" bedeutet dabei im Rahmen der vorliegenden Erfindung eine Erhöhung der Expression endogener Gene, die Proteine mit der Aktivität einer Glucan-Wasser-Dikinase codieren und/oder eine Erhöhung der Menge an Proteinen mit der Aktivität einer Glucan-Wasser-Dikinase in den Zellen und/oder eine Erhöhung der enzymatischen Aktivität von Proteinen mit der Aktivität einer Glucan-Wasser-Dikinase in den Zellen.

Die Erhöhung der Expression kann beispielsweise bestimmt werden durch Messung der Menge an Transkripten, die Proteine mit der Aktivität einer Stärkesynthase II oder die Proteine mit der Aktivität einer Glucan-Wasser-Dikinase codieren. Dieses kann z.B. durch Northern-Blot-Analyse oder RT-PCR erfolgen. Eine Erhöhung der Menge an Transkripten, die ein Protein mit der Aktivität einer Stärkesynthase II codieren, bedeutet dabei vorzugsweise eine Erhöhung der Menge an Transkripten im Vergleich zu entsprechenden nicht genetisch modifizierten Zellen um mindestens 100%, insbesondere um mindestens 125%, bevorzugt um mindestens 150% und besonders bevorzugt um mindestens 200%. Eine Erhöhung der Menge an Transkripten, codierend ein Protein mit der Aktivität einer Stärkesynthase II, bedeutet auch, dass Pflanzen oder Pflanzenzellen, die keine nachweisbaren Mengen an Transkripten, codierend ein Protein mit der Aktivität einer Stärkesynthase II, aufweisen, nach erfindungsgemäßer genetischer Modifikation nachweisbare Mengen an Transkripten, codierend ein Protein mit der Aktivität einer Stärkesynthase II aufweisen.

Eine Erhöhung der Menge an Transkripten, die ein Protein mit der Aktivität einer Glucan-Wasser-Dikinase codieren, bedeutet dabei vorzugsweise eine Erhöhung der Menge an Transkripten im Vergleich zu entsprechenden nicht genetisch modifizierten Zellen um mindestens 50%, insbesondere um mindestens 70%, bevorzugt um mindestens 85% und besonders bevorzugt um mindestens 100%.

Eine Erhöhung der Menge an Transkripten, codierend ein Protein mit der Aktivität einer Glucan-Wasser-Dikinase, bedeutet auch, dass Pflanzen oder Pflanzenzellen, die keine nachweisbaren Mengen an Transkripten, codierend ein Protein mit der Aktivität einer Glucan-Wasser-Dikinase, aufweisen, nach erfindungsgemäßer genetischer Modifikation nachweisbare Mengen an Transkripten, codierend ein Protein mit der Aktivität einer Glucan-Wasser-Dikinase, aufweisen.

Die Erhöhung der Menge an Protein mit der Aktivität einer Stärkesynthase II oder mit der Aktivität einer Glucan-Wasser-Dikinase, die eine erhöhte Aktivität dieser Proteine in den betreffenden Pflanzenzellen zur Folge hat, kann beispielsweise bestimmt werden durch immunologische Methoden wie Western-Blot-Analyse, ELISA (Enzyme Linked Immuno Sorbent Assay) oder RIA (Radio Immune Assay). Eine Erhöhung der Menge eines Proteins mit der Aktivität einer Stärkesynthase II bedeutet dabei vorzugsweise eine Erhöhung der Menge an betreffendem Protein im Vergleich zu entsprechenden nicht genetisch modifizierten Zellen um mindestens 100%, insbesondere um mindestens 125%, bevorzugt um mindestens 150% und besonders bevorzugt um mindestens 200%. Eine Erhöhung der Menge an Proteinen mit der Aktivität einer Stärkesynthase II bedeutet auch, dass Pflanzen oder Pflanzenzellen, die keine nachweisbaren Mengen an Protein mit der Aktivität einer Stärkesynthase II aufweisen, nach erfindungsgemäßer genetischer Modifikation eine nachweisbare Menge an Protein mit der Aktivität einer Stärkesynthase II aufweisen.

Eine Erhöhung der Menge eines Proteins mit der Aktivität einer Glucan-Wasser-Dikinase bedeutet dabei vorzugsweise eine Erhöhung der Menge an betreffendem Protein im Vergleich zu entsprechenden nicht genetisch modifizierten Zellen um mindestens 50%, insbesondere um mindestens 70%, bevorzugt um mindestens 85% und besonders bevorzugt um mindestens 100%.

Eine Erhöhung der Menge an Protein mit der Aktivität einer Glucan-Wasser-Dikinase bedeutet auch, dass Pflanzen oder Pflanzenzellen, die keine nachweisbaren Mengen an Proteinen mit der Aktivität einer Glucan-Wasser-Dikinase aufweisen, nach erfindungsgemäßer genetischer Modifikation eine nachweisbare Menge an Protein mit der Aktivität einer Glucan-Wasser-Dikinase aufweisen.

Methoden zur Herstellung von Antikörpern, die spezifisch mit einem bestimmten Protein reagieren, d.h. die spezifisch an besagtes Protein binden, sind dem Fachmann bekannt (siehe z.B. Lottspeich und Zorbas (Eds.), 1998, Bioanalytik, Spektrum akad, Verlag, Heidelberg, Berlin, ISBN 3-8274-0041-4). Die Herstellung solcher Antikörper wird von einigen Firmen (z.B. Eurogentec, Belgien) als Auftragsservice angeboten. Antikörper, mit welchem eine Erhöhung der Menge an Protein mit der Aktivität einer Glucan-Wasser-Dikinase mittels immunologischer Methoden festgestellt werden kann, sind bei Lorberth et al. (1998, Nature Biotechnology 16, 473-477) und Ritte et al. (2000, Plant Journal 21, 387-391) beschrieben. Antikörper, mit welchem eine Erhöhung der Menge an Protein mit der Aktivität einer Stärkesynthase II mittels immunologischer Methoden festgestellt werden kann, sind bei Walter ("Untersuchungen der Expression und Funktion der Stärkesynthase II (SSII) aus Weiten (Triticum aestivum)", Dissertation am Fachbereich Biologie der Universität Hamburg, ISBN 3-8265-8212-8) beschrieben.

Die Menge der Aktivität eines Proteins mit der Aktivität einer Glucan-Wasser-Dikinase kann z.B. nachgewiesen werden wie in der Literatur beschrieben (Mikkelsen et al., 2004, Biochemical Journal 377, 525-532; Ritte et al., 2002, PNAS 99, 7166-7171).

Die Menge der Aktivität eines Proteins mit der Aktivität einer Stärkesynthase II kann z.B. wie in der Literatur beschrieben (Nishi et al., 2001, Plant Physiology 127, 459-472) bestimmt werden. Eine bevorzugte Methode zur Bestimmung der Menge an Aktivität eines Proteins mit der Aktivität einer Stärkesynthase II ist unter allgemeine Methoden Punkt 9 beschrieben.

Vorzugsweise weisen erfindungsgemäße Pflanzenzellen oder erfindungsgemäße Pflanzen eine Aktivität eines Proteins mit der Aktivität einer Stärkesynthase II auf, die mindestens 6 fach, bevorzugt mindestens 7 fach, besonders bevorzugt mindestens 8 fach, insbesondere bevorzugt mindestens 9 fach und speziell bevorzugt mindestens 10 fach erhöht ist, im Vergleich zu entsprechenden genetisch nicht modifizierten Wildtyp-Pflanzenzellen bzw. Wildtyp-Pflanzen.

Proteine mit der Aktivität einer Stärkesynthase II (ADP-Glukose-1,4-alpha-D-glucan-4-alpha-D-glucosyltransferase; EC 2.4.1.21) weisen in ihrem Aufbau eine Abfolge bestimmter Domänen auf. Am N-Terminus weisen sie ein Signalpeptid für den Transport in Plastiden auf. In Richtung vom N-Terminus nach C-Terminus folgen eine N-terminale Region und eine katalytische Domäne. (Li et al., 2003, Funct Integr Genomics 3, 76-85). Weitere Analysen, basierend auf Aminosäure Sequenzvergleichen (http://hits.isb-sib.ch/cgi-bin/PFSCAN) verschiedener Proteine mit der Aktivität einer Stärkesynthase II ergaben, dass diese Proteine drei spezifische Domänen aufweisen. In der unter SEQ ID NO 6 dargestellten Aminosäuresequenz stellen die Aminosäuren 322 bis 351 Domäne 1, die Aminosäuren 423 bis 462 Domäne 2 und die Aminosäuren 641 bis 705 Domäne 3 dar. Domäne 1 wird von den Nucleotiden 1190 bis 1279, Domäne 2 von den Nucleotiden 1493 bis 1612 und Domäne 3 von den Nucleotiden 2147 bis 2350 der unter SEQ ID NO 5 dargestellten Nucleinsäuresequenz codiert.

Im Zusammenhang mit der vorliegenden Erfindung soll unter dem Begriff "Protein mit der Aktivität einer Stärkesynthase II" ein Protein verstanden werden, dass eine Glucosylierungsreaktion katalysiert, bei der Glucosereste des Substrates ADP-Glucose unter Bildung einer alpha-1,4-Verknüpfung auf alpha-1,4-verknüpfte Glucanketten übertragen werden (ADP-Glukose + {(1,4)- alpha-D-glucosyl}(N) <=> ADP + {(1,4)- alpha-D-glucosyl}(N+1)), wobei die Aminosäuresequenz des Proteins mit der Aktivität eines Proteins einer Stärkesynthase II mit den Aminosäuren 322 bis 351 (Domäne 1) der unter SEQ ID NO 6 dargestellten Aminosäuresequenz eine Identität von mindestens 86%, bevorzugt mindestens 93%, besonders bevorzugt mindestens 95% und mit den Aminosäuren 423 bis 462 (Domäne 2) der unter SEQ ID NO 6 dargestellten Aminosäuresequenz eine Identität von mindestens 83%, bevorzugt mindestens 86%, besonders bevorzugt mindestens 95% und mit den Aminosäuren 641 bis 705 (Domäne 3) der unter SEQ ID NO 6 dargestellten Aminosäuresequenz eine Identität von mindestens 70%, vorzugsweise mindestens 82%, bevorzugt 86%, besonders bevorzugt 98%, insbesondere bevorzugt von mindestens 95% aufweist.

Nucleinsäuresequenzen und die dazu korrespondierenden Aminosäuresequenzen, die eine besagte Identität mit den Domänen 1, 2 und 3 aufweisen und ein Protein mit der Aktivität einer Stärkesynthase II codieren, sind dem Fachmann bekannt und z.B. veröffentlicht unter Accession No AY133249 (*Hordeum vulgare*), Accession No AY133248 (*Aegilops tauschii*), Accession Nos XP467757, AAK64284 (*Oryza sativa*), Accession No AAK81729 (*Oryza sativa*) Accession Nos AAD13341, AAS77569, No AAD13342 (*Zea mays*), Accession No AAF13168 (*Manihut esculenta*), Accession No BAD18846 (*Phaseolus vulgaris*), Accession No CAA61241 *(Solanum tuberosum),* Accession No CAA61269 (*Pisum sativum*), Accession No AAC19119 (*Ipomea batatas*), Accession No AAF 26156 (*Arabidopsis thaliana*), Accession No AAP41030 (*Colocasia esculenta*), Accession No AAS88880 (*Ostraeococcus tauri*), oder Accession No AAC17970 (*Chlamydomonas reinhardii*). Die genannten Nucleinsäuresequenzen und Aminosäuresequenzen codierend ein Protein mit der Aktivität einer Stärkesynthase II sind zugänglich über NCBI (http://www.ncbi.nlm.nih.gov/entrez/) und sind durch Nennung der Referenzen ausdrücklich in die Beschreibung der vorliegenden Anmeldung aufgenommen.

Im Rahmen der vorliegenden Erfindung soll unter dem Begriff "Protein mit der Aktivität einer Glucan-Wasser-Dikinase" ein Protein verstanden werden, welches einen beta-Phosphatrest von ATP auf Stärke überträgt. Stärken, isoliert aus Blättern einer *Arabidopsis thaliana sex1-3* Mutante weisen keine nachweisbaren Mengen an kovalent gebundenen Phosphatresten auf, werden jedoch von einem Protein mit der Aktivität einer Glucan-Wasser-Dikinase phosphoryliert. D.h. nicht-phosphorylierte-Stärke, z.B. isoliert aus Blättern einer *Arabidopsis thaliana sex1-3* Mutante, wird in einer durch ein Protein mit der Aktivität einer Glucan-Wasser-Dikinase katalysierten Phosphorylierungsreaktion als Substrat verwendet.

Von einem Protein mit der Aktivität einer Glucan-Wasser-Dikinase wird der beta-Phosphatrest des ATP auf die Stärke und der gamma-Phosphatrest des ATP auf Wasser übertragen. Als weiteres Reaktionsprodukt entsteht AMP (Adenosinmonophosphat). Ein Protein mit der Aktivität einer Glucan-Wasser-Dikinase wird daher auch als [alpha-1,4-Glucan]-Wasser-Dikinase bzw. als Stärke-Wasser-Dikinase bezeichnet (E.C.: 2.7.9.4; Ritte et al., 2002, PNAS 99, 7166-7171).

Bei der durch ein Protein mit der Aktivität einer Glucan-Wasser-Dikinase katalysierten Phosphorylierung von Stärke entstehen zusätzliche Phosphatmonoesterbindungen ausschließlich in C6-Position der Glucosemoleküle (Ritte et al., Manuskript in Vorbereitung). Bei der Katalyse der Phosphorylierungsreaktion einer Stärke durch ein Protein mit der Aktivität einer Glucan-Wasser-Dikinase entsteht als Zwischenprodukt ein phosphoryliertes Protein, bei welchem der beta-Phosphatrest des ATP kovalent an eine Aminosäure des Proteins mit der Aktivität einer Glucan-Wasser-Dikinase gebunden ist (Ritte et al., 2002, PNAS 99, 7166-7171). Das Zwischenprodukt entsteht durch Autophosphorylierung des Proteins mit der Aktivität einer Glucan-Wasser-Dikinase, d.h. das Protein mit der Aktivität einer Glucan-Wasser-Dikinase selbst katalysiert die Reaktion, die zu dem Zwischenprodukt führt. Aminosäuresequenzen, die Proteine mit der Aktivität einer Glucan-Wasser-Dikinase codieren, enthalten eine Phosphohistidindomäne. Phosphohistidindomänen sind z.B. beschrieben bei Tien-Shin Yu et al. (2001, Plant Cell 13, 1907-1918). Bei der Autophosphorylierung eines Proteins mit der Aktivität einer Glucan-Wasser-Dikinase wird ein Histidinrest in der Phosphohistidindomäne der Aminosäuresequenz, codierend ein Protein mit der Aktivität einer Glucan-Wasser-Dikinase, phosphoryliert (Mikkelsen et al., 2004, Biochemical Journal 377, 525-532). In der beispielsweise unter SEQ ID NO 2 dargestellten Proteinsequenz eines Proteins mit der Aktivität einer Glucan-Wasser-Dikinase aus *Solanum tuberosum* stellen die Aminosäuren 1064 bis 1075 die Phosphohistidindomäne dar. Wird der konservierte Histidinrest (in der beispielsweise unter SEQ ID NO 2 dargestellten Proteinsequenz Aminosäure 1069) der Phosphohistidindomäne durch eine andere Aminosäure ersetzt, findet keine Autophosphorylierung und damit auch keine Phosphorylierung von Glucanen durch das mutagenisierte Protein mehr statt (Mikkelsen et al., 2004, Biochemical Journal 377, 525-532). Weiterhin zeichnet sich ein Protein mit der Aktivität einer Glucan-Wasser-Dikinase dadurch aus, dass es eine C-terminale Nucleotidbindedomäne aufweist, die in der beispielsweise unter SEQ ID NO 2 dargestellten Aminosäuresequenz von den Aminosäuren 1121 bis 1464 umfasst wird. Eine Deletion der Nucleotidbindedomäne führt zur Inaktivierung eines Proteins mit der Aktivität einer Glucan-Wasser-Dikinase (Mikkelsen und Blennow, 2005, Biochemical Journal 385, 355-361). Am N-Terminus weisen Proteine mit der Aktivität einer Glucan-Wasser-Dikinase eine Kohlenhydratbindedomäne (CBM, Carbohydrate Binding Domain) auf, die in der unter SEQ ID NO 2 dargestellten Aminosäuresequenz von den Aminosäuren 78 bis 362 umfasst wird. Kohlenhydratbindedomänen zeichnen sich u.a. dadurch aus, dass ihre Aminosäuresequenz konservierte Tryptophanreste aufweisen. Werden diese konservierten Aminosäurereste gegen andere Aminosäuren ausgetauscht, so verlieren die Kohlenhydratbindedomänen ihre Fähigkeit, Glucane zu binden. So führt z.B. ein Austausch der Aminosäuren W139 oder W194 in der unter SEQ ID NO 2 dargestellten Aminosäuresequenz zu einem Verlust der Funktion dieser Kohlenhydratbindedomäne. Wird die Kohlenhydratbindedomäne einer Glucan-Wasser-Dikinase deletiert (beispielsweise eine Deletion der Aminosäuren 1 bis 362,

wobei die Aminosäuren 1 bis 77 in der unter SEQ ID NO 2 dargestellten Aminosäuresequenz ein plastidäres Signalpeptid darstellen), führt dies jedoch nicht zur Inaktivierung der phosphorylierenden Aktivität des Enzyms (Mikkelsen et al., 2006, Biochemistry 45,4674-4682).

Nukleinsäuresequenzen und zu diesen korrespondierende Aminosäuresequenzen, codierend ein Protein mit der Aktivität einer Glucan-Wasser-Dikinase sind aus unterschiedlichen Spezies, wie z.B. Kartoffel (WO 97 11188, GenBank Acc.: AY027522, Y09533), Weizen (WO 00 77229, US 6,462,256, GenBank Acc.: AAN93923, GenBank Acc.: AR236165), Reis (GenBank Acc.: AAR61445, GenBank Acc.: AR400814), Mais (GenBank Acc.: AAR61444, GenBank Acc.: AR400813), Soyabohne (GenBank Acc.: AAR61446, GenBank Acc.: AR400815), *Curcuma longa* (SEQ ID NO 3), Citrus (GenBank Acc.: AY094062), *Arabidopsis* (GenBank Acc.: AF312027) und der Grünalge *Ostreococcus tauri* (GenBank Acc.: AY570720.1) beschrieben. Die genannten Nucleinsäuresequenzen und Aminosäuresequenzen codierend ein Protein mit der Aktivität einer Glucan-Wasser-Dikinase sind u.a. veröffentlicht vom NCBI (http://www.ncbi.nlm.nih.gov/entrez/) und sind durch Nennung der Referenzen ausdrücklich in die Beschreibung der vorliegenden Anmeldung aufgenommen.

Eine weitere Ausführungsform der vorliegenden Erfindung betrifft eine erfindungsgemäße genetisch modifizierte Pflanzenzelle oder eine erfindungsgemäße genetisch modifizierte Pflanze, wobei die genetische Modifikation in der Einführung mindestens eines fremden Nucleinsäuremoleküls in das Genom der Pflanzenzelle bzw. in das Genom der Pflanze besteht.

In diesem Zusammenhang bedeutet der Begriff "genetische Modifikation" das Einführen von homologen und/oder heterologen fremden Nucleinsäuremolekülen in das Genom einer Pflanzenzelle oder in das Genom einer Pflanze, wobei besagtes Einführen dieser Moleküle zur Erhöhung der Aktivität eines Proteins mit der Aktivität einer Glucan-Wasser-Dikinase und zur Erhöhung der Aktivität eines Proteins mit der Aktivität einer Stärkesynthase II führt. Durch Einführung eines fremden Nucleinsäuremoleküls sind die erfindungsgemäßen Pflanzenzellen oder erfindungsgemäßen Pflanzen in ihrer genetischen Information verändert. Das Vorhandensein oder die Expression mindestens eines fremden Nucleinsäuremoleküls führt zu einer phänotypischen Veränderung. "Phänotypische" Veränderung bedeutet dabei vorzugsweise eine messbare Veränderung einer oder mehrerer Funktionen der Zellen. Beispielsweise zeigen die genetisch modifizierten erfindungsgemäßen Pflanzenzellen und die genetisch modifizierten erfindungsgemäßen Pflanzen aufgrund des Vorhandenseins oder bei Expression eingeführter fremder Nucleinsäuremoleküle eine Erhöhung der Aktivität eines Proteins mit der Aktivität einer Glucan-Wasser-Dikinase und eine Erhöhung der Aktivität eines Proteins mit der Aktivität einer Stärkesynthase II.

Unter dem Begriff "fremdes Nucleinsäuremolekül" versteht man im Zusammenhang mit der vorliegenden Erfindung ein solches Molekül, das entweder natürlicherweise in entsprechenden Wildtyp-Pflanzenzellen nicht vorkommt, oder das in der konkreten räumlichen Anordnung nicht natürlicherweise in Wildtyp-Pflanzenzellen vorkommt oder das an einem Ort im Genom der Wildtyp-Pflanzenzelle lokalisiert ist, an dem es natürlicherweise nicht vorkommt. Bevorzugt ist das fremde Nucleinsäuremolekül ein rekombinantes Molekül, das aus verschiedenen Elementen besteht, deren Kombination oder spezifische räumliche Anordnung natürlicherweise in pflanzlichen Zellen nicht auftritt.

Prinzipiell kann ein fremdes Nucleinsäuremolekül jedes beliebige Nucleinsäuremolekül sein, das in der Pflanzenzelle oder Pflanze eine Erhöhung der Aktivität eines Proteins mit der Aktivität einer Glucan-Wasser-Dikinase und eines Proteins mit der Aktivität einer Stärkesynthase II bewirkt.

Unter dem Begriff "rekombinantes Nukleinsäuremolekül" soll im Zusammenhang mit der vorliegenden Erfindung ein Nukleinsäuremolekül verstanden werden, welches unterschiedliche Nucleinsäuremoleküle aufweist, die natürlicherweise nicht in einer Kombination vorliegen, wie sie in einem rekombinanten Nucleinsäuremolekül vorliegen. So können rekombinante Nucleinsäuremoleküle z.B., neben Nucleinsäuremolekülen, die ein Protein mit der Aktivität einer Glucan-Wasser-Dikinase und/oder ein Protein mit der Aktivität einer Stärkesynthase II codieren, zusätzliche Nucleinsäuresequenzen aufweisen, welche natürlicherweise nicht in Kombination mit den genannten Nucleinsäuremolekülen vorliegen. Die genannten zusätzlichen Nucleinsäuresequenzen, die auf einem rekombinanten Nucleinsäuremolekül in Kombination mit einem Protein mit der Aktivität einer Glucan-Wasser-Dikinase oder einem Protein mit der Aktivität einer Stärkesynthase II codierenden Nucleinsäuremolekül vorliegen, können dabei beliebige Sequenzen sein. Sie können z.B. genomische und/oder pflanzliche Nucleinsäuresequenzen darstellen. Bevorzugt handelt es sich bei genannten zusätzlichen Nucleinsäuresequenzen um regulatorische Sequenzen (Promotoren, Terminationssignale, Enhancer), besonders bevorzugt um regulatorische Sequenzen, die in pflanzlichem Gewebe aktiv sind, insbesondere bevorzugt um gewebespezifische regulatorische Sequenzen, die in pflanzlichem Gewebe aktiv sind. Methoden zur Erzeugung rekombinanter Nucleinsäuremoleküle sind dem Fachmann bekannt und umfassen gentechnische Methoden, wie z.B. die Verbindung von Nucleinsäuremolekülen durch Ligation, genetische Rekombination oder die Neusynthese von Nucleinsäuremolekülen (siehe z.B. Sambrok et al., Molecular Cloning, A Laboratory Manual, 3rd edition (2001) Cold Spring Harbour Laboratory Press, Cold Spring Harbour, NY. ISBN: 0879695773, Ausubel et al., Short Protocols in Molecular Biology, John Wiley & Sons; 5th edition (2002),ISBN: 0471250929).

Unter dem Begriff "Genom" soll im Zusammenhang mit der vorliegenden Erfindung die Gesamtheit des in einer pflanzlichen Zelle vorliegenden Erbmaterials verstanden werden. Dem Fachmann ist bekannt, dass neben dem Zellkern auch andere Kompartimente (z.B. Plastiden, Mitochondrien) Erbmaterial enthalten.

In einer weiteren Ausführungsform sind die erfindungsgemäßen Pflanzenzellen und die erfindungsgemäßen Pflanzen dadurch gekennzeichnet, dass mindestens ein fremdes Nucleinsäuremolekül ein Protein mit der Aktivität einer Glucan-Wasser-Dikinase codiert. Bevorzugt handelt es sich bei den fremden Nucleinsäuremolekülen, codierend ein Protein mit der Aktivität einer Glucan-Wasser-Dikinase um die bereits genannten, dem Fachmann bekannten Nucleinsäuremoleküle aus den verschiedenen Pflanzenspezies, besonders bevorzugt um Nucleinsäuremoleküle, codierend ein Protein mit der Aktivität einer Glucan-Wasser-Dikinase aus Kartoffel oder *Curcuma longa,* insbesondere bevorzugt ein Protein mit der Aktivität einer Glucan-Wasser-Dikinase, welches die unter SEQ ID NO 2 dargestellte Aminosäuresequenz aufweist oder von der in SEQ ID NO 1 dargestellten Nucleinsäuresequenz codiert wird.

Die unter SEQ ID NO 3 und SEQ ID NO 4 dargestellten Sequenzen sind bisher unveröffentlicht. Pflanzenzellen oder Pflanzen, insbesondere Reispflanzenzellen bzw. Reispflanzen, die ein fremdes Nucleinsäuremolekül, codierend ein Protein mit der Aktivität einer Glucan-Wasser-Dikinase aus *Curcuma longa,* aufweisen, zeichnen sich dadurch aus, dass sie eine Stärke synthetisieren, die einen höheren Stärkephosphatgehalt aufweist, als Pflanzenzellen oder Pflanzen, die ein fremdes Nucleinsäuremolekül, codierend ein Protein mit der Aktivität einer Glucan-Wasser-Dikinase aus anderen Spezies (z.B. Kartoffel) aufweisen.

Die vorliegende Erfindung betrifft daher auch Nucleinsäuremoleküle, codierend ein Protein mit der Aktivität einer Glucan-Wasser-Dikinase, ausgesucht aus der Gruppe bestehend aus

>   a) Nucleinsäuremolekülen, die ein Protein mit der unter SEQ ID NO 4 Aminosäuresequenz codieren;
>   b) Nucleinsäuremolekülen, die ein Protein codieren, dessen Aminosäuresequenz mindestens 90%, vorzugsweise von mindestens 93%, bevorzugt von mindestens 96% und insbesondere bevorzugt von mindestens 99% zu der unter SEQ ID NO 4 dargestellten Aminosäuresequenz aufweisen;
>   c) Nucleinsäuremolekülen, die die unter SEQ ID NO 3 dargestellte Nucleinsäuresequenz oder eine komplementäre Sequenz umfassen;
>   d) Nucleinsäuremolekülen, die mit der unter SEQ ID NO 3 dargestellten Nucleinsäuresequenz eine Identität von mindestens 90%, vorzugsweise von mindestens 93%, bevorzugt von mindestens 96% und insbesondere bevorzugt von mindestens 99% aufweisen,
>   e) Nucleinsäuremolekülen, welche mit mindestens einem Strang der unter a) oder c) beschriebenen Nucleinsäuremolekülen unter stringenten Bedingungen hybridisieren;
>   f) Nucleinsäuremolekülen, deren Nucleotidsequenz aufgrund der Degeneration des genetisches Codes von der Sequenz der unter a), oder c) genannten Nucleinsäuremoleküle abweicht;
>   g) Nucleinsäuremolekülen, die Fragmente, allelische Varianten und/oder Derivate der unter a), b), c), d), e) oder f) genannten Nucleinsäuremoleküle darstellen,
>   h) Nucleinsäuremoleküle, nach a), b), c), d), e), f) oder g), die mit regulatorischen Elementen (Promotor) verknüpft sind, die die Transkription in Pflanzenzellen initiieren oder
>   i) Nucleinsäuremoleküle, nach h), wobei die Promotoren gewebespezifische Promotoren, besonders bevorzugt Promotoren, die die Initiation der Transkription spezifisch in pflanzlichen Endospermzellen initiieren, darstellen.

Weiterhin sind Plasmide, Vektoren und Pflanzenzellen oder Pflanzen, die ein fremdes erfindungsgemäßes Nucleinsäuremolekül aufweisen, Gegenstand der vorliegenden Erfindung.

In einer weiteren Ausführungsform sind die erfindungsgemäßen Pflanzenzellen und die erfindungsgemäßen Pflanzen dadurch gekennzeichnet, dass mindestens ein fremdes Nucleinsäuremolekül ein Protein mit der Aktivität einer Stärkesynthase II codiert. Bevorzugt handelt es sich bei den fremden Nucleinsäuremolekülen, codierend ein Protein mit der Aktivität einer Stärkesynthase II, um die bereits genannten, dem Fachmann bekannten Nucleinsäuremoleküle aus den verschiedenen Pflanzenspezies, besonders bevorzugt um Nucleinsäuremoleküle, codierend ein Protein mit der Aktivität einer Stärkesynthase II aus Weizen, insbesondere bevorzugt ein Protein mit der Aktivität einer Stärkesynthase II, welches die unter SEQ ID NO 6 dargestellte Aminosäuresequenz aufweist oder von der in SEQ ID NO 5 dargestellten Nucleinsäuresequenz codiert wird.

In einer weiteren Ausführungsform sind die erfindungsgemäßen Pflanzenzellen und die erfindungsgemäßen Pflanzen dadurch gekennzeichnet, dass ein erstes fremdes Nucleinsäuremolekül ein Protein mit der Aktivität einer Glucan-Wasser-Dikinase codiert und ein zweites fremdes Nucleinsäuremolekül ein Protein mit der Aktivität einer Stärkesynthase II codiert.

Bei den zur genetischen Modifikation in die Pflanzenzelle oder Pflanze eingebrachten fremden Nukleinsäuremolekülen kann es sich um ein einzelnes Nucleinsäuremolekül oder um mehrere Nucleinsäuremoleküle handeln. Es kann sich daher sowohl um Nucleinsäuremoleküle handeln, die für ein Protein mit der Aktivität einer Glucan-Wasser-Dikinase codierende Nucleinsäuresequenzen und für ein Protein mit der Aktivität einer Stärkesynthase II codierende Nucleinsäuresequenzen enthalten, als auch um Nucleinsäuremoleküle, bei denen die für ein Protein mit der Aktivität einer Glucan-Wasser-Dikinase codierenden Nucleinsäuresequenzen und die für ein Protein mit der Aktivität einer Stärkesynthase II codierenden Nucleinsäuresequenzen auf verschiedenen Nucleinsäuremolekülen vorliegen. Die für ein Protein mit der Aktivität einer Glucan-Wasser-Dikinase codierenden Nucleinsäuresequenzen und die für ein Protein mit der Aktivität einer Stärkesynthase II codierenden Nucleinsäuresequenzen können beispielsweise in einem Vektor, Plasmid oder in linear vorliegenden Nucleinsäuremolekülen gleichzeitig enthalten sein, oder aber Bestandteile von zwei jeweils voneinander getrennten Vektoren, Plasmiden oder linearen Nucleinsäuremolekülen sein.

Liegen die für ein Protein mit der Aktivität einer Glucan-Wasser-Dikinase codierenden Nucleinsäuresequenzen und die für ein Protein mit der Aktivität einer Stärkesynthase II codierenden Nucleinsäuresequenzen in zwei voneinander getrennten Nucleinsäuremolekülen vor, so können sie entweder zeitgleich ("Cotransformation") oder auch nacheinander, d.h. zeitlich aufeinander folgend ("Supertransformation") in das Genom der Pflanzenzelle oder Pflanze eingeführt werden. Die voneinander getrennten Nukleinsäuremoleküle können auch in verschiedene individuelle Pflanzenzellen oder Pflanzen einer Spezies eingeführt werden. Es können dadurch Pflanzenzellen oder Pflanzen erzeugt werden, bei welchen die Aktivität von entweder mindestens einem Protein mit der Aktivität einer Glucan-Wasser-Dikinase oder aber mindestens einem Protein mit der Aktivität einer Stärkesynthase II erhöht ist. Erfindungsgemäße Pflanzen können dann durch anschließendes Kreuzen der Pflanzen, bei welchen die Aktivität eines Proteins mit der Aktivität einer Glucan-Wasser-Dikinase erhöht ist, mit solchen, bei welchen die Aktivität eines Proteins mit der Aktivität einer Stärkesynthase II erhöht ist, hergestellt werden.

Weiterhin können zur Einführung eines fremden Nukleinsäuremoleküls anstelle einer Wildtyp-Pflanzenzelle bzw. Wildtyp-Pflanze, eine Mutantenzelle bzw. eine Mutante, die sich dadurch auszeichnet, dass sie bereits eine erhöhte Aktivität eines Proteins mit der Aktivität einer Glucan-Wasser-Dikinase oder eine erhöhte Aktivität eines Proteins mit der Aktivität einer Stärkesynthase II aufweist, verwendet werden. Bei den Mutanten kann es sich sowohl um spontan (natürlich) auftretende Mutanten, als auch um solche handeln, die durch den gezielten Einsatz von Mutagenen (wie z.B. chemische Agentien, ionisierende Strahlung) oder gentechnischen Verfahren (z.B. T-DNA-activation-tagging, Transposon-activation tagging, *in situ* activation, *in* vivo-Mutagenese) erzeugt wurden.

Erfindungsgemäße Pflanzenzellen oder erfindungsgemäße Pflanzen können daher auch durch Einführung eines fremden Nucleinsäuremoleküls, welches zur Erhöhung der Aktivität eines Proteins mit der Aktivität einer Glucan-Wasser-Dikinase führt, in eine Mutantenzelle oder eine Mutante, die bereits eine erhöhte Aktivität eines Proteins mit der Aktivität einer Stärkesynthase II aufweist, hergestellt werden. Erfindungsgemäße Pflanzenzellen oder erfindungsgemäße Pflanzen können auch durch Einführung eines fremden Nucleinsäuremoleküls, welches zur Erhöhung der Aktivität eines Proteins mit der Aktivität einer Stärkesynthase II führt, in eine Mutantenzelle oder eine Mutante, die bereits eine erhöhte Aktivität eines Proteins mit der Aktivität einer Glucan-Wasser-Dikinase aufweist, hergestellt werden.

Erfindungsgemäße Pflanzenzellen oder erfindungsgemäße Pflanzen können auch erzeugt werden, indem man eine Mutante, bei welcher die Aktivität eines Proteins mit der Aktivität einer Glucan-Wasser-Dikinase erhöht ist, mit einer Pflanze, die aufgrund der Einführung eines fremden Nucleinsäuremoleküls eine erhöhte Aktivität eines Proteins mit der Aktivität einer Stärkesynthase II aufweist, kreuzt. Ebenso ist es möglich, erfindungsgemäße Pflanzenzellen oder erfindungsgemäße Pflanzen zu erzeugen, indem man eine Mutante, bei welcher die Aktivität eines Proteins mit der Aktivität einer Stärkesynthase II erhöht ist, mit einer Pflanze, die aufgrund der Einführung eines fremden Nucleinsäuremoleküls eine erhöhte Aktivität eines Proteins mit der Aktivität einer Glucan-Wasser-Dikinase aufweist, kreuzt.

Man kann erfindungsgemäße Pflanzen auch dadurch erzeugen, dass man eine Mutante, bei welcher die Aktivität eines Proteins mit der Aktivität einer Stärkesynthase II erhöht ist mit einer Mutante, bei welcher die Aktivität eines Proteins mit der Aktivität einer Glucan-Wasser-Dikinase erhöht ist, kreuzt.

Für die Einführung von DNA in eine pflanzliche Wirtszelle steht eine Vielzahl von Techniken zur Verfügung. Diese Techniken umfassen die Transformation pflanzlicher Zellen mit T-DNA unter Verwendung von *Agrobacterium tumefaciens* oder *Agrobacterium rhizogenes* als Transformationsmittel, die Fusion von Protoplasten, die Injektion, die Elektroporation von DNA, die Einbringung der DNA mittels des biolistischen Ansatzes sowie weitere Möglichkeiten. Die Verwendung der Agrobakterien-vermittelten Transformation von Pflanzenzellen ist intensiv untersucht und u.a. in EP 120516; Hoekema, (In: The Binary Plant Vector System Offsetdrukkerij Kanters B.V. Alblasserdam (1985), Chapter V; Fraley et al., Crit. Rev. Plant Sci. 4, 1-46) und bei An et al. (1985, EMBO J. 4, , 277-287) beschrieben worden. Für die Transformation von Kartoffel, siehe z.B. Rocha-Sosa et al.( 1989, EMBO J. 8, 29-33).

Auch die Transformation monocotyler Pflanzen mittels auf *Agrobacterium* Transformation basierender Vektoren wurde beschrieben (1993, Chan et al., Plant Mol. Biol. 22, 491-506; Hiei et al., 1994, Plant J. 6, 271-282; Deng et al, 1990, Science in China 33, 28-34; Wilmink et al., 1992, Plant Cell Reports 11, 76-80; May et al., 1995, Bio/Technology 13, 486-492; Conner und Domisse, 1992, Int. J. Plant Sci. 153, 550-555; Ritchie et al, 1993, Transgenic Res. 2, 252-265). Alternative Methoden zur Transformation von monocotylen Pflanzen sind die Transformation mittels des biolistischen Ansatzes (Wan und Lemaux, 1994, Plant Physiol. 104, 37-48; Vasil et al., 1993, Bio/Technology 11, 1553-1558; Ritala et al., 1994, Plant Mol. Biol. 24, 317-325; Spencer et al., 1990, Theor. Appl. Genet. 79, 625-631), die Protoplastentransformation, die Elektroporation von partiell permeabilisierten Zellen oder die Einbringung von DNA mittels Glasfasern. Insbesondere die Transformation von Mais wird in der Literatur mehrfach beschrieben (vgl. z. B. WO95/06128, EP0513849, EP0465875, EP0292435; Fromm et al., 1990, Biotechnology 8, 833-844; Gordon-Kamm et al., 1990, Plant Cell 2, 603-618; Koziel et al., 1993, Biotechnology 11, 194-200; Moroc et a1.,1990, Theor. Appl. Genet. 80, 721-726). Auch die erfolgreiche Transformation anderer Getreidearten wurde bereits beschrieben, z.B. für Gerste (Wan und Lemaux, s.o.; Ritala et al., s.o.; Krens et al., 1982, Nature 296, 72-74) und für Weizen (Nehra et al., 1994, Plant J. 5, 285-297; Becker et al., 1994, Plant Journal 5, 299-307). Alle vorstehenden Methoden sind im Rahmen der vorliegenden Erfindung geeignet.

Pflanzenzellen und Pflanzen, die durch Einführung eines Proteins mit der Aktivität einer Glucan-Wasser-Dikinase und/oder eines Proteins mit der Aktivität einer Stärkesynthase II genetisch modifiziert sind, lassen sich von Wildtyp-Pflanzenzellen bzw. Wildtyp-Pflanzen unter anderem dadurch unterscheiden, dass sie mindestens ein fremdes Nucleinsäuremolekül aufweisen, das natürlicherweise in Wildtyp-Pflanzenzellen bzw. Wildtyp-Pflanzen nicht vorkommt oder dadurch, dass ein solches Molekül an einem Ort im Genom der erfindungsgemäßen Pflanzenzelle oder im Genom der erfindungsgemäßen Pflanze integriert vorliegt, an dem es bei Wildtyp-Pflanzenzellen bzw. Wildtyp-Pflanzen nicht vorkommt, d.h. in einer anderen genomischen Umgebung. Ferner lassen sich derartige erfindungsgemäße Pflanzenzellen und erfindungsgemäße Pflanzen von Wildtyp-Pflanzenzellen bzw. Wildtyp-Pflanzen dadurch unterscheiden, dass sie mindestens eine Kopie des fremden Nucleinsäuremoleküls stabil integriert in ihr Genom enthalten, gegebenenfalls zusätzlich zu natürlicherweise in den Wildtyp-Pflanzenzellen bzw. Wildtyp-Pflanzen vorkommenden Kopien eines solchen Moleküls. Handelt es sich bei dem (den) in die erfindungsgemäßen Pflanzenzellen oder erfindungsgemäßen Pflanzen eingeführten fremden Nucleinsäuremolekül(en) um zusätzliche Kopien zu bereits natürlicherweise in den Wildtyp-Pflanzenzellen bzw. Wildtyp-Pflanzen vorkommenden Molekülen, so lassen sich die erfindungsgemäßen Pflanzenzellen und die erfindungsgemäßen Pflanzen von Wildtyp-Pflanzenzellen bzw. Wildtyp-Pflanzen insbesondere dadurch unterscheiden, dass diese zusätzliche(n) Kopie(n) an Orten im Genom lokalisiert ist (sind), an denen sie bei Wildtyp-Pflanzenzellen bzw. Wildtyp-Pflanzen nicht vorkommt (vorkommen). Dies lässt sich beispielsweise mit Hilfe einer Southern Blot-Analyse nachprüfen.

Weiterhin lassen sich die erfindungsgemäßen Pflanzenzellen und die erfindungsgemäßen Pflanzen von Wildtyp-Pflanzenzellen bzw. Wildtyp-Pflanzen vorzugsweise durch mindestens eines der folgenden Merkmale unterscheiden: Ist ein eingeführtes fremdes Nucleinsäuremolekül heterolog in bezug auf die Pflanzenzelle oder Pflanze, so weisen die erfindungsgemäßen Pflanzenzellen oder erfindungsgemäßen Pflanzen Transkripte der eingeführten Nucleinsäuremoleküle auf. Diese lassen sich z. B. durch Northern-Blot-Analyse oder durch RT-PCR (Reverse Transcription Polymerase Chain Reaction) nachweisen. Vorzugsweise enthalten die erfindungsgemäßen Pflanzenzellen und die erfindungsgemäßen Pflanzen ein Protein, das durch ein eingeführtes Nucleinsäuremolekül codiert wird. Dies kann z. B. durch immunologische Methoden, insbesondere durch eine Western-Blot-Analyse nachgewiesen werden.

Ist ein eingeführtes fremde Nucleinsäuremolekül homolog in bezug auf die Pflanzenzelle oder Pflanze, können die erfindungsgemäßen Pflanzenzellen und die erfindungsgemäßen Pflanzen von Wildtyp-Pflanzenzellen bzw. Wildtyp-Pflanzen beispielsweise aufgrund der zusätzlichen Expression der eingeführten fremden Nucleinsäuremoleküle unterschieden werden. Die erfindungsgemäßen Pflanzenzellen und die erfindungsgemäßen Pflanzen enthalten vorzugsweise Transkripte der fremden Nucleinsäuremoleküle. Dies kann z. B. durch Northern-Blot-Analyse oder mit Hilfe der so genannten quantitativen RT-PCR nachgewiesen werden.

Bei den erfindungsgemäßen Pflanzen kann es sich prinzipiell um Pflanzen jeder beliebigen Pflanzenspezies handeln, d.h. sowohl um monocotyle als auch dicotyle Pflanzen. Bevorzugt handelt es sich um Nutzpflanzen, d.h. Pflanzen, die vom Menschen kultiviert werden für Zwecke der Ernährung oder für technische, insbesondere industrielle Zwecke.

In einer weiteren Ausführungsform ist die erfindungsgemäße Pflanze, eine Stärke speichernde Pflanze.

Der Begriff "Stärke speichernde Pflanze" meint im Zusammenhang mit der vorliegenden Erfindung alle Pflanzen mit Pflanzenteilen, die eine Speicherstärke enthalten, wie z.B., Mais, Reis, Weizen, Roggen, Hafer, Gerste, Maniok, Kartoffel, Sago, Taro, Mungbohne, Erbse, *Sorghum,* Süßkartoffel.

In einer bevorzugten Ausführungsform betrifft die vorliegende Erfindung erfindungsgemäße stärkespeichernde monocotyle Pflanzen, insbesondere bevorzugt Pflanzen der (systematischen) Familie Poaceae. Besonders bevorzugt handelt es sich dabei um Reis-, Mais- oder Weizenpflanzen.

Der Begriff "Weizenpflanze" meint im Zusammenhang mit der vorliegenden Erfindung Pflanzenspezies der Gattung *Triticum* oder Pflanzen, die aus Kreuzungen mit Pflanzen der Gattung *Triticum* hervorgegangen sind, besonders in der Agrarwirtschaft zu kommerziellen Zwecken angebaute Pflanzenspezies der Gattung *Triticum* bzw. Pflanzen, die aus Kreuzungen mit Pflanzen der Gattung *Triticum* hervorgegangen sind, insbesondere bevorzugt ist *Triticum aestivum.*

Der Begriff "Maispflanze" meint im Zusammenhang mit der vorliegenden Erfindung Pflanzenspezies der Gattung Zea, besonders in der Agrarwirtschaft zu kommerziellen Zwecken angebaute Pflanzenspezies der Gattung Zea, besonders bevorzugt *Zea mais.*

Der Begriff "Reispflanze" meint im Zusammenhang mit der vorliegenden Erfindung Pflanzenspezies der Gattung Oryza, besonders in der Agrarwirtschaft zu kommerziellen Zwecken angebaute Pflanzenspezies der Gattung Oryza, besonders bevorzugt *Oryza sativa.*

In einer weiteren Ausführungsform handelt es sich bei den erfindungsgemäßen Pflanzenzellen und bei den erfindungsgemäßen Pflanzen um transgene Pflanzenzellen bzw. transgene Pflanzen.

Erfindungsgemäße Pflanzenzellen und erfindungsgemäße Pflanzen synthetisieren eine modifizierte Stärke im Vergleich zu Stärke, isoliert aus nicht genetisch modifizierten Wildtyp-Pflanzenzellen bzw. Wildtyp-Pflanzen.

Ein weiterer Gegenstand der vorliegenden Erfindung betrifft daher erfindungsgemäße Pflanzenzellen oder erfindungsgemäße Pflanzen, dadurch charakterisiert, dass sie eine modifizierte Stärke synthetisieren im Vergleich zu Stärke, isoliert aus entsprechenden nicht genetisch modifizierten Wildtyp-Pflanzenzellen bzw. isoliert aus entsprechenden nicht genetisch modifizierten Wildtyp-Pflanzen.

Ferner sind Gegenstand der Erfindung genetisch modifizierte Pflanzen, die erfindungsgemäße Pflanzenzellen enthalten. Derartige Pflanzen können durch Regeneration aus erfindungsgemäßen Pflanzenzellen erzeugt werden.

Die vorliegende Erfindung betrifft auch Vermehrungsmaterial erfindungsgemäßer Pflanzen, enthaltend eine erfindungsgemäße Pflanzenzelle.

Der Begriff "Vermehrungsmaterial" umfasst dabei jene Bestandteile der Pflanze, die geeignet sind zur Erzeugung von Nachkommen auf vegetativem oder sexuellem Weg. Für die vegetative Vermehrung eignen sich beispielsweise Stecklinge, Calluskulturen, Rhizome oder Knollen. Anderes Vermehrungsmaterial umfasst beispielsweise Früchte, Samen, Sämlinge, Protoplasten, Zellkulturen, etc. Besonders bevorzugt handelt es sich bei dem Vermehrungsmaterial um endospermhaltige Samen (Körner).

In einer weiteren Ausführungsform betrifft die vorliegende Erfindung erntebare Pflanzenteile erfindungsgemäßer Pflanzen, wie Früchte, Speicherwurzeln, Wurzeln, Blüten, Knospen, Sprosse oder Stämme, vorzugsweise Samen, Körner oder Knollen, wobei diese erntebaren Teile erfindungsgemäße Pflanzenzellen enthalten.

Stärke, die von erfindungsgemäßen Pflanzenzellen oder von erfindungsgemäßen Pflanzen synthetisiert wird, zeichnet sich im Vergleich zu Stärke, isoliert aus entsprechenden nicht genetisch modifizierten Wildtyp-Pflanzenzellen bzw. im Vergleich zu Stärke, isoliert aus entsprechenden nicht genetisch modifizierten Wildtyp-Pflanzen insbesondere dadurch aus, dass sie ein erhöhtes Heißwasser Quellvermögen aufweist.

Weiterhin betrifft die vorliegende Erfindung auch ein Verfahren zur Herstellung einer genetisch modifizierten Pflanze, worin

a) eine Pflanzenzelle, genetisch modifiziert wird, wobei die genetische Modifikation die folgenden Schritte i und ii in beliebiger Reihenfolge, einzeln oder gleichzeitig umfasst

i) Einführung einer genetischen Modifikation in die Pflanzenzelle, wobei die genetische Modifikation zur Erhöhung der Aktivität eines Proteins mit der Aktivität einer Stärkesynthase II, im Vergleich zu entsprechenden nicht genetisch modifizierten Wildtyp-Pflanzenzellen führt,
ii) Einführung einer genetischen Modifikation in die Pflanzenzelle, wobei die genetische Modifikation zur Erhöhung der Aktivität eines Proteins mit der Aktivität einer Glucan-Wasser-Dikinase, im Vergleich zu entsprechenden nicht genetisch modifizierten Wildtyp-Pflanzenzellen führt

b) aus Pflanzenzellen von Schritt a) eine Pflanze regeneriert wird;
c) gegebenenfalls weitere Pflanzen mit Hilfe der Pflanzen nach Schritt b) erzeugt werden

wobei gegebenenfalls aus Pflanzen gemäß Schritt b) oder c) Pflanzenzellen isoliert werden und die Verfahrensschritte a) bis c) solange wiederholt werden, bis eine Pflanze erzeugt wurde, die ein fremdes Nucleinsäuremolekül, codierend ein Protein mit der Aktivität einer Stärkesynthase II und ein fremdes Nucleinsäuremolekül, codierend ein Protein mit der Aktivität einer Glucan-Wasser-Dikinase aufweist.

In einer bevorzugten Ausführungsform umfasst das erfindungsgemäße Verfahren zur Herstellung einer genetisch modifizierten Pflanze folgende Schritte:

a) eine Pflanzenzelle, genetisch modifiziert wird, wobei die genetische Modifikation die folgenden Schritte i und ii in beliebiger Reihenfolge umfasst oder beliebige Kombinationen der folgenden Schritte i und ii einzeln oder gleichzeitig durchgeführt werden

i) Einführung einer genetischen Modifikation in die Pflanzenzelle, wobei die genetische Modifikation zur Erhöhung der Aktivität eines Proteins mit der Aktivität einer Stärkesynthase II, im Vergleich zu entsprechenden nicht genetisch modifizierten Wildtyp-Pflanzenzellen führt

ii) Einführung einer genetischen Modifikation in die Pflanzenzelle, wobei die genetische Modifikation zur Erhöhung der Aktivität eines Proteins mit der Aktivität einer Glucan-Wasser-Dikinase, im Vergleich zu entsprechenden nicht genetisch modifizierten Wildtyp-Pflanzenzellen führt

b) aus Pflanzenzellen enthaltend die genetische Modifikation gemäß den Schritten

i) a) i
ii) a) ii
iii) a) i und a) ii,

eine Pflanze regeneriert wird

c) in Pflanzenzellen von Pflanzen gemäß Schritt

i) b) i eine genetische Modifikation gemäß Schritt a) ii,
ii) b) ii eine genetische Modifikation gemäß Schritt a) i,

eingeführt und eine Pflanze regeneriert wird

d) gegebenenfalls weitere Pflanzen mit Hilfe der Pflanzen, erhalten nach einem der Schritte b) iii oder c) i oder c) ii erzeugt werden.

Für die laut Schritt a) in die Pflanzenzelle eingeführten genetischen Modifikationen gilt, dass es sich grundsätzlich um jede Art von Modifikation handeln kann, die zur Erhöhung der Aktivität eines Proteins mit der enzymatischen Aktivität einer Stärkesynthase II und/oder die zur Erhöhung der Aktivität eines Proteins mit der enzymatischen Aktivität einer Glucan-Wasser-Dikinase führt.

Die Regeneration der Pflanzen gemäß Schritt b) und gegebenenfalls Schritt c) der erfindungsgemäßen Verfahren kann nach dem Fachmann bekannten Methoden erfolgen (z.B. beschrieben in "Plant Cell Culture Protocols", 1999, edt. by R.D. Hall, Humana Press, ISBN 0-89603-549-2).

Die Erzeugung weiterer Pflanzen (je nach Verfahren gemäß Schritt c) oder Schritt d)) der erfindungsgemäßen Verfahren kann z.B. erfolgen durch vegetative Vermehrung (beispielsweise über Stecklinge, Knollen oder über Calluskultur und Regeneration ganzer Pflanzen) oder durch sexuelle Vermehrung. Die sexuelle Vermehrung findet dabei vorzugsweise kontrolliert statt, d.h. es werden ausgewählte Pflanzen mit bestimmten Eigenschaften miteinander gekreuzt und vermehrt. Die Auswahl erfolgt dabei bevorzugt in der Weise, dass die weiteren Pflanzen (die je nach Verfahren gemäß Schritt c) oder Schritt d) erzeugt werden) die in den vorangegangenen Schritten eingeführten Modifikationen aufweisen.

In erfindungsgemäßen Verfahren zur Herstellung von genetisch modifizierten Pflanzen können die genetischen Modifikationen zur Erzeugung der erfindungsgemäßen genetisch modifizierten Pflanzenzellen gleichzeitig oder in aufeinander folgenden Schritten erfolgen. Es ist dabei unerheblich, ob für aufeinander folgende genetische Modifikationen, die zu einer erhöhten Aktivität eines Proteins mit der enzymatischen Aktivität einer Stärkesynthase II führt, die gleiche Methode verwendet wird, wie für die genetische Modifikation, die zu einer erhöhten Aktivität eines Proteins mit der enzymatischen Aktivität einer Glucan-Wasser-Dikinase führt.

In einer bevorzugten Ausführungsform des erfindungsgemäßen Verfahrens zur Herstellung einer genetisch modifizierten Pflanze folgt auf Schritt b) ein Verfahrensschritt b)-1, worin Pflanzen selektiert werden, die eine erhöhte Aktivität eines Proteins mit der Aktivität einer Stärkesynthase II gemäß Schritt a) i aufweisen und/oder die eine erhöhte Aktivität eines Proteins mit der Aktivität einer Glucan-Wasser-Dikinase gemäß Schritt a) ii aufweisen. Die selektierten Pflanzen werden dann für die weiteren Verfahrensschritte verwendet.

Vorzugsweise werden dabei Pflanzen selektiert, die die genetische Modifikation gemäß Schritt a) i enthalten und eine Erhöhung der Aktivität eines Proteins mit der Aktivität einer Stärkesynthase II aufweisen, die mindestens 6 fach, bevorzugt mindestens 7 fach, besonders bevorzugt mindestens 8 fach, insbesondere bevorzugt mindestens 9 fach und ganz besonders bevorzugt mindestens 10 fach erhöht ist, im Vergleich zu entsprechenden genetisch nicht modifizierten Wildtyp-Pflanzen.

Vorzugsweise werden dabei Pflanzen selektiert, die die genetische Modifikation gemäß Schritt a) ii enthalten und die eine Stärke synthetisieren, die einen Stärkephosphatgehalt aufweist, der mindestens 4 fach, besonders bevorzugt mindestens 5 fach, insbesondere bevorzugt mindestens 6 fach erhöht ist, im Vergleich zu entsprechenden genetisch nicht modifizierten Wildtyp-Pflanzen.

In einer weiteren Ausführungsform des erfindungsgemäßen Verfahrens zur Herstellung einer genetisch modifizierten Pflanze besteht die genetische Modifikation in der Einführung mindestens eines fremden Nucleinsäuremoleküls in das Genom der Pflanzenzelle, wobei das Vorhandensein oder die Expression des/der fremden Nucleinsäuremoleküls/ Nukleinsäuremoleküle zu einer erhöhten Aktivität eines Proteins mit der enzymatischen Aktivität einer Stärkesynthase II und/oder zu einer erhöhten Aktivität eines Proteins mit der enzymatischen Aktivität einer Glucan-Wasser-Dikinase in der Zelle führt/führen.

In einer weiteren Ausführungsform des erfindungsgemäßen Verfahrens zur Herstellung einer genetisch modifizierten Pflanze besteht die genetische Modifikation in der Einführung von mindestens einem fremden Nucleinsäuremolekül in das Genom der Pflanzenzelle, wobei das/die fremde/fremden Nucleinsäuremolekül/Nukleinsäuremoleküle ein Protein mit der enzymatischen Aktivität einer Stärkesynthase II und/oder ein Protein mit der enzymatischen Aktivität einer Glucan-Wasser-Dikinase codierende Sequenzen enthalten.

In einer weiteren Ausführungsform des erfindungsgemäßen Verfahrens zur Herstellung einer erfindungsgemäßen genetisch modifizierten Pflanze, codiert mindestens ein fremdes Nucleinsäuremolekül ein Protein mit der enzymatischen Aktivität einer Glucan-Wasser-Dikinase aus Kartoffel, Weizen, Reis, Mais, Sojabohne, Citrus, *Curcuma* oder *Arabidopsis.* Bevorzugt codiert mindestens ein fremdes Nucleinsäuremolekül ein Protein mit der enzymatischen Aktivität einer Glucan-Wasser-Dikinase aus *Curcuma longa* oder Kartoffel, besonders bevorzugt aus Kartoffel und insbesondere bevorzugt ein Protein, das die unter SEQ ID NO 6 dargestellte Aminosäuresequenz aufweist oder von der unter SEQ ID NO 5 dargestellten Nucleinsäuresequenz codiert wird. Referenzen für Nucleinsäuresequenzen codierend Proteine mit der enzymatischen Aktivität einer Glucan-Wasser-Dikinase aus den genannten Pflanzen sind bereits weiter oben angegeben.

In einer weiteren Ausführungsform des erfindungsgemäßen Verfahrens zur Herstellung einer erfindungsgemäßen genetisch modifizierten Pflanze, codiert mindestens ein fremdes Nucleinsäuremolekül ein Protein mit der enzymatischen Aktivität einer Stärkesynthase II aus Gerste, *Aegilops,* Reis, Mais, Maniok, Bohne, Kartoffel, Erbse, Süßkartoffel, *Arabidopsis,* Taro, *Ostreococcus* oder *Chlamydomonas.* Bevorzugt codiert mindestens ein fremdes Nucleinsäuremolekül ein Protein mit der enzymatischen Aktivität einer Stärkesynthase II aus Weizen. Referenzen für die genannten Nucleinsäuresequenzen codierend Proteine mit der enzymatischen Aktivität einer Stärkesynthase II aus den genannten Pflanzen sind bereits weiter oben angegeben.

Wie oben bereits für zur genetischen Modifikation in eine Pflanzezelle oder Pflanze eingebrachte fremde Nukleinsäuremoleküle beschrieben, kann es sich in Schritt a) des erfindungsgemäßen Verfahrens zur Herstellung einer genetisch modifizierten Pflanze um ein einzelnes Nucleinsäuremolekül oder um mehrere Nucleinsäuremoleküle handeln. So können die fremden Nucleinsäuremoleküle, codierend ein Protein mit der enzymatischen Aktivität einer Stärkesynthase II bzw. codierend ein Protein mit der enzymatischen Aktivität einer Glucan-Wasser-Dikinase zusammen auf einem einzigen Nucleinsäuremolekül vorliegen oder sie können auf getrennten Nucleinsäuremolekülen vorliegen. Liegen die Nucleinsäuremoleküle codierend ein Protein mit der enzymatischen Aktivität einer Stärkesynthase II und codierend ein Protein mit der Aktivität einer Glucan-Wasser-Dikinase auf mehreren Nucleinsäuremolekülen vor, so können diese Nucleinsäuremoleküle gleichzeitig oder in aufeinander folgenden Schritten in eine Pflanzenzelle eingeführt werden.

Weiterhin können zur Einführung eines fremden Nukleinsäuremoleküls bei der Durchführung erfindungsgemäßer Verfahren anstelle einer Wildtyp-Pflanzenzelle bzw. Wildtyp-Pflanze, eine Mutantenzelle bzw. eine Mutante, die sich dadurch auszeichnet, dass sie bereits eine erhöhte Aktivität eines Proteins mit der enzymatischen Aktivität einer Stärkesynthase II oder eine erhöhte Aktivität eines Proteins mit der enzymatischen Aktivität einer Glucan-Wasser-Dikinase aufweist, verwendet werden. Die weiter oben gemachten Angaben zur Verwendung von Mutanten für die Herstellung erfindungsgemäßer Pflanzenzellen oder Pflanzen, sind hier entsprechend anzuwenden.

In einer bevorzugten Ausführungsform betrifft die vorliegende Erfindung erfindungsgemäße Verfahren zur Herstellung einer genetisch modifizierten Pflanze, worin das Nucleinsäuremolekül, codierend ein Protein mit der enzymatischen Aktivität einer Stärkesynthase II ausgewählt ist, aus der Gruppe bestehend aus

a) Nucleinsäuremolekülen, die ein Protein mit der unter SEQ ID NO 6 Aminosäuresequenz codieren;
b) Nucleinsäuremolekülen, die ein Protein mit der Aktivität einer Stärkesynthase II codieren, dessen Aminosäuresequenz mindestens 70%, vorzugsweise von mindestens 80%, bevorzugt von mindestens 90% und insbesondere bevorzugt von mindestens 95% zu der unter SEQ ID NO 6 dargestellten Aminosäuresequenz aufweisen;
c) Nucleinsäuremolekülen, die die unter SEQ ID NO 5 dargestellte Nucleinsäuresequenz oder eine komplementäre Sequenz umfassen;
d) Nucleinsäuremolekülen, welche zu den unter c) beschriebenen Nucleinsäuresequenzen eine Identität von mindestens 70%, vorzugsweise von mindestens 80%, bevorzugt von mindestens 90% und insbesondere bevorzugt von mindestens 95% aufweisen,
e) Nucleinsäuremolekülen, welche mit mindestens einem Strang der unter a) oder c) beschriebenen Nucleinsäuremolekülen unter stringenten Bedingungen hybridisieren;
f) Nucleinsäuremolekülen, deren Nucleotidsequenz aufgrund der Degeneration des genetisches Codes von der Sequenz der unter a), oder c) genannten Nucleinsäuremoleküle abweicht;

g) Nucleinsäuremolekülen, die Fragmente, allelische Varianten und/oder Derivate der unter a), b), c), d), e) oder f) genannten Nucleinsäuremoleküle darstellen,
h) Nucleinsäuremoleküle, codierend ein Protein mit der Aktivität einer Stärkesynthase II, wobei die ein Protein mit der Aktivität einer Stärkesynthase II codierenden Nucleinsäuresequenzen mit regulatorischen Elementen (Promotor) verknüpft sind, die die Transkription in Pflanzenzellen initiieren oder
i) Nucleinsäuremoleküle, nach h), wobei die Promotoren gewebespezifische Promotoren, besonders bevorzugt Promotoren, die die Initiation der Transkription spezifisch in pflanzlichen Endospermzellen initiieren, darstellen.

In einer weiteren bevorzugten Ausführungsform betrifft die vorliegende Erfindung erfindungsgemäße Verfahren zur Herstellung einer genetisch modifizierten Pflanze, worin das Nucleinsäuremolekül, codierend ein Protein mit der enzymatischen Aktivität einer Glucan-Wasser-Dikinase ausgewählt ist, aus der Gruppe bestehend aus

a) Nucleinsäuremolekülen, die ein Protein mit der unter SEQ ID NO 2 oder SEQ ID NO 4 dargestellten Aminosäuresequenz codieren;
b) Nucleinsäuremolekülen, die ein Protein codieren, das die Aktivität einer Glucan-Wasser-Dikinase aufweist und dessen Sequenz eine Identität von mindestens 70%, vorzugsweise von mindestens 80%, bevorzugt von mindestens 90% und insbesondere bevorzugt von mindestens 95% zu der unter SEQ ID NO 2 oder SEQ ID NO 4 dargestellten Aminosäuresequenz aufweisen;
c) Nucleinsäuremolekülen, die die unter SEQ ID NO 1 oder SEQ ID NO 3 dargestellte Nucleinsäuresequenz oder eine komplementäre Sequenz umfassen;
d) Nucleinsäuremolekülen, welche zu den unter c) beschriebenen Nucleinsäuresequenzen eine Identität von mindestens 70%, vorzugsweise von mindestens 80%, bevorzugt von mindestens 90% und insbesondere bevorzugt von mindestens 95% aufweisen,
e) Nucleinsäuremolekülen, welche mit mindestens einem Strang der unter a) oder c) beschriebenen Nucleinsäuremolekülen unter stringenten Bedingungen hybridisieren;
f) Nucleinsäuremolekülen, deren Nucleotidsequenz aufgrund der Degeneration des genetischen Codes von der Sequenz der unter a), oder c) genannten Nucleinsäuremoleküle abweicht;
g) Nucleinsäuremolekülen, die Fragmente, allelische Varianten und/oder Derivate der unter a), b), c), d), e) oder f) genannten Nucleinsäuremoleküle darstellen,
h) Nucleinsäuremoleküle, codierend ein Protein mit der Aktivität einer Glucan-Wasser-Dikinase, wobei die ein Protein mit der Aktivität einer Glucan-Wasser-Dikinase codierenden Nucleinsäuresequenzen mit regulatorischen Elementen (Promotor) verknüpft sind, die die Transkription in Pflanzenzellen initiieren oder
i) Nucleinsäuremoleküle, nach h), wobei die Promotoren gewebespezifische Promotoren, besonders bevorzugt Promotoren, die die Initiation der Transkription spezifisch in pflanzlichen Endospermzellen initiieren, darstellen.

Unter dem Begriff "Identität" soll im Zusammenhang mit der vorliegenden Erfindung die Anzahl der übereinstimmenden Aminosäuren/Nucleotide (Identität) mit anderen Proteinen/Nudeinsäuren, ausgedrückt in Prozent verstanden werden. Bevorzugt wird die Identität betreffend ein Protein mit der Aktivität einer Stärkesynthase II durch Vergleiche der unter SEQ ID NO 6 angegebenen Aminosäuresequenz bzw. die Identität betreffend ein Nucleinsäuremolekül codierend ein Protein mit der Aktivität einer Stärkesynthase II durch Vergleiche der unter SEQ ID NO 5 angegebenen Nucleinsäuresequenz und die Identität betreffend ein Protein mit der Aktivität einer Glucan-Wasser-Dikinase durch Vergleiche der unter SEQ ID NO 2 oder SEQ ID NO 4 angegebenen Aminosäuresequenz bzw. die Identität betreffend ein Nucleinsäuremolekül codierend ein Protein mit der Aktivität einer Glucan-Wasser-Dikinase durch Vergleiche der unter SEQ ID NO 1 oder SEQ ID NO 3 angegebenen Nucleinsäuresequenz zu anderen Proteinen/Nudeinsäuren mit Hilfe von Computerprogrammen ermittelt. Weisen Sequenzen, die miteinander verglichen werden, unterschiedliche Längen auf, ist die Identität so zu ermitteln, dass die Anzahl an Aminosäuren/Nucleotiden, welche die kürzere Sequenz mit der längeren Sequenz gemeinsam hat, den prozentualen Anteil der Identität bestimmt. Vorzugsweise wird die Identität mittels des bekannten und der Öffentlichkeit zur Verfügung stehenden Computerprogramms ClustalW (Thompson et al., Nucleic Acids Research 22 (1994), 4673-4680) ermittelt. ClustalW wird öffentlich zur Verfügung gestellt von Julie Thompson (Thompson@EMBL-Heidelberg.DE) und Toby Gibson (Gibson@EMBL-Heidelberg.DE), European Molecular Biology Laboratory, Meyerhofstrasse 1, D 69117 Heidelberg, Germany. ClustalW kann ebenfalls von verschiedenen Internetseiten, u.a. beim IGBMC (Institut de Génétique et de Biologie Moléculaire et Cellulaire, B.P.163, 67404 Illkirch Cedex, France; ftp://ftp-igbmc.u-strasbg.fr/pub/) und beim EBI (ftp://ftp.ebi.ac.uk/pub/software/) sowie bei allen gespiegelten Internetseiten des EBI (European Bioinformatics Institute, Wellcome Trust Genome Campus, Hinxton, Cambridge CB10 1SD, UK) heruntergeladen werden.
Vorzugsweise wird das ClustalW Computerprogramm der Version 1.8 benutzt, um die Identität zwischen im Rahmen der vorliegenden Erfindung beschriebenen Proteinen und anderen Proteinen zu bestimmen. Dabei sind folgende Parameter einzustellen: KTUPLE=1, TOPDIAG=5, WINDOW=5, PAIRGAP=3, GAPOPEN=10, GAPEXTEND=0.05, GAPDIST=8, MAXDIV=40, MATRIX=GONNET, ENDGAPS(OFF), NOPGAP, NOHGAP.

Vorzugsweise wird das ClustalW Computerprogramm der Version 1.8 benutzt, um die Identität zwischen z.B. der Nucleotidsequenz der im Rahmen der vorliegenden Erfindung beschriebenen Nucleinsäuremoleküle und der Nucleotidsequenz von anderen Nucleinsäuremolekülen zu bestimmen. Dabei sind folgende Parameter einzustellen: KTUPLE=2, TOPDIAGS=4, PAIRGAP=5, DNAMATRIX:IUB, GAPOPEN=10, GAPEXT=5, MAXDIV=40, TRANSITIONS: unweighted.

Identität bedeutet ferner, dass funktionelle und/oder strukturelle Äquivalenz zwischen den betreffenden Nucleinsäuremolekülen oder den durch sie codierten Proteinen, besteht. Bei den Nucleinsäuremolekülen, die homolog zu den oben beschriebenen Molekülen sind und Derivate dieser Moleküle darstellen, handelt es sich in der Regel um Variationen dieser Moleküle, die Modifikationen darstellen, die dieselbe biologische Funktion ausüben. Es kann sich dabei sowohl um natürlicherweise auftretende Variationen handeln, beispielsweise um Sequenzen aus anderen Spezies oder um Mutationen, wobei diese Mutationen auf natürliche Weise aufgetreten sein können oder durch gezielte Mutagenese eingeführt wurden. Ferner kann es sich bei den Variationen um synthetisch hergestellte Sequenzen handeln. Bei den allelischen Varianten kann es sich sowohl um natürlich auftretende Varianten handeln, als auch um synthetisch hergestellte oder durch rekombinante DNA-Techniken erzeugte Varianten. Eine spezielle Form von Derivaten stellen z.B. Nucleinsäuremoleküle dar, die auf Grund der Degeneration des genetischen Codes von im Rahmen der vorliegenden Erfindung beschriebenen Nucleinsäuremolekülen abweichen.

Der Begriff "Hybridisierung" bedeutet im Rahmen der vorliegenden Erfindung eine Hybridisierung unter konventionellen Hybridisierungsbedingungen, vorzugsweise unter stringenten Bedingungen, wie sie beispielsweise in Sambrok et al., (Molecular Cloning, A Laboratory Manual, 3rd edition (2001) Cold Spring Harbour Laboratory Press, Cold Spring Harbour, NY. ISBN: 0879695773) beschrieben sind. Besonders bevorzugt bedeutet "hybridisieren" eine Hybridisierung unter den folgenden Bedingungen:

Hybridisierungspuffer:
2xSSC; 10xDenhardt-Lösung (Fikoll 400+PEG+BSA; Verhältnis 1:1:1); 0,1% SDS; 5 mM EDTA; 50 mM Na2HPO4; 250 $\mu$g/ml Heringssperma DNA; 50 $\mu$g/ml tRNA; oder
25 M Natriumphoshphatpuffer pH 7,2; 1 mM EDTA; 7% SDS
Hybridisierungstemperatur:
T=65 bis 68°C
Waschpuffer: 0,1xSSC; 0,1% SDS
Waschtemperatur: T=65 bis 68°C.

Nucleinsäuremoleküle, die mit den genannten Molekülen hybridisieren können z.B. aus genomischen oder aus cDNA-Bibliotheken isoliert werden. Die Identifizierung und Isolierung derartiger Nucleinsäuremoleküle kann dabei unter Verwendung der genannten Nucleinsäuremoleküle oder Teile dieser Moleküle bzw. der reversen Komplemente dieser Moleküle erfolgen, z.B. mittels Hybridisierung nach Standardverfahren oder durch Amplifikation mittels PCR.

Als Hybridisierungsprobe zur Isolierung einer Nucleinsäuresequenz, codierend ein Protein mit der Aktivität einer Stärkesynthase II oder mit der Aktivität einer Glucan-Wasser-Dikinase können z.B. Nucleinsäuremoleküle verwendet werden, die exakt die oder im Wesentlichen die unter SEQ ID NO 5 (Stärkesynthase II) oder unter SEQ ID NO 1 bzw. SEQ ID NO 3 (Glucan-Wasser-Dikinase) angegebene Nucleotidsequenz oder Teile dieser Sequenzen aufweisen.

Bei den als Hybridisierungsprobe verwendeten Fragmenten kann es sich auch um synthetische Fragmente oder Oligonucleotide handeln, die mit Hilfe der gängigen Synthesetechniken hergestellt wurden und deren Sequenz im wesentlichen mit der eines im Rahmen der vorliegenden Erfindung beschriebenen Nucleinsäuremoleküls übereinstimmt. Hat man Gene identifiziert und isoliert, die mit den im Rahmen der vorliegenden Erfindung beschriebenen Nucleinsäuresequenzen hybridisieren, sollte eine Bestimmung der Sequenz und eine Analyse der Eigenschaften der von dieser Sequenz codierten Proteine erfolgen, um festzustellen, ob es sich um Proteine handelt, die die Aktivität einer Stärkesynthase II bzw. die Aktivität einer Glucan-Wasser-Dikinase aufweisen.

Die mit den im Rahmen der vorliegenden Erfindung beschriebenen Nucleinsäuremolekülen hybridisierenden Moleküle umfassen insbesondere Fragmente, Derivate und allelische Varianten der genannten Nucleinsäuremoleküle. Der Begriff "Derivat" bedeutet im Zusammenhang mit der vorliegenden Erfindung, dass die Sequenzen dieser Moleküle sich von den Sequenzen der oben beschriebenen Nucleinsäuremoleküle an einer oder mehreren Positionen unterscheiden und einen hohen Grad an Identität zu diesen Sequenzen aufweisen. Die Abweichungen zu den oben beschriebenen Nucleinsäuremolekülen können dabei z.B. durch Deletion, Addition, Substitution, Insertion oder Rekombination entstanden sein.

Zur Expression von erfindungsgemäßen Nucleinsäuremolekülen, die ein Protein mit der Aktivität einer Stärkesynthase II und/oder ein Protein mit der Aktivität einer Glucan-Wasser-Dikinase codieren, werden diese vorzugsweise mit regulatorischen DNA-Sequenzen verknüpft, die die Transkription in pflanzlichen Zellen gewährleisten. Hierzu zählen insbesondere Promotoren. Generell kommt für die Expression jeder in pflanzlichen Zellen aktive Promotor in Frage.

Der Promotor kann dabei so gewählt sein, dass die Expression konstitutiv erfolgt oder nur in einem bestimmten Gewebe, zu einem bestimmten Zeitpunkt der Pflanzenentwicklung oder zu einem durch äußere Einflüsse determinierten Zeitpunkt. Sowohl in bezug auf die Pflanze als auch in bezug auf das Nucleinsäuremolekül kann der Promotor homolog oder heterolog sein.

Geeignete Promotoren sind z.B. der Promotor der 35S RNA des Cauliflower Mosaic Virus und der Ubiquitin-Promotor aus Mais für eine konstitutive Expression, der Patatingen-Promotor B33 (Rocha-Sosa et al., EMBO J. 8 (1989), 23-29) für eine knollenspezifische Expression in Kartoffeln oder ein Promotor, der eine Expression lediglich in photosynthetisch aktiven Geweben sicherstellt, z.B. der ST-LS1-Promotor (Stockhaus et al., Proc. Natl. Acad. Sci. USA 84 (1987), 7943-7947; Stockhaus et al., EMBO J. 8 (1989), 2445-2451) oder für eine endosperm-spezifische Expression der HMG-Promotor aus Weizen, der USP-Promotor, der Phaseolinpromotor, Promotoren von Zein-Genen aus Mais (Pedersen et al., Cell 29 (1982), 1015-1026; Quatroccio et al., Plant Mol. Biol. 15 (1990), 81-93), ein Glutelin-Promotor (Leisy et al., Plant Mol. Biol. 14 (1990), 41-50; Zheng et al., Plant J. 4 (1993), 357-366; Yoshihara et al., FEBS Lett. 383 (1996), 213-218), ein Globulin Promotor (Nakase et al., 1996, Gene 170(2), 223-226), ein Prolamin Promotor (Qu und Takaiwa, 2004, Plant Biotechnology Journal 2(2), 113-125) oder ein Shrunken-1 Promotor (Werr et al., EMBO J. 4 (1985), 1373-1380). Es können jedoch auch Promotoren verwendet werden, die nur zu einem durch äußere Einflüsse determinierten Zeitpunkt aktiviert werden (siehe beispielsweise WO 9307279). Von Interesse können auch Promotoren von heat-shock Proteinen sein, die eine einfache Induktion erlauben. Ferner können samenspezifische Promotoren verwendet werden, wie z.B. der USP-Promoter aus *Vicia faba,* der eine samenspezifische Expression in *Vicia faba* und anderen Pflanzen gewährleistet (Fiedler et al., Plant Mol. Biol. 22 (1993), 669-679; Bäumlein et al., Mol. Gen. Genet. 225 (1991), 459-467).

Ferner kann eine Terminationssequenz (Polyandenylierungssignal) vorhanden sein, die der Addition eines Poly-A-Schwanzes an das Transkript dient. Dem Poly-A-Schwanz wird eine Funktion bei der Stabilisierung der Transkripte beigemessen. Derartige Elemente sind in der Literatur beschrieben (vgl. Gielen et al., EMBO J. 8 (1989), 23-29) und sind beliebig austauschbar.

Es können auch Intronsequenzen zwischen dem Promotor und der codierenden Region vorhanden sein. Solche Intronsequenzen können zur Stabilität der Expression und zu einer erhöhten Expression in Pflanzen führen (Callis et al., 1987, Genes Devel. 1, 1183-1200; Luehrsen, and Walbot, 1991, Mol. Gen. Genet. 225, 81-93; Rethmeier, et al., 1997; Plant Journal. 12(4):895-899; Rose and Beliakoff, 2000, Plant Physiol. 122 (2), 535-542; Vasil et al., 1989, Plant Physiol. 91, 1575-1579; XU et al., 2003, Science in China Series C Vol.46 No.6, 561-569). Geeignete Intronsequenzen sind beispielsweise das erste Intron des sh1-Gens aus Mais, das erste Intron des Poly-Ubiquitin Gens 1 aus Mais, das erste Intron des EPSPS Gens aus Reis oder eines der beiden ersten Introns des PAT1 Gens aus *Arabidopsis.*

Eine weitere Ausführungsform der vorliegenden Erfindung betrifft ein Verfahren zur Herstellung einer erfindungsgemäßen genetisch modifizierten Pflanze, worin

a) eine Pflanzenzelle genetisch modifiziert wird, wobei die genetische Modifikation zur Erhöhung der Aktivität eines Proteins mit der Aktivität einer Stärkesynthase II im Vergleich zu entsprechenden nicht genetisch modifizierten Wildtyp-Pflanzenzellen führt;
b) aus Pflanzenzellen von Schritt a) eine Pflanze regeneriert wird;
c) gegebenenfalls weitere Pflanzen mit Hilfe der Pflanzen nach Schritt b) erzeugt werden und
d) Pflanzen erhalten nach Schritt b) oder c) mit einer Pflanze gekreuzt werden, die eine Erhöhung der Aktivität eines Proteins mit der Aktivität einer Glucan-Wasser-Dikinase aufweist, im Vergleich zu entsprechenden nicht genetisch modifizierten Wildtyp-Pflanzenzellen.

Eine weitere Ausführungsform der vorliegenden Erfindung betrifft ein Verfahren zur Herstellung einer erfindungsgemäßen genetisch modifizierten Pflanze, worin

a) eine Pflanzenzelle genetisch modifiziert wird, wobei die genetische Modifikation zur Erhöhung der enzymatischen Aktivität Proteins mit der Aktivität einer Glucan-Wasser-Dikinase im Vergleich zu entsprechenden nicht genetisch modifizierten Wildtyp-Pflanzenzellen führt;
b) aus Pflanzenzellen von Schritt a) eine Pflanze regeneriert wird;
c) gegebenenfalls weitere Pflanzen mit Hilfe der Pflanzen nach Schritt b) erzeugt werden und
d) Pflanzen erhalten nach Schritt b) oder c) mit einer Pflanze gekreuzt werden, die eine Erhöhung der enzymatischen Aktivität eines Proteins mit der Aktivität einer Stärkesynthase II aufweist, im Vergleich zu entsprechenden nicht genetisch modifizierten Wildtyp-Pflanzenzellen.

In den beiden letztgenannten Verfahren zur Herstellung einer genetisch modifizierten Pflanze können die Pflanzen nach Schritt a) wie bereits oben beschrieben, genetisch modifiziert werden. Die Regeneration von Pflanzen nach Schritt b) und die Erzeugung weiterer Pflanzen nach Schritt c) wurden ebenfalls bereits weiter oben dargestellt.

Eine Pflanze, die nach Schritt d) der beiden letztgenannten Ausführungsformen mit Pflanzen oder Nachkommen der Pflanzen, erhalten aus Schritt b) oder c), gekreuzt wird, kann jede Pflanze sein, die eine erhöhte Aktivität eines Proteins mit der Aktivität einer Stärkesynthase II bzw. eine erhöhte Aktivität eines Proteins mit der Aktivität einer Glucan-Wasser-Dikinase aufweist, im Vergleich zu entsprechenden Wildtyp-Pflanzen. Die Erhöhung der Aktivität eines Proteins mit der Aktivität einer Stärkesynthase II bzw. eines Proteins mit der Aktivität einer Glucan-Wasser-Dikinase kann dabei durch jede Modifikation hervorgerufen sein, die zu einer Erhöhung der Aktivität der betreffenden Proteine in den entsprechenden Pflanzen führt. Es kann sich bei diesen Pflanzen um Mutanten oder mittels gentechnischer Methoden modifizierte Pflanzen handeln. Bei den Mutanten kann es sich sowohl um spontan (natürlich) auftretende Mutanten, als auch um solche handeln, die durch den gezielten Einsatz von Mutagenen (wie z.B. chemische Agentien, ionisierende Strahlung) oder gentechnischen Verfahren (z.B. Transposon activation tagging, T-DNA activation tagging, *in vivo*-Mutagenese) erzeugt wurden. Bevorzugt handelt es sich bei den durch gentechnische Verfahren erzeugten Pflanzen um mittels Insertionsmutagenese hergestellte Mutanten, besonders bevorzugt um genetisch modifizierte Pflanzen, die ein fremdes Nucleinsäuremolekül exprimieren, insbesondere bevorzugt um genetisch modifizierte Pflanzen, bei welchen das fremde Nucleinsäuremolekül ein Protein mit der Aktivität einer Stärkesynthase II bzw. ein Proteins mit der Aktivität einer Glucan-Wasser-Dikinase codiert.

Bevorzugt werden für die Kreuzung in den beiden letztgenannten erfindungsgemäßen Verfahren Pflanzen verwendet, die eine Aktivität eines Proteins mit der Aktivität einer Stärkesynthase II aufweisen, die mindestens 6 fach, bevorzugt mindestens 7 fach, besonders bevorzugt mindestens 8 fach, insbesondere bevorzugt mindestens 9 fach und ganz besonders bevorzugt mindestens 10 fach erhöht ist, im Vergleich zu entsprechenden genetisch nicht modifizierten Wildtyp-Pflanzen.

Betreffend Pflanzen, die eine erhöhte Aktivität eines Proteins mit der Aktivität einer Glucan-Wasser-Dikinase aufweisen, werden für die Kreuzung in den beiden letztgenannten erfindungsgemäßen Verfahren vorzugsweise Pflanzen verwendet, die eine Stärke synthetisieren, die einen Stärkephosphatgehalt aufweist, der mindestens 4 fach, besonders bevorzugt mindestens 5 fach, insbesondere bevorzugt mindestens 6 fach erhöht ist, im Vergleich zu entsprechenden genetisch nicht modifizierten Wildtyp-Pflanzen.

In einer bevorzugten Ausführungsform werden erfindungsgemäße Verfahren zur Herstellung einer genetisch modifizierten Pflanze zur Herstellung von erfindungsgemäßen Pflanzen oder von Pflanzen, die Eigenschaften erfindungsgemäßer Pflanzen aufweisen, verwendet.

Die vorliegende Erfindung betrifft auch die durch erfindungsgemäße Verfahren erhältlichen Pflanzen.

Es wurde überraschenderweise gefunden, dass erfindungsgemäße Pflanzenzellen und erfindungsgemäße Pflanzen, die eine erhöhte Aktivität eines Proteins mit der Aktivität einer Stärkesynthase II und eine erhöhte Aktivität eines Proteins mit der Aktivität einer Glucan-Wasser-Dikinase aufweisen, eine modifizierte Stärke synthetisieren. Insbesondere die Tatsache, dass Stärke, synthetisiert von erfindungsgemäßen Pflanzenzellen oder erfindungsgemäßen Pflanzen ein erhöhtes Heißwasser Quellvermögen aufweist, war überraschend. Das erhöhte Heißwasser Quellvermögen von Stärken, isolierbar aus erfindungsgemäßen Pflanzenzellen und erfindungsgemäßen Pflanzen verleiht den Stärken Eigenschaften, die sie für bestimmte Anwendungen besser geeignet machen, als herkömmliche Stärken. Wird Stärke z.B. als Dickungsmittel eingesetzt, so führt das erhöhte Heißwasser Quellvermögen der Stärke dazu, dass deutlich weniger Stärke eingesetzt werden muss, um eine gleiche Dickungsleistung zu erzielen. Dieses hat zur Folge, dass z.B. der Kaloriengehalt von mit Stärke angedickten Lebensmitteln reduziert wird.

Ein weiterer Gegenstand der vorliegenden Erfindung betrifft modifizierte Stärke, dadurch gekennzeichnet, dass sie ein erhöhtes Heißwasser Quellvermögen aufweist. Besonders bevorzugt ist das Heißwasser Quellvermögen von erfindungsgemäßer modifizierter Stärke um mindestens den Faktor 2, insbesondere um mindestens den Faktor 3 und ganz besonders bevorzugt um mindestens den Faktor 4 erhöht, im Vergleich zu Stärke, isoliert aus entsprechenden nicht genetisch modifizierten Wildtyp-Pflanzezellen bzw. isoliert aus entsprechenden nicht genetisch modifizierten Wildtyp-Pflanzen.

Methoden zur Ermittlung des Heißwasser Quellvermögens sind dem Fachmann bekannt und in der Literatur beschrieben (z.B. Leach et al., 1959, Cereal Chemistry 36, 534-544). Eine im Zusammenhang mit der vorliegenden Erfindung bevorzugt zu verwendende Methode für die Bestimmung des Heißwasser Quellvermögens ist unter Allgemeine Methoden, Punkt 1 beschrieben.

Bevorzugt betrifft die vorliegende Erfindung modifizierte Stärke dadurch gekennzeichnet, dass sie ein Heißwasser Quellvermögen von mindestens 110 g/g, bevorzugt von mindestens 115 g/g, besonders bevorzugt von mindestens 120 g/g und insbesondere bevorzugt von mindestens 125 g/g aufweist. Vorzugsweise weist die modifizierte Stärke ein Heißwasser Quellvermögen von maximal 350 g/g, besonders bevorzugt von maximal 300 g/g, insbesondere bevorzugt von maximal 250 g/g und speziell bevorzugt von maximal 200 g/g auf.

Ein weiterer Gegenstand der vorliegenden Erfindung betrifft modifizierte Stärke, isoliert aus einer monocotylen Pflanzenzelle oder aus einer monocotylen Pflanze, dadurch charakterisiert, dass sie ein Heißwasser Quellvermögen von mindestens 60 g/g, bevorzugt von mindestens 75 g/g, besonders bevorzugt von mindestens 90 g/g, insbesondere bevorzugt von mindestens 105 g/g und im speziellen bevorzugt von mindestens 120 g/g aufweist. Vorzugsweise weist die modifizierte Stärke, isoliert aus einer monocotylen Pflanzenzelle oder monocotylen Pflanze, ein Heißwasser Quellvermögen von maximal 250 g/g, besonders bevorzugt von maximal 200 g/g, insbesondere bevorzugt von maximal 175 g/g und speziell bevorzugt von maximal 150 g/g auf.

Ein weiterer Gegenstand der vorliegenden Erfindung betrifft modifizierte Stärke, isoliert aus Reispflanzenzellen oder Reispflanzen, dadurch charakterisiert, dass sie ein Heißwasser Quellvermögen von mindestens 65 g/g, bevorzugt von mindestens 80 g/g, besonders bevorzugt von mindestens 100 g/g, insbesondere bevorzugt von mindestens 115 g/g und im speziellen bevorzugt von mindestens 125 g/g aufweist. Vorzugsweise weist die modifizierte Stärke, isoliert aus einer Reispflanzenzelle oder Reispflanze, ein Heißwasser Quellvermögen von maximal 250 g/g, besonders bevorzugt von maximal 200 g/g, insbesondere bevorzugt von maximal 175 g/g und speziell bevorzugt von maximal 150 g/g auf.

Ein weiterer bevorzugter Gegenstand der vorliegenden Erfindung betrifft modifizierte Stärke, isoliert aus Maispflanzenzellen oder Maispflanzen, dadurch charakterisiert, dass sie ein Heißwasser Quellvermögen von mindestens 40 g/g, bevorzugt von mindestens 55 g/g aufweist.

Ein weiterer bevorzugter Gegenstand der vorliegenden Erfindung betrifft modifizierte Stärke, isoliert aus Weizenpflanzenzellen oder Weizenpflanzen, dadurch charakterisiert, dass sie ein Heißwasser Quellvermögen von mindestens 35 g/g, bevorzugt von mindestens 50 g/g aufweist.

Stärke, synthetisiert von erfindungsgemäßen genetisch modifizierten Pflanzenzellen oder erfindungsgemäßen genetisch modifizierten Pflanzen weist vorzugsweise einen erhöhten Stärkephosphatgehalt auf. Der Stärkephosphatgehalt von Stärke, isoliert aus erfindungsgemäßen Pflanzenzellen oder erfindungsgemäßen Pflanzen ist dabei deutlich höher, als der Stärkephosphatgehalt, den man nach Kreuzung aus der Summe der Phosphatgehalte der betreffenden Elternpflanzen erwarten würde.

Ein bevorzugter Gegenstand der vorliegenden Erfindung betrifft daher erfindungsgemäße modifizierte Stärke, die einen erhöhten Stärkephosphatgehalt aufweist, im Vergleich zu Stärke, isoliert aus entsprechenden nicht genetisch modifizierten Wildtyp-Pflanzenzellen oder entsprechenden nicht genetisch modifizierten Wildtyp-Pflanzen. Vorzugsweise ist der Stärkephosphatgehalt von erfindungsgemäßer Stärke mindestens 10 fach, besonders bevorzugt mindestens 15 fach, insbesondere bevorzugt mindestens 20 fach und ganz besonders bevorzugt mindestens 25 fach erhöht, im Vergleich zu Stärke, isoliert aus entsprechenden nicht genetisch modifizierten Wildtyp-Pflanzenzellen, bzw. isoliert aus entsprechenden nicht genetisch modifizierten Wildtyp-Pflanzen.

Vorzugsweise weist erfindungsgemäße modifizierte Stärke, mindestens 10 fach mehr, besonders bevorzugt mindestens 15 fach mehr, insbesondere bevorzugt mindestens 20 fach mehr und ganz besonders bevorzugt mindestens 25 fach mehr Stärkephosphat in C6-Position der Glucosemoleküle der Stärke auf, als Stärke, isoliert aus entsprechenden Wildtyp-Pflanzenzellen bzw. isoliert aus entsprechenden Wildtyp-Pflanzen.

Die Menge des in C6-Position der Glucosemoleküle gebundenen Stärkephosphates kann mit dem Fachmann bekannten Methoden wie z.B. photometrisch mittels gekoppelten enzymatischen Test oder mittels $^{31}$P-NMR nach der bei Kasemusuwan und Jane (1996, Cereal Chemistry 73, 702-707) beschriebenen Methode ermittelt werden. Bevorzugt wird im Zusammenhang mit der vorliegenden Erfindung die Menge an in C6-Position der Glucosemoleküle gebundenem Stärkephosphat mit der unter Allgemeine Methoden, Punkt 2 beschriebenen Methode ermittelt.

Ein weiterer bevorzugter Gegenstand der vorliegenden Erfindung betrifft erfindungsgemäße modifizierte Stärke, dadurch charakterisiert, dass sie aus einer monocotylen Pflanzenzelle oder aus einer monocotylen Pflanze isoliert wurde und einen in C6-Position der Glucosemoleküle der Stäke gebundenen Stärkephosphatgehalt von mindestens 11 nmol pro mg Stärke, besonders bevorzugt von mindestens 12 nmol pro mg Stärke aufweist. Insbesondere bevorzugt handelt es sich bei dieser erfindungsgemäßen modifizierten Stärke, um Mais-, Reis- oder Weizenstärke.

In einer weiteren Ausführungsform der vorliegenden Erfindung handelt es sich bei den erfindungsgemäßen modifizierten Stärken um native Stärken.

Der Begriff "native Stärke" bedeutet im Zusammenhang mit der vorliegenden Erfindung, dass die Stärke nach dem Fachmann bekannten Methoden aus erfindungsgemäßen Pflanzen, erfindungsgemäßem erntebaren Pflanzenteilen, erfindungsgemäßen Stärke speichernden Teilen oder erfindungsgemäßem Vermehrungsmaterial von Pflanzen isoliert wird.

Die vorliegende Erfindung betrifft auch erfindungsgemäße modifizierte Stärke, erhältlich aus erfindungsgemäßen Pflanzenzellen oder erfindungsgemäßen Pflanzen, aus erfindungsgemäßem Vermehrungsmaterial oder aus erfindungsgemäßen erntebaren Pflanzenteilen, oder erhältlich aus Pflanzen, die mit einem erfindungsgemäßen Verfahren zur Herstellung einer genetisch modifizierten Pflanze hergestellt wurden.

Pflanzenzellen oder Pflanzen, die eine erfindungsgemäße modifizierte Stärke synthetisieren, sind ebenfalls Gegenstand der vorliegenden Erfindung.

Die vorliegende Erfindung betrifft ferner ein Verfahren zur Herstellung einer modifizierten Stärke, umfassend den Schritt der Extraktion der Stärke aus einer erfindungsgemäßen Pflanzenzelle oder einer erfindungsgemäßen Pflanze, aus erfindungsgemäßem Vermehrungsmaterial einer solchen Pflanze und/oder aus erfindungsgemäßen erntebaren Pflanzenteilen einer solchen Pflanze, vorzugsweise aus erfindungsgemäßen Stärke speichernden Teilen einer solchen Pflanze. Vorzugsweise umfasst ein solches Verfahren auch den Schritt des Erntens der kultivierten Pflanzen bzw.

Pflanzenteile und/oder des Vermehrungsmaterials dieser Pflanzen vor der Extraktion der Stärke und besonders bevorzugt ferner den Schritt der Kultivierung erfindungsgemäßer Pflanzen vor dem Ernten.

Verfahren zur Extraktion der Stärke aus Pflanzen oder von Stärke speichernden Teilen von Pflanzen sind dem Fachmann bekannt. Weiterhin sind Verfahren zur Extraktion der Stärke aus verschiedenen Stärke speichernden Pflanzen beschrieben, z. B. in Starch: Chemistry and Technology (Hrsg.: Whistler, BeMiller und Paschall (1994), 2. Ausgabe, Academic Press Inc. London Ltd; ISBN 0-12-746270-8; siehe z.B. Kapitel XII, Seite 412-468: Mais und Sorghum Stärken: Herstellung; von Watson; Kapitel XIII, Seite 469-479: Tapioca, Arrowroot und Sago Stärken: Herstellung; von Corbishley und Miller; Kapitel XIV, Seite 479-490: Kartoffelstärke: Herstellung und Verwendungen; von Mitch; Kapitel XV, Seite 491 bis 506: Weizenstärke: Herstellung, Modifizierung und Verwendungen; von Knight und Oson; und Kapitel XVI, Seite 507 bis 528: Reisstärke: Herstellung und Verwendungen; von Rohmer und Klem; Maisstärke: Eckhoff et al., Cereal Chem. 73 (1996), 54-57, die Extraktion von Maisstärke im industriellen Maßstab wird in der Regel durch das so genannte "wet milling" erreicht.). Vorrichtungen, die für gewöhnlich bei Verfahren zur Extraktion von Stärke von Pflanzenmaterial verwendet werden, sind Separatoren, Dekanter, Hydrocyclone, Sprühtrockner und Wirbelschichttrockner.

Unter dem Begriff "Stärke speichernde Teile" sollen im Zusammenhang mit der vorliegenden Erfindung solche Teile einer Pflanze verstanden werden, in welchen Stärke, im Gegensatz zu transitorischer Blattstärke, zur Überdauerung von längeren Zeiträumen als Depot gespeichert wird. Bevorzugte Stärke speichernde Pflanzenteile sind z.B. Knollen, Speicherwurzeln und Körner, besonders bevorzugt sind Körner enthaltend ein Endosperm, insbesondere bevorzugt sind Körner enthaltend ein Endosperm von Mais-, Reis- oder Weizenpflanzen.

In einer bevorzugten Ausführungsform werden erfindungsgemäße Verfahren zur Herstellung einer modifizierten Stärke zur Herstellung einer erfindungsgemäßen Stärke verwendet.

Modifizierte Stärke, erhältlich nach einem erfindungsgemäßen Verfahren zur Herstellung modifizierter Stärke, ist ebenfalls Gegenstand der vorliegenden Erfindung.

Weiterhin ist die Verwendung erfindungsgemäßer Pflanzenzellen oder erfindungsgemäßer Pflanzen zur Herstellung einer modifizierten Stärke Gegenstand der vorliegenden Erfindung.

Dem Fachmann ist bekannt, dass die Eigenschaften von Stärke durch z.B. thermische, chemische, enzymatische oder mechanische Derivatisierung verändert werden können. Derivatisierte Stärken sind für verschiedene Anwendungen im Nahrungsmittel- und/oder Nicht-Nahrungsmittelbereich besonders geeignet. Die erfindungsgemäßen Stärken sind als Ausgangssubstanz besser geeignet zur Herstellung von derivatisierten Stärken als herkömmliche Stärken, da sie z.B. durch den höheren Gehalt an Stärkephosphat einen höheren Anteil an reaktiven funktionalen Gruppen aufweisen. Weiterhin können die Derivatisierungen auf Grund des erhöhten Heißwasser Quellvermögens erfindungsgemäßer Stärken bei höheren Temperaturen durchgeführt werden, ohne dabei die Stärkekornstruktur wesentlich zu zerstören.

Die vorliegende Erfindung betrifft daher auch Verfahren zur Herstellung einer derivatisierten Stärke, worin erfindungsgemäße modifizierte Stärke, nachträglich derivatisiert wird.

Unter dem Begriff "derivatisierte Stärke" soll im Zusammenhang mit der vorliegenden Erfindung eine erfindungsgemäße modifizierte Stärke verstanden werden, deren Eigenschaften nach der Isolierung aus pflanzlichen Zellen mit Hilfe von chemischen, enzymatischen, thermischen oder mechanischen Verfahren verändert wurde.

In einer weiteren Ausführungsform der vorliegenden Erfindung handelt es sich bei der erfindungsgemäßen derivatisierten Stärke um mit Hitze und/oder mit Säure behandelte Stärke.

In einer weiteren Ausführungsform handelt es sich bei den derivatisierten Stärken um Stärkeether, insbesondere um Stärke-Alkylether, O-Allylether, Hydroxylalkylether, O-Carboxylmethylether, stickstoffhaltige Stärkeether, phosphathaltige Stärkeether oder schwefelhaltige Stärkeether.

In einer weiteren Ausführungsform handelt es sich bei den derivatisierten Stärken um vernetzte Stärken.

In einer weiteren Ausführungsform handelt es sich bei den derivatisierten Stärken um Stärke-Pfropf-Polymerisate.

In einer weiteren Ausführungsform handelt es sich bei den derivatisierten Stärken um oxidierte Stärken.

In einer weiteren Ausführungsform handelt es sich bei den derivatisierten Stärken um Stärkeester, insbesondere um Stärkeester, die unter Verwendung von organischen Säuren in die Stärke eingeführt wurden. Besonders bevorzugt handelt es sich um so genannte Phosphat-, Nitrat-, Sulfat-, Xanthat-, Acetat- oder Citratstärken.

Die erfindungsgemäßen derivatisierten Stärken eignen sich für verschiedene Verwendungen in der Pharmaindustrie, im Nahrungsmittel- und/oder Nicht-Nahrungsmittelbereich. Methoden zur Herstellung von erfindungsgemäßen derivatisierten Stärken sind dem Fachmann bekannt und in der allgemeinen Literatur ausreichend beschrieben. Eine Übersicht zur Herstellung von derivatisierten Stärken findet sich z.B. bei Orthoefer (in Corn, Chemistry and Technology, 1987, eds. Watson und Ramstad, Chapter 16, 479-499).

Derivatisierte Stärke, erhältlich nach dem erfindungsgemäßen Verfahren zur Herstellung einer derivatisierten Stärke ist ebenfalls Gegenstand der vorliegenden Erfindung.

Ferner ist die Verwendung erfindungsgemäßer modifizierter Stärken zur Herstellung von derivatisierter Stärke Gegenstand der vorliegenden Erfindung.

Stärke speichernde Teile von Pflanzen werden oft zu Mehlen verarbeitet. Beispiele für Teile von Pflanzen, aus welchen Mehle hergestellt werden, sind z.B. Knollen von Kartoffelpflanzen und Körner von Getreidepflanzen. Zur Herstellung von Mehlen aus Getreidepflanzen werden die endospermhaltigen Körner dieser Pflanzen gemahlen und gesiebt. Stärke ist ein Hauptbestandteil des Endosperms. Bei anderen Pflanzen, die kein Endosperm, sondern andere Stärke speichernde Teile, wie z.B. Knollen oder Wurzeln enthalten, wird Mehl häufig durch Zerkleinern, Trocknen und anschließendem Mahlen der betreffenden Speicherorgane hergestellt. Die Stärke des Endosperms oder enthaltend in Stärke speichernden Teilen von Pflanzen ist ein wesentlicher Anteil des Mehls, welches aus den betreffenden Pflanzenteilen hergestellt wird. Die Eigenschaften von Mehlen werden daher auch durch die in dem betreffenden Mehl vorliegende Stärke beeinflusst. Erfindungsgemäße Pflanzenzellen und erfindungsgemäße Pflanzen synthetisieren eine veränderte Stärke im Vergleich zu entsprechenden nicht genetisch modifizierten Wildtyp-Pflanzenzellen bzw. nicht genetisch modifizierten Wildtyp-Pflanzen. Mehle, hergestellt aus erfindungsgemäßen Pflanzenzellen, erfindungsgemäßen Pflanzen, erfindungsgemäßem Vermehrungsmaterial oder erfindungsgemäßen erntebaren Teilen weisen daher veränderte Eigenschaften auf. Die Eigenschaften von Mehlen können auch durch Mischen von Stärke mit Mehlen oder durch das Mischen von Mehlen mit unterschiedlichen Eigenschaften beeinflusst werden.

Ein weiterer Gegenstand der vorliegenden Erfindung betrifft daher Mehle, enthaltend eine erfindungsgemäße Stärke.

Ein weiterer Gegenstand der vorliegenden Erfindung betrifft Mehle, herstellbar aus erfindungsgemäßen Pflanzenzellen, erfindungsgemäßen Pflanzen, Stärke speichernden Teilen erfindungsgemäßer Pflanzen, aus erfindungsgemäßem Vermehrungsmaterial oder aus erfindungsgemäßen erntebaren Pflanzenteilen. Bevorzugte Stärke speichernde Teile erfindungsgemäßer Pflanzen für die Herstellung von Mehlen sind Knollen, Speicherwurzeln und ein Endosperm enthaltende Körner. Im Zusammenhang mit der vorliegenden Erfindung besonders bevorzugt sind Körner von Pflanzen der (systematischen) Familie Poaceae, insbesondere bevorzugt stammen Körner von Mais-, Reis- oder Weizenpflanzen.

Unter dem Begriff "Mehl" soll im Zusammenhang mit der vorliegenden Erfindung ein durch Mahlen von Pflanzenteilen erhaltenes Pulver verstanden werden. Gegebenenfalls werden Pflanzenteile vor dem Mahlen getrocknet und nach dem Mahlen zerkleinert und/oder gesiebt.

Erfindungsgemäße Mehle zeichnen sich auf Grund der in ihnen vorliegenden erfindungsgemäßen Stärke, dadurch aus, dass sie einen veränderten Phosphatgehalt und/oder ein erhöhtes Heißwasser Quellvermögen aufweisen. Dieses ist z.B. bei der Verarbeitung von Mehlen in der Lebensmittelindustrie für viele Anwendungen, insbesondere bei der Herstellung von Backwaren gewünscht.

Ein bevorzugter Gegenstand der vorliegenden Erfindung betrifft Mehle, hergestellt aus Körnern einer monocotylen Pflanze, dadurch gekennzeichnet, dass sie ein Heißwasser Quellvermögen von mindestens 28 g/g, bevorzugt von mindestens 33 g/g, besonders bevorzugt von mindestens 38 g/g und insbesondere bevorzugt von mindestens 43 g/g aufweisen.

Die Bestimmung des Heißwasser Quellvermögens von Mehlen erfolgt dabei analog zu der bereits beschriebenen Methode zur Bestimmung des Heißwasser Quellvermögens für Stärke, mit dem Unterschied, dass hierbei Mehle anstelle von Stärke eingesetzt werden. Eine bevorzugte Methode zur Bestimmung des Heißwasser Quellvermögens von Mehlen ist unter Allgemeine Methoden, Punkt 1 beschrieben.

Ein weiterer Gegenstand der vorliegenden Erfindung ist ein Verfahren zur Herstellung von Mehlen, umfassend den Schritt des Mahlens von erfindungsgemäßen Pflanzenzellen, erfindungsgemäßen Pflanzen, von Teilen erfindungsgemäßer Pflanzen, Stärke speichernden Teilen von erfindungsgemäßen Pflanzen, erfindungsgemäßem Vermehrungsmaterial oder erfindungsgemäßem erntebarem Material.

Mehle können durch Mahlen von Stärke speichernden Teilen von erfindungsgemäßen Pflanzen hergestellt werden. Dem Fachmann ist bekannt, wie er Mehle herstellt. Vorzugsweise umfasst ein Verfahren zur Herstellung von Mehlen auch den Schritt des Erntens der kultivierten Pflanzen bzw. Pflanzenteile und/oder des Vermehrungsmaterials bzw. der Stärke speichernden Teile dieser Pflanzen vor dem Mahlen und besonders bevorzugt ferner den Schritt der Kultivierung erfindungsgemäßer Pflanzen vor dem Ernten.

In einer weiteren Ausführungsform der vorliegenden Erfindung umfasst das Verfahren zur Herstellung von Mehlen eine Prozessierung von erfindungsgemäßen Pflanzen, von Stärke speichernden Teilen erfindungsgemäßer Pflanzen, von erfindungsgemäßem Vermehrungsmaterial oder von erfindungsgemäßem erntebarem Material vor dem Mahlen.

Die Prozessierung kann dabei z.B. eine Hitzebehandlung und/oder eine Trocknung sein. Hitzebehandlung gefolgt von einer Trocknung des Hitze behandelten Materials wird z.B. bei der Herstellung von Mehlen aus Speicherwurzeln oder Knollen wie z.B. aus Kartoffelknollen angewendet, bevor das Mahlen erfolgt. Die Zerkleinerung von erfindungsgemäßen Pflanzen, von Stärke speichernden Teilen erfindungsgemäßer Pflanzen, von erfindungsgemäßem Vermehrungsmaterial oder von erfindungsgemäßem erntebarem Material vor dem Mahlen kann ebenfalls eine Prozessierung im Sinne der vorliegenden Erfindung darstellen. Die Entfernung von pflanzlichem Gewebe, wie z.B. von Spelzen der Körner, vor dem Mahlen stellt auch eine Prozessierung vor dem Mahlen in Sinne der vorliegenden Erfindung dar.

In einer weiteren Ausführungsform der vorliegenden Erfindung umfasst das Verfahren zur Herstellung von Mehlen nach dem Mahlen eine Prozessierung des Mahlgutes.

Das Mahlgut kann dabei z.B. nach dem Mahlen gesiebt werden, um z.B. verschiedene Typenmehle herzustellen.

Ein weiterer Gegenstand der vorliegenden Erfindung ist die Verwendung von erfindungsgemäßen genetisch modifizierten Pflanzenzellen, erfindungsgemäßen Pflanzen, von Teilen erfindungsgemäßer Pflanzen, Stärke speichernden Teilen von erfindungsgemäßen Pflanzen, erfindungsgemäßem Vermehrungsmaterial oder erfindungsgemäßem erntebarem Material zur Herstellung von Mehlen.

Beschreibung der Sequenzen

SEQ ID NO 1: Nucleinsäuresequenz codierend ein Protein mit der Aktivität einer Glucan-Wasser-Dikinase aus *Solanum tuberosum.*

SEQ ID NO 2: Aminosäuresequenz des durch SEQ ID NO 1 codierten Proteins mit der Aktivität einer Glucan-Wasser-Dikinase aus *Solanum tuberosum.*

SEQ ID NO 3: Nucleinsäuresequenz codierend ein Protein mit der Aktivität einer Glucan-Wasser-Dikinase aus *Curcuma longa.*

SEQ ID NO 4: Aminosäuresequenz des durch SEQ ID NO 3 codierten Proteins mit der Aktivität einer Glucan-Wasser-Dikinase aus *Curcuma longa.*

SEQ ID NO 5: Nucleinsäuresequenz codierend ein Protein mit der Aktivität einer Stärkesynthase II aus *Triticum aestivum.*

SEQ ID NO 6: Aminosäuresequenz des durch SEQ ID NO 3 codierten Proteins mit der Aktivität einer Stärkesynthase II aus *Triticum aestivum.*

**Beschreibung der Figuren**

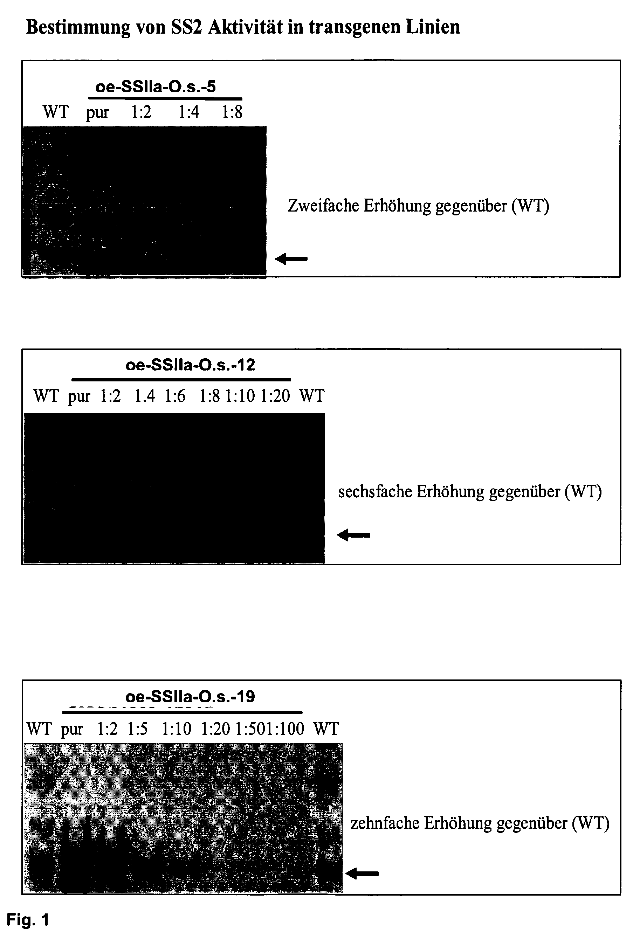

Fig. 1     zeigt Zymogrammme zur Bestimmung der Aktivität von Proteinen mit der Aktivität einer Stärkesynthase II im Vergleich zum Wildtyp. Verwendet wurden Gesamtproteinextrakte aus unreifen Körnern (15 Tage nach Blühbeginn) von Wildtyp-Pflanzen (WT) und den von drei unabhängig voneinander aus der Transformation mit dem Expressionsvektor AH32-191 hervorgegangenen genetisch modifizierten Pflanzen (oe-SSII-O.s.-5, oe-SSII-O.s.-12, oe-SSII-O.s.-19). In den Spuren WT und pur sind jeweils gleiche Mengen an Protein der jeweiligen Extrakte aufgetragen. Die Proteinextrakte der genetisch modifizierten Pflanzen wurden sequentiell (1:2, 1:4, 1:6, 1:8, 1:10, 1:20, 1:50 oder 1:100) verdünnt und diese Verdünnungen ebenfalls getrennt voneinander elektrophoretisch aufgetrennt. Durch Vergleich der Intensität der nach Anfärbung mit Lugol'scher Lösung im Zymogrammm vorliegenden spezifischen Produkte synthetisiert durch ein Protein mit der Aktivität einer Stärkesynthase II (durch einen Pfeil gekennzeichnet) von Proteinextrakten aus Wildtyp-Pflanzen mit der Intensität der betreffenden Banden von Proteinextrakten aus genetisch veränderten Pflanzen, kann die Erhöhung der Aktivität einer Stärkesynthase II gegenüber Wildtyp-Pflanzen ermittelt werden. Gleiche Intensitäten bedeuten dabei gleiche Aktivitäten.

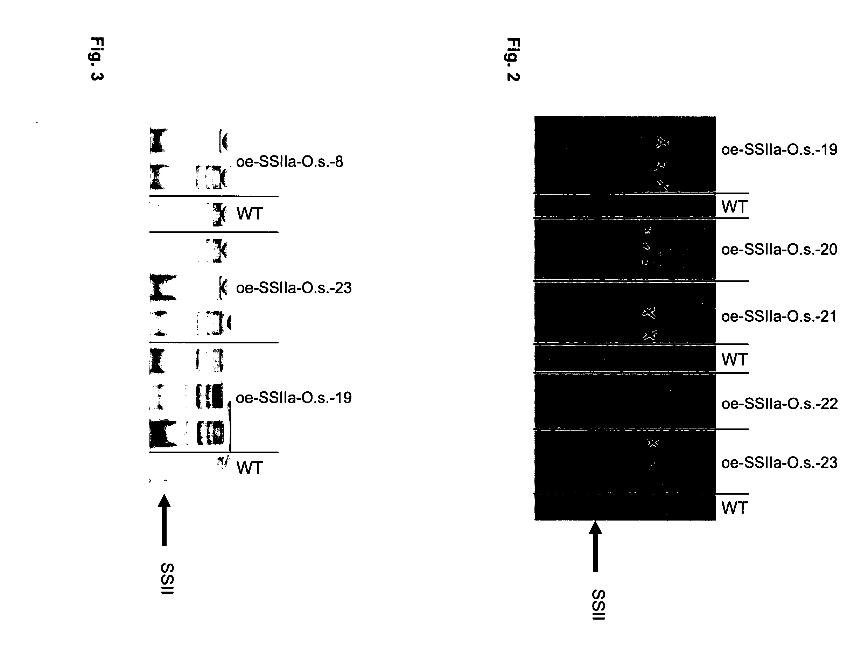

Fig. 2     zeigt das das Autoradiogram einer Northern-Blot Analyse unreifer T1-Samen der Reislinien oe-SSII-O.s.-19, oe-SSII-O.s.-20, oe-SSII-O.s.-21, oe-SSII-O.s.-22, oe-SSII-O.s.-23 im Vergleich zu nicht genetisch modifizierten Wildtyp-Pflanzen (WT). Dazu wurde aus jeweils drei Samen von unabhängig aus der Transformation mit dem Expressionsvektor AH32-191 hervorgegangenen Linien RNA extrahiert und nach der unter Allgemeine Methoden, Punkt 8 beschriebenen Methode analysiert. Die mit einer markierten Nucleinsäuresonde codierend für ein Protein mit der Aktivität einer Stärkesynthase II aus Weizen hybridisierende Bande ist mit SSII gekennzeichnet.

Fig.3     Zeigt ein Zymogramm von Proteinextrakten aus unreifen T1-Samen der Reislinien oe-SSII-O.s.-8, oe-SSII-O.s.-19, oe-SSII-O.s.-23 im Vergleich zu Samen von nicht genetisch modifizierten Wildtyp-Pflanzen (WT) nach Anfärbung mit Lugol'scher Lösung. Je Linie wurden Proteinextrakte aus zwei (oe-SSII-O.s.-8) bzw. drei (oe-SSII-O.s.-19, oe-SSII-O.s.-23) verschiedenen Körnern analysiert. Die Analyse mittels Zymogramm erfolgte dabei nach der unter Allgemeine Methoden, Punkt 9 beschriebenen Methode. Die Bande im Zymogramm, die für ein Protein mit der Aktivität einer Stärkesynthase II spezifisch ist, ist mit SSII gekennzeichnet.

**Allgemeine Methoden**

Im Folgenden werden Methoden beschrieben, welche zur Durchführung der erfindungsgemäßen Verfahren verwendet werden können. Diese Methoden stellen konkrete Ausführungsformen der vorliegenden Erfindung dar, beschränken die vorliegende Erfindung jedoch nicht auf diese Methoden. Dem Fachmann ist bekannt, dass er durch Modifikation der beschriebenen Methoden und/oder durch Ersetzen einzelner Methodenteile durch alternative Methodenteile die Erfindung in gleicher Weise ausführen kann. Der Inhalt aller zitierten Veröffentlichungen ist durch die Nennung des Zitates in die Beschreibung der Anmeldung mit aufgenommen.

1. Bestimmung des Heißwasser Quellvermögens (SP, Swelling Power) 100 mg Probe (Stärke oder Mehl) werden in 6 ml Wasser suspendiert und anschließend für 20 Minuten bei 92,5°C gequollen. Während der Inkubation der Probe bei 92,5°C wird die Suspension mehrfach (für die ersten 2 Minuten kontinuierlich, nach 3, 4, 5, 10, 15 bzw. 25 Minuten) durch vorsichtiges, Drehen der Probenbehälter um 360° gemischt. Nach insgesamt 30 Minuten Inkubation bei 92,5°C wird die Suspension ca. 1 Minute in Eiswasser abgekühlt, bevor eine Inkubation bei 25°C für 5 Minuten erfolgt. Nach Zentrifugation (Raumtemperatur, 1000xg, 15 Minuten) wird der erhaltene Überstand vorsichtig von dem gelartigen Sediment abgezogen und das Gewicht des Sedimentes ermittelt. Das Heißwasser Quellvermögen wird nach folgender Formel errechnet:

SP = (Gewicht des gelartigen Sedimentes) / (Gewicht der eingewogenen Probe (Mehl oder Stärke))

2. Bestimmung des Gehaltes an Stärkephosphat

a) Bestimmung des Phosphatgehaltes in C6-Position der Glucosemoleküle

In der Stärke können die Positionen C2, C3 und C6 der Glucoseeinheiten phosphoryliert sein. Zur Bestimmung des C6-P-Gehaltes der Stärke bzw. des Mehls (modifiziert nach Nielsen et al., 1994, Plant Physiol. 105: 111-117) wurden 50 mg Reismehl oder -stärke in 500μl 0,7 M HCl 4 h bei 95°C unter ständigem Schütteln hydrolysiert. Anschließend wurden die Ansätze für 10 min bei 15.500xg zentrifugiert und die Überstände mittels einer Filtermembran (0,45 μM) von Schwebstoffen und Trübungen gereinigt. Von dem klaren Hydrolysat wurden 20 μl mit 180 μl Imidazol-Puffer (300 mM Imidazol, pH 7,4; 7,5 mM MgCl2, 1 mM EDTA und 0,4 mM NADP) gemischt und die Proben im Photometer bei 340 nm vermessen. Nach Erfassung der Basisabsorption wurde eine Enzymreaktion durch Zugabe von je 2 Einheiten (units) Glucose-6-Phosphat Dehydrogenase (von *Leuconostoc mesenteroides*, Boehringer Mannheim) gestartet. Die gemessene Änderung (OD) beruht auf einer äquimolaren Umsetzung von Glucose-6-Phosphat und NADP zu 6-Phosphorglukonat und NADPH, wobei die Bildung des NADPH bei der o. g. Wellenlänge erfasst wird. Die Reaktion wurde bis zum Erreichen eines Endpunktes verfolgt. Aus dem Ergebnis dieser Messung kann der Gehalt an Glucose-6-Phosphat im Hydrolysat errechnet werden:

$$\text{nmol Glucose-6-Phosphat/mg FG} = \frac{\text{OD x Messvolumen (200}\mu\text{l) x Volumen des Hydrolysates (500}\mu\text{l)}}{\text{Extinktionskoeffizient x Volumen der Messprobe (20}\mu\text{l) x mg Einwaage (50mg)}}$$

Um keine fehlerhaften Ergebnisse durch nicht vollständige Hydrolyse der Stärke im eingewogenen Material (Mehl oder Stärke) zu erhalten, wurde anschließend der Grad der Hydrolyse bestimmt. Dazu wurde den jeweiligen in Bezug auf Glucose-6-Phosphat vermessenen Hydrolysaten 10μl Hydrolysat entnommen, mit 10μl 0,7 M NaOH neutralisiert, mit Wasser auf ein Endvolumen von 2ml gebracht und 1:100 mit Wasser verdünnt. 4 μl dieser Verdünnung wurden mit 196μl Messpuffer (100mM Imidazol pH 6,9; 5 mM MgCl2, 1 mM ATP, 0,4 mM NADP) versetzt und zur photometrischen Bestimmung des Glucosegehaltes verwendet. Nach Ermittlung der Basisabsorption bei 340nm wurde die Reaktion durch Zugabe von 2 μl Enzym-Mix (Hexokinase 1:10; Glucose-6-Phosphat Dehydrogenase aus Hefe 1:10 in Messpuffer) bis zur Erreichung des Endpunktes im Photometer (340nm) verfolgt. Das Messprinzip entspricht dem der ersten Reaktion. Aus den erhaltenen Messwerten kann für die jeweilige Probe die Glucosemenge berechnet werden:

$$\text{mmol Glucose/g FG} = \frac{\text{OD x Messvolumen (200}\mu\text{l)} \times \text{Volumen des Hydrolysates (500}\mu\text{l)} \times \text{Gesamtvolumen der Verdünnung (2ml)}}{\text{Extinktionskoeffizient} \times \text{Volumen der Messprobe (20}\mu\text{l)} \times \text{für die Verdünnung eingesetztes Volumen (10}\mu\text{l)} \times \text{mg Einwaage (50mg)}}$$

Die Menge an nachgewiesener Glucose der einzelnen Proben entspricht dabei dem Anteil der Stärke, der für die C6-Phosphat Bestimmung zur Verfügung steht. Zur Vereinfachung bei der weiteren Berechnung wird der Glucosegehalt in Stärkegehalt umgerechnet.

$$\text{Stärkegehalt (\%)} = \frac{\text{Glucosegehalt (mmol/G FG)} \times \text{Molekulargewicht von Glucose in Stärke (162 g/mol)} \times \text{Umrechnugnsfaktor (\%=100)}}{\text{Umrechnugsfaktor (mmol zu mol =1000)}}$$

Nachfolgend wird das Ergebnis der Glucose-6-Phosphat-Messung mit dem Stärkegehalt der entsprechenden Probe in Relation gesetzt, um so den Gehalt an Glucose-6-Phosphat pro mg hydrolysierter Stärke auszudrücken:

$$\text{nmol Glc-6-P/mg Stärke} = \frac{\text{nmol Glucose-6-Phosphat /mg Einwaage x 100}}{\text{Stärkegehalt ( mg Stärke/ 100mg Einwaage)}}$$

Anders als bei einem Bezug der Menge an Glucose-6-Phosphat auf das eingewogene Gewicht der Probe (Mehl oder Stärke) wird durch diese Weise der Berechnung die Menge an Glucose-6-Phosphat lediglich auf den Teil der Stärke bezogen, welcher vollständig zu Glucose hydrolysiert wurde.

b) Bestimmung des Gesamtphosphatgehaltes

Die Bestimmung des Gesamtphosphatgehaltes erfolgte nach der Methode von Ames (Methods in Enzymology VIII, (1966), 115-118).

Es werden ca. 50 mg Stärke mit 30 μl ethanolischer Magnesiumnitrat-Lösung versetzt und drei Stunden bei 500°C im Muffelofen verascht. Der Rückstand wird mit 300 μl 0,5 M Salzsäure versetzt und 30 min bei 60°C inkubiert. Anschließend wird ein Aliquot auf 300 μl 0,5 M Salzsäure aufgefüllt, zu einer Mischung aus 100 μl 10%iger Ascorbinsäure und 600 μl 0,42% Ammoniummolybdat in 2 M Schwefelsäure gegeben und 20 min bei 45°C inkubiert.

3. Transformation von Reispflanzen

Reispflanzen wurden nach der von Hiei et al. (1994, Plant Journal 6(2), 271-282) beschriebenen Methode transformiert.

4. Transformation von Weizenpflanzen

Weizenpflanzen wurden nach der bei Becker et al. (1994, Plant Journal 5, 299-307) beschriebenen Methode transformiert.

5. Transformation von Maispflanzen

Unreife Embryonen von Maispflanzen der Linie A188 wurden nach der bei Ishida et al. (1996, Nature Biotechnology 14, 745-750) beschriebenen Methode transformiert.

6. Prozessierung von Reiskörnern und Herstellung von Reismehlen

Zur Erzeugung von ausreichenden Mengen an Untersuchungsmaterial wurden Reispflanzen im Gewächshaus angezogen und nach Erreichen vollständiger Reife geerntet. Zum weiteren Trocknen wurden die reifen Reiskörner für 3-7 Tage bei 37°C gelagert.

Nachfolgend wurden die Körner mittels eines Entspelzers (Laboratory Paddy sheller, Grainman, Miami, Florida, USA) von den Spelzen befreit und der erhaltene braune Reis wurde durch 1-minütiges Polieren (Pearlest Rice Polisher, Kett, Villa Park, CA, USA) zu weißem Reis prozessiert. Für Untersuchungen der Kornzusammensetzung sowie der Stärkeeigenschaften wurden die weißen Körner mittels einer Labormühle (Cyclotec, Sample mill, Foss, Dänemark) zu sog. Reismehl vermahlen.

7. Extraktion von Reisstärke aus Reismehl

Die Extraktion von Reisstärke aus Reismehl erfolgte in Anlehnung an die bei Wang und Wang (2004; Journal of Cereal Science 39: 291-296) beschriebene Methode.

Ca. 10g Reismehl wurden mit 40ml 0,05% (w/v) NaOH für 16-18 Stunden auf einem Schüttler bei Raumtemperatur inkubiert. Nachfolgend wurde die Suspension zur Vervollständigung des Aufschlusses in einen Warring Blender überführt und für 15 Sekunden bei geringer Geschwindigkeit sowie anschließend 45 Sekunden bei hoher Geschwindigkeit durchmischt. Zur Abtrennung von größeren Bestandteilen (z.B. Zellwand) wurde die Suspension auf einander folgend durch Siebe mit einer Maschenweite von 125 $\mu$m und 63 $\mu$m gegeben. Nach Zentrifugation bei 1500 rpm für 15 Minuten (Microfuge 3.OR; Heraeus) wurde der Überstand abgegossen und die an der Oberfläche des Niederschlages liegende Proteinschicht mit einem Spatel entfernt. Der restliche Niederschlag wurde nochmals in 0,05 % (w/v) NaOH resuspendiert und der oben beschriebene Vorgang wiederholt. Nachfolgend wurde der Niederschlag in Wasser resuspendiert und der pH-Wert der Suspension mit HCl auf 6,5 bis 7 eingestellt. Die erhaltene Reisstärke wurde insgesamt dreimal mit Wasser gewaschen, wobei jeder Waschschritt eine Sedimentation (Zentrifugation bei 1500 rpm, 15 min, RT), ein Verwerfen des Überstandes und das Resuspendieren des Niederschlages in frischem Wasser umfasste. Vor dem letzten Waschschritt wurde erneut der pH-Wert überprüft und ggf. mit HCl auf pH 7 eingestellt. Der Niederschlag des letzten Waschschrittes wurde in Aceton resuspendiert, sedimentiert und der Überstand verworfen. Nach erneutem Resuspendieren des Niederschlages in Aceton wurde die Suspension in eine Petrischale gegossen und unter dem Abzug für mindestens 18 Stunden bei Raumtemperatur getrocknet.

In einem letzten Schritt wurde die so erhaltene Reisstärke durch Mörsern in ein feines Pulver überführt, welches direkt für weitere Untersuchungen eingesetzt werden kann.

8. Analyse der Expressionshöhe eines Proteins mittels Northern-Blot

Die Expression einer Nucleinsäure, die ein Protein codiert, wurde mittels Northern-Blot Analyse untersucht. Hierzu wurden für jede unabhängige mittels Transformation erhaltene Pflanze drei unreife Reiskörner (ca. 15 Tage nach der Blüte) geerntet und in flüssigem Stickstoff eingefroren. Zur Homogenisierung wurden die gefrorenen Reiskörner in einer 96 Loch-Mikrotiter-Platte mit einer 4,5 mm Stahlkugel in einer Retsch-Mühle (Modell MM300) für 30 Sekunden bei einer Frequenz von 30 Hertz zerkleinert. Anschließend wurde die RNA mittels Promega RNA-Extraktionskit nach Angaben des Herstellers isoliert (SV 96 Total RNA Isolation System, Bestell-Nr. Z3505, Promega, Mannheim). Die Konzentration der RNA in den einzelnen Proben wurde durch photometrische Bestimmung der Absorption bei 260 nm bestimmt.

Pro Probe wurden jeweils 2 $\mu$g RNA auf ein einheitliches Volumen gebracht und mit einem identischen Volumen RNA-Probenpuffer (65% (v/v) Formamid, 8% Formaldehyd, 13% (v/v) Gelpuffer (s. o.), 50 $\mu$g/ml Ethidiumbromid) versetzt. Nach Erhitzen (10 min, 65°C) und sofortigem Abkühlen auf Eis wurde die RNA über ein 1,2% (w/v) Agarosegel (20 mM MOPS pH 8,0, 5 mM Na-Acetat, 1 mM EDTA, 6% (v/v) Formaldehyd) unter Verwendung von RNA-Laufpuffer (20 mM MOPS pH 8,0, 5 mM Na-Acetat, 1 mM EDTA) bei einer konstanten Stromstärke von 50-80 mA für ca. 2 Stunden aufgetrennt.

Nachfolgend wurde die RNA mittels Diffusionsblot unter Verwendung von 10x SSC (1,5 M NaCl, 150 mM Na-Citrat pH 7,0) auf eine Hybond-N-Membran transferiert und mittels UV-Bestrahlung auf der Membran immobilisiert.

Für die Hybridisierung des Northern-Blots zum Nachweis der Expression eines Nucleinsäuremoleküls, das ein Protein mit der Aktivität einer Stärkesynthase II codiert, wurde ein ca. 1 kb Spel/BspHl-Fragment des Plasmids AH32-191 (Bp 4568-5686), welches den 5'-Bereich der cDNA, codierend ein Protein mit der Aktivität einer Stärkesynthase II aus Weizen umfasst, verwendet. Die radioaktive Markierung des DNA-Fragmentes erfolgte mittels des Random primed DNA labeling Kit der Firma Roche (Bestell-Nr. 1004 760) unter Verwendung von $^{32}$P-alpha-dCTP nach Angaben des Herstellers.

Die Nylonmembran, enthaltend die transferierte RNA, wurde für vier Stunden bei 60°C unter leichtem Schütteln im Wasserbad mit Hybridisierungspuffer (250 mM Na-Phosphat-Puffer pH 7,2, 1 mM EDTA, 6% (w/v) SDS, 1% (w/v) BSA) inkubiert, bevor zum Hybridisierungspuffer die radioaktiv markierte DNA hinzugefügt wurde. Nach 16 Stunden Inkubation wurde der Hybridisierungspuffer entfernt und die Membran zur Entfernung unspezifisch gebundener DNA-Moleküle auf einander folgend einmal mit 3xSSC und einmal mit 2xSSC (s. o.) bei 60°C unter leichtem Schütteln im Wasserbad gewaschen.

Zum Nachweis markierter RNA erfolgte eine Autoradiographie der Nylonmembran auf einen Röntgenfilm bei -70°C für ein bis drei Tage.

9. Bestimmung der Aktivität eines Proteins mit der Aktivität einer Stärkesynthase II mittels Aktivitätsgel (Zymogrammm)

Der Nachweis von der Aktivität von Proteinen mit der Aktivität einer Stärkesynthase in unreifen Reiskörnern erfolgte mittels Aktivitätsgelen (Zymogrammmen), bei denen Proteinextrakte unter nativen Bedingungen in einem Polyacrylamidgel aufgetrennt und nachfolgend mit entsprechenden Substraten inkubiert werden. Das entstandene Reaktionsprodukt (alpha-Glucan) wurde mittels Lugol'scher Lösung im Gel angefärbt.

Einzelne unreife Reiskörner (ca. 15 Tage nach Blüte) wurden in flüssigem Stickstoff eingefroren und in 150-200 μl kaltem Extraktionspuffer (50 mM Tris/HCl pH 7,6, 2,5 mM EDTA, 2 mM DTT, 4 mM PMSF, 0,1% (w/v) Glykogen, 10% (v/v) Glyzerin) homogenisiert. Nach Zentrifugation (15 min, 13000 g, 4°C) wurde der klare Überstand in ein frisches Reaktionsgefäss überführt und ein Aliquot des Extraktes zur Bestimmung des Proteingehaltes nach Bradford (1976, Anal Biochem 72: 248-254) verwendet.

Die Auftrennung der Proteinextrakte erfolgte mittels kontinuierlichem 7,5% Polyacrylamid-Gel (7,5% Acrylamid: Bisacrylamid 37,5:1; 25 mM Tris/HCl pH 7,6, 192 mM Gylcin, 0,1% (w/v) APS, 0,05% (v/v) TEMED) unter Verwendung von einfach konzentriertem Laufpuffer (25 mM Tris/HCl, 192 mM Glycin). Für jede Probe wurden jeweils Mengen entsprechend 15 μg Protein aufgetragen und die Elektrophorese für 2 bis 2,5 Stunden bei 4°C durchgeführt.

Nachfolgend wurden die Gele in 15 ml Inkubationspuffer (0,5 mM Natriumcitrat pH 7,0, 25 mM Kaliumacetat, 2 mM EDTA, 2 mM DTT, 0,1% (w/v) Amylopektin, 50 mM Tricine/NaOH pH 8,5, 1 mM ADP-Glucose) über Nacht bei Raumtemperatur unter ständigem Schütteln inkubiert. Die Anfärbung der gebildeten Stärke erfolgte mittels Lugol'scher Lösung.

Um zu ermitteln, um das wie viel Fache die Aktivität eines Proteins mit der Aktivität einer Stärkesynthase II im Vergleich zu entsprechenden nicht genetisch modifizierten Wildtyp-Pflanzen erhöht ist, wurden Proteinextrakte der genetisch modifizierten Linien jeweils sequentiell verdünnt und entsprechend der oben beschriebenen Methode elektrophoretisch aufgetrennt. Die Durchführung der weiteren Schritte erfolgte wie oben bereits beschrieben. Nach Anfärbung der Zymogrammme mit Lugol'scher Lösung wurde ein optischer Vergleich der Intensität der angefärbten Produkte, produziert durch ein Protein mit der Aktivität einer Stärkesynthase II (in Fig. 1 mit einem Pfeil gekennzeichnet) für die verschiedenen Verdünnungen der Proteinextrakte von genetisch modifizierten Pflanzen mit den betreffenden Produkten des unverdünnten Wildtyp-Proteinextraktes durchgeführt. Da die Intensität der Färbung der Produkte direkt korreliert mit der Aktivität eines Proteins mit der Aktivität einer Stärkesynthase II, weisen Banden der Produkte mit gleichen Intensitäten die gleiche Aktivität auf. Weist die Bande des Produktes eines Proteins mit der Aktivität einer Stärkesynthase II im verdünnten Proteinextrakt die gleiche Intensität auf, wie die betreffende Bande des Produktes von entsprechendem, unverdünntem Proteinextrakt aus Wildtyp-Pflanzen, so entspricht der Verdünnungsfaktor dem Grad der Erhöhung der Aktivität in der betreffenden genetisch modifizierten Pflanze (Vergleiche dazu Fig. 1).

10. Erzeugung von Pflanzen mittels Reisembryos (Embryo rescue)

Samen werden von der Rispe abgetrennt und die Hüllspelzen entfernt. Das Endosperm wird mit einem Skalpell vom Embryo abgetrennt und für entsprechende Analysen verwendet. Der Embryo wird zur Verbesserung der Benetzbarkeit kurz mit 70% Ethanol behandelt und nachfolgend zur Sterilisation für 20 Minuten in einer Lösung enthaltend 2% NaOCl und einen Tropfen handelsübliches Spülmittel inkubiert.

Anschließend wird die Sterilisationslösung möglichst vollständig entfernt und der Embryo wird einmal für eine Minute und nachfolgend zweimal für jeweils 10 Minuten mit sterilem demineralisiertem Wasser gewaschen. Die Samen werden in Petrischalen auf mit Agar verfestigtem Medium enthaltend jeweils ein Viertel der Salzkonzentration von MS-Medium (Murashige-Skoog Medium) und 4% Saccharose ausgelegt. Anschließend werden die Petrischalen mit Parafilm verschlossen und bei 23°C im Dunkeln inkubiert. Nach der Keimung (ca. 5-7 Tage nach dem Auslegen der Embryos) werden die Petrischalen ins Licht überführt. Haben die Hypocotyle der Keimlinge eine Länge von ca. 2 cm erreicht, werden die Pflanzen in Gläser enthaltend mit Agar verfestigtes MS-Medium mit 2% Saccharose überführt. Nach ausreichender Wurzelbildung können die Pflanzen in Erde getopft werden.

**Beispiele**

1. Herstellung des pflanzlichen Expressionsvektors AH32-191, der eine codierende Sequenz für ein Protein mit der Aktivität einer Stärkesynthase II umfasst

Die vollständige codierende Sequenz des Proteins mit der Aktivität einer Stärkesynthase II aus Weizen (T.a.-SSII) wurde aus dem Plasmid pCF31 (beschrieben in WO 97 45545 unter der Bezeichnung pTaSS1) mittels der Restriktionsendonucleasen *Ecl*136I/ und Xho I herausgeschnitten und in das mit den Restriktionsenducleasen Eco RV und Xho I geschnittene Plasmid IR103-123 (beschrieben in WO 05 030941) kloniert. Der erhaltene Expressionsvektor wurde mit AH32-191 bezeichnet. Der pflanzliche Expressionsvektor IR103-123 dient der endospermspezifischen Expression des Zielgenes unter Kontrolle des Globulin-Promotors aus Reis. Zusätzlich enthält der pflanzliche Expressionsvektor IR103-123 das *bar*-Gen unter Kontrolle des CaMV35S Promotors, welches als Selektionsmarker für die Transformation von Pflanzen verwendet wurde.

2. Herstellung von Reispflanzen, die eine erhöhte Aktivität eines Proteins mit der Aktivität einer Stärkesynthase II aufweisen

Reispflanzen (Varietät M202) wurden mittels *Agrobacterium,* enthaltend das Plasmid AH32-191, unter Verwendung der bei Hiei et al. (1994, Plant Journal 6(2), 271-282) beschriebenen Methode transformiert. Die erhaltenen Pflanzen erhielten die Bezeichnung oe-SSII-O.s.-X, wobei X unabhängige aus der Transformation hervorgegangene Pflanzen bezeichnet.

3. Herstellung von Reispflanzen, die eine erhöhte Aktivität eines Proteins mit der Aktivität einer Glucan-Wasser-Dikinase aufweisen

Reispflanzen (Varietät M202) wurden mittels *Agrobacterium,* enthaltend das Plasmid pML82 (beschrieben in WO 05 095619) unter Verwendung der bei Hiei et al. (1994, Plant Journal 6(2), 271-282) beschriebenen Methode transformiert. Die erhaltenen Pflanzen erhielten die Bezeichnung oe-GWD-O.s.-X, wobei X unabhängige aus der Transformation hervorgegangene Pflanzen bezeichnet.

4. Analyse der Reispflanzen, die mit dem Expressionsvektor AH32-191 transformiert wurden

Aus der Transformation mit dem Expressionsvektor AH32-191 hervorgegangene Reispflanzen (T0 Pflanzen) der Linien mit der Bezeichnung oe-SSII-O.s.-X wurden im Gewächshaus in Erde kultiviert. Aus unreifen Körnern (T1-Samen) verschiedener Linien mit der Bezeichnung oe-SSII-O.s.-X wurde RNA isoliert und eine Northern-Blot Analyse nach der unter allgemeine Methoden, Punkt 8 beschriebenen Methode durchgeführt. Es konnten mehrere Linien identifiziert werden, die eine erhöhte Expression eines Proteins mit der Aktivität der Stärkesynthase II aus Weizen im Vergleich zu entsprechenden genetisch nicht modifizierten Wildtyp-Pflanzen aufwiesen (siehe beispielhafte Darstellung in Fig. 2)

Zusätzlich wurde eine erhöhte Aktivität eines Proteins mit der Aktivität einer Stärkesynthase II in unreifen T1-Samen verschiedener Linien oe-SSII-O.s.-X mittels Zymogrammm nachgewiesen (siehe beispielhafte Darstellung in Fig. 1 und 2). Die Durchführung der Analyse mittels Zymogramm erfolgte nach der unter Allgemeine Methoden, Punkt 9 beschriebenen Methode.

5. Analyse der Reispflanzen, die mit dem Expressionsvektor pML82 transformiert wurden

Aus der Transformation mit dem Expressionsvektor pML82 hervorgegangene Reispflanzen (T0 Pflanzen) der Linien mit der Bezeichnung oe-GWD-O.s.-X wurden im Gewächshaus in Erde kultiviert. Aus einzelnen, reifen Körnern (T1-Samen) verschiedener Linien mit der Bezeichnung oe-GWD-O.s.-X wurde Mehl hergestellt. Dazu wurden einzelne Körner fein zerrieben und das geriebene Material anschließend in einer Kugelmühle (Firma Retsch, Modell MM300) für 30 Sekunden bei einer Frequenz von 30 Hertz zerkleinert. Anschließend erfolgte eine Bestimmung des Stärkephosphatgehaltes in der C6-Position von Glucosemolekülen der Mehle nach der unter Allgemeine Methoden, Punkt 2 beschriebenen Methode. Für ausgewählte Pflanzen wurden folgende Ergebnisse erhalten:

**Tabelle 1:** Gehalt von in C6 -Position der Glucosemoleküle gebundenem Phosphat in Mehlen, hergestellt aus einzelnen T1-Samen unterschiedlicher Linien mit der Bezeichnung oe-GWD-O.s.-X im Vergleich zu Mehlen, hergestellt aus Samen von entsprechenden nicht genetisch modifizierten Wildtyp-Pflanzen (WT) der Varietät M202.

| Bezeichnung der Pflanze | nmol C6-Phosphat pro mg Frischgewicht der Samen |
|---|---|
| oe-GWD-O.s.-2 | 1,68 |
| oe-GWD-O.s.-4 | 1,70 |
| oe-GWD-O.s.-9 | 1,47 |

(fortgesetzt)

| Bezeichnung der Pflanze | nmol C6-Phosphat pro mg Frischgewicht der Samen |
|---|---|
| WT | 0,30 |

Wie aus Tabelle 1 hervorgeht, konnten unabhängige Linien, hervorgegangen aus der Transformation mit dem Expressionsvektor pML82, identifiziert werden, die im Vergleich zu entsprechenden nicht genetisch modifizierten Wildtyp-Pflanzen einen erhöhten Gehalt an in C6-Position der Glucosemoleküle gebundenem Phosphat in Mehlen aufweisen. Da bekannt ist, dass Pflanzenzellen, die eine erhöhte Expression eines Proteins mit der Aktivität einer Glucan-Wasser-Dikinase aufweisen, eine Stärke synthetisieren, die einen höheren Stärkephosphatgehalt aufweist, im Vergleich zu entsprechenden genetisch nicht modifizierten Wildtyp-Pflanzen (siehe z.B. WO 02 34923), ist die Erhöhung des Phosphatgehaltes in Linien mit der Bezeichnung oe-GWD-O.s.-X auf eine erhöhte Aktivität des Proteins mit der Aktivität einer Glucan-Wasser-Dikinase zurückzuführen.

6. Herstellung von Pflanzen, die eine erhöhte Aktivität eines Proteins mit der Aktivität einer Stärkesynthase II und eine erhöhte Aktivität eines Proteins mit der Aktivität einer Glucan-Wasser-Dikinase aufweisen

Jeweils 30 T1 Samen von Pflanzen verschiedener Linien mit der Bezeichnung oe-SSII-O.s.-X bzw. der Linien oe-GWD-O.s.-X wurden erneut im Gewächshaus kultiviert und die betreffenden Pflanzen mit einer Lösung, enthaltend 0,5% Basta® (Bayer CropScience) besprüht. Etwa ein Viertel der behandelten Pflanzen der Linien oe-SSII-O.s.-19, oe-GWD-O.s.-2, oe-GWD-O.s.-4 und oe-GWD-O.s.-9 reagierte sensitiv auf die Behandlung mit Basta®, was darauf schließen ließ, dass sie kein gegen Basta® Resistenz vermittelndes bar-Gen enthielten und die T-DNA der Expressionsvektoren an einem Ort im Genom integriert war bzw. an Orten im Genom integriert waren, die so dicht beieinander liegen, dass sie nicht segregieren. T2 Samen von T1 Pflanzen dieser Linien die resistent gegenüber der Behandlung mit Basta® waren, wurden erneut im Gewächshaus ausgelegt und eine Behandlung mit Basta®, wie soeben beschrieben, durchgeführt. Anschließend wurde die gleiche Behandlung mit Basta® mit T3 Pflanzen dieser Linien durchgeführt: Es konnten dabei verschiedene T3 Pflanzen der Linien oe-GWD-O.s.-19, oe-GWD-O.s.-2, oe-GWD-O.s.-4 und oe-GWD-O.s.-9 identifiziert werden, bei welchen alle Pflanzen resistent gegenüber Basta® waren. Dieses ließ darauf schließen, dass T2 Pflanzen, von denen die betreffenden T3-Samen stammten, homozygot für die integrierte T-DNA waren. T2 Samen von homozygoten Pflanzen der Linie oe-SSII-O.s.-19, GWD-O.s.-2, oe-GWD-O.s.-4 und oe-GWD-O.s.-9 wurden erneut ausgelegt und verschiedene Pflanzen der Linie oe-SSII-O.s.-19 jeweils mit Pollen der Linien oe-GWD-O.s.-2, oe-GWD-O.s.-4 oder oe-GWD-O.s.-9 bestäubt. Die daraus entstandenen Kreuzungsnachkommen wurden mit oe-SSII/GWD-O.s.-1 (oe-SSII-O.s.-19 X GWD-O.s.-2), oe-SSII/GWD-O.s.-2 (oe-SSII-O.s.-19 X oe-GWD-O.s.-4) bzw. oe-SSII/GWD-O.s.-3 (oe-SSII-O.s.-19 X oe-GWD-O.s.-9) bezeichnet.

7. Analyse von Pflanzen, die eine erhöhte Aktivität eines Proteins mit der Aktivität einer Stärkesynthase II und eine erhöhte Aktivität eines Proteins mit der Aktivität einer Glucan-Wasser-Dikinase aufweisen

Von den aus Kreuzungen hervorgegangenen Linien oe-SSII/GWD-O.s.-1, oe-SSII/GWD-O.s.-2, oe-SSII/GWD-O.s.-3 und homozygoten Elternpflanzen (oe-SSII-O.s.-19, oe-GWD-O.s.-2, oe-GWD-O.s.-4 und oe-GWD-O.s.-9) wurden jeweils einzelne F1-Samen geerntet, die Embryos abgetrennt und bei Raumtemperatur gelagert. Mehl, gewonnen aus dem verbleibenden Endosperm der jeweiligen einzelnen F1 Samen, wurde mit der unter Allgemeine Methoden, Punkt 6 beschriebenen Methode auf den Gehalt an in C6-Position von Glucosemolekülen gebundenem Phosphat hin untersucht. Es wurden folgende Ergebnisse erhalten.

Tabelle 2: : Gehalt von in C6-Position der Glucosemoleküle gebundenem Phosphat in Mehlen, hergestellt aus einzelnen F1-Samen von Linien mit der Bezeichnung oe-SSII/GWD-O.s.-X" im Vergleich zu Mehlen, hergestellt aus einzelnen Samen von entsprechenden nicht genetisch modifizierten Wildtyp-Pflanzen der Varietät M202 (WT). Ebenfalls dargestellt ist der Gehalt von in C6-Position der Glucosemoleküle gebundenem Phosphat in Mehlen, hergestellt von einzelnen homozygoten Samen der Elternlinien.

| Bezeichnung der Pflanze | Nr. des F1-Samens | nmol C6-Phosphat pro mg Stärke |
|---|---|---|
| oe-SSII/GWD-O.s-1 | 1 | 6,5 |
| | 2 | 2,8 |
| | 3 | 2,6 |
| | 4 | 2,5 |
| | 5 | 2,6 |

(fortgesetzt)

| Bezeichnung der Pflanze | Nr. des F1-Samens | nmol C6-Phosphat pro mg Stärke |
|---|---|---|
| oe-SSII/GWD-O.s.-2 | 1 | 7,9 |
|  | 2 | 7,2 |
|  | 3 | 7,1 |
|  | 4 | 8,4 |
|  | 5 | 6,9 |
| oe-SSII/GWD-O.s.-3 | 1 | 6,7 |
|  | 2 | 6,0 |
|  | 3 | 7,7 |
|  | 4 | 7,5 |
|  | 5 | 7,0 |
| SSII-O.s.-19 (Mutter) | 1 | 1,5 |
|  | 2 | 1,4 |
| oe-GWD-O.s.-2 (Vater 1) | 1 | 3,6 |
| oe-GWD-O.s.-4 (Vater 2) | 1 | 3,5 |
| oe-GWD-O.s.-9 (Vater 3) | 1 | 4,1 |
| WT | 1 | 0,5 |
|  | 2 | 0,5 |

Embryos von Samen der Linien oe-SSII/GWD-O.s.X, deren Mehle ein Gehalt von in C6-Position der Glucosemoleküle gebundenem Phosphat von mindestens 6,0 nmol C6-Phosphat pro mg Stärke aufwiesen, wurden mittels der unter Allgemeine Methoden Punkt 10 beschriebenen Methode zur Keimung gebracht und anschließend zur Produktion von F2-Samen im Gewächshaus kultiviert. Zur Identifizierung von Nachkommen, die für die beiden integrierten T DNAs, vermittelnd eine erhöhte Aktivität eines Proteins mit der Aktivität einer Stärkesynthase II bzw. vermittelnd eine erhöhte Aktivität eines Proteins mit der Aktivität einer Glucan-Wasser-Dikinase homozygot waren, wurde das soeben für F1-Samen beschriebene Verfahren mit F2-Samen wiederholt. Anschließend wurden wiederum Embryos von Samen, deren Mehle ein Gehalt von in C6-Position der Glucosemoleküle gebundenem Phosphat von mindestens 6,0 nmol C6-Phosphat pro mg Frischgewicht des Samens aufwiesen zur Keimung gebracht und für die Produktion von F3-Samen im Gewächshaus kultiviert. Für einzelne F3-Samen, stammend von jeweils einer F2-Pflanze wurden folgende Ergebnisse erhalten:

**Tabelle 3**: Gehalt von in C6-Position der Glucosemoleküle gebundenem Phosphat in Mehlen, hergestellt aus einzelnen F3-Samen von Linien mit der Bezeichnung oe-SSII/GWD-O.s.-X; die durch Kreuzung der Elternlinien SSII-O.s.-19 (Mutter) mit Pflanzen der Linien oe-GWD-O.s.-X (Vater) hergestellt wurden, im Vergleich zu Mehlen, hergestellt aus einzelnen Samen von entsprechenden nicht genetisch modifizierten Wildtyp-Pflanzen der Varietät M202 (WT). Ebenfalls dargestellt ist der Gehalt von in C66-Position der Glucosemoleküle gebundenem Phosphat in Mehlen, hergestellt von einzelnen homozygoten Samen der einzelner Elternlinien.

| Bezeichnung der Pflanze | Nr. des F3-Samens | nmol C6-Phosphat pro mg Stärke |
|---|---|---|
| oe-SSII/GWD-O.s-1 | 1 | 9,7 |
| | 2 | 9,7 |
| | 3 | 10,0 |
| | 4 | 9,7 |
| | 5 | 9,8 |
| | 6 | 9,1 |
| | 7 | 8,4 |
| | 8 | 9,7 |
| | 9 | 9,9 |
| | 10 | 10,0 |
| | 11 | 9,8 |
| | 12 | 9,8 |
| oe-SSII/GWD-O.s.-2 | 1 | 10,4 |
| | 2 | 9,8 |
| | 3 | 10,9 |
| | 4 | 10,1 |
| | 5 | 11,2 |
| | 6 | 10,0 |
| | 7 | 11,0 |
| | 8 | 9,7 |
| | 9 | 10,4 |
| | 10 | 10,5 |
| | 11 | 11,9 |
| | 12 | 10,6 |

(fortgesetzt)

| Bezeichnung der Pflanze | Nr. des F3-Samens | nmol C6-Phosphat pro mg Stärke |
|---|---|---|
| oe-SSII/GWD-O.s.-3 | 1 | 12,5 |
| | 2 | 11,5 |
| | 3 | 11,3 |
| | 4 | 11,4 |
| | 5 | 11,0 |
| | 6 | 11,6 |
| | 7 | 11,5 |
| | 8 | 11,5 |
| | 9 | 12,1 |
| | 10 | 10,0 |
| | 11 | 11,5 |
| | 12 | 10,6 |
| SSII-O.s.-19 (Mutter) | 1 | 1,5 |
| | 2 | 1,7 |
| | 3 | 2,2 |
| | 4 | 1,9 |
| oe-GWD-O.s.-9 (Vater 3) | 1 | 3,3 |
| | 2 | 2,9 |
| | 3 | 3,3 |
| | 4 | 3,3 |
| WT | 1 | 0,5 |
| | 2 | 0,9 |

Die Tatsache, dass der Gehalt von in C6-Position der Glucosemoleküle gebundenem Phosphat in Mehlen, hergestellt aus einzelnen F3-Samen, die jeweils von einer F2-Pflanze der betreffenden Linie stammten, etwa gleich war, deutet darauf hin, dass die betreffenden F2-Pflanzen homozygot für die beiden integrierten T-DNAs sind.

F3-Samen von F2-Pflanzen der Linien oe-SSII/GWD-O.s.-1, oe-SSII/GWD-O.s.-2, oe-SSII/GWD-O.s.-3, die homozygot für die beiden integrierten T DNAs, vermittelnd eine erhöhte Aktivität eines Proteins mit der Aktivität einer Stärkesynthase II bzw. vermittelnd eine erhöhte Aktivität eines Proteins mit der Aktivität einer Glucan-Wasser-Dikinase waren, wurden nach der unter Allgemeine Methoden Punkt 6 beschriebenen Methode zu Mehlen verarbeitet. Aus einem Teil dieser Mehle wurde nach der unter Allgemeine Methoden Punkt 7 beschriebenen Methode Stärke isoliert. Anschließend wurde der Gehalt von in C6-Position der Glucosemoleküle gebundenem Phosphat in Mehlen und Stärke bestimmt. Es wurden folgende Ergebnisse erhalten:

**Tabelle 4:** Gehalt von in C6-Position der Glucosemoleküle gebundenem Phosphat in Mehlen bzw. Stärke, hergestellt aus Samen homozygoter Pflanzen von Linien mit der Bezeichnung oe-SSII/GWD-O.s.-X; die durch Kreuzung erzeugt wurden, im Vergleich zu Mehlen bzw. Stärke, hergestellt aus Samen der Elternlinien SSII-O.s.-19 (Mutter) und oe-GWD-O.s.-X (Vater) bzw. Wildtyp-Pflanzen der Varietät M202 (WT).

| Bezeichnung der Pflanze | nmol C6-Phosphat pro mg Stärke | nmol C6-Phosphat pro mg Stärke |
|---|---|---|
| oe-SSII/GWD-O.s-1 | 12,9 | 11,5 |
| oe-SSII/GWD-O.s.-2 | 13,4 | 12,6 |
| oe-SSII/GWD-O.s.-3 | 13,0 | 12,4 |
| SSII-O.s.-19 (Mutter) | 1,5 | 1,2 |

(fortgesetzt)

| Bezeichnung der Pflanze | nmol C6-Phosphat pro mg Stärke | nmol C6-Phosphat pro mg Stärke |
|---|---|---|
| oe-GWD-O.s.-2 (Vater 1) | 3,9 | 3,3 |
| oe-GWD-O.s.-4 (Vater 2) | 3,9 | 3,5 |
| oe-GWD-O.s.-9 (Vater 3) | 3,9 | 3,5 |
| WT | 1,1 | 0,4 |

Die Ermittlung des Heißwasser Quellvermögens von Mehlen bzw. Stärken, hergestellt aus F3-Samen der Linien oe-SSII/GWD-O.s.-X und bezüglich der T-DNA Integrationen homozygoten Pflanzen der Linien SSII-O.s.-19 und oe-GWD-O.s.-X und von Wildtyp-Pflanzen erfolgte nach der unter Allgemeine Methode Punkt 1 beschriebenen Methode. Für die Linien oe-SSII/GWD-O.s.-X wurde dabei in Abweichung von der unter Allgemeine Methoden Punkt 1 beschriebenen Methode die doppelte Menge an Wasser bezogen auf die Menge an Mehl bzw. Stärke eingesetzt, da bei Verwendung der unter Allgemeine Methode Punkt 1 angegebenen Menge an Wasser bei diesen Linien keine Trennung der gequollenen Substanz von dem wässrigen Überstand erkennbar war. Es wurden folgende Ergebnisse erhalten:

**Tabelle 5:** Heißwasser Quellvermögen von Mehlen bzw. Stärke, hergestellt aus Samen homozygoter Pflanzen von Linien mit der Bezeichnung oe-SSII/GWD-O.s.-X; die durch Kreuzung erzeugt wurden, im Vergleich zu Mehlen bzw. Stärke, hergestellt aus Samen der Elternlinien SSII-O.s.-19 (Mutter) und oe-GWD-O.s.-X (Vater) bzw. Wildtyp-Pflanzen der Varietät M202 (WT).

| Bezeichnung der Pflanze | Quellvermögen von Mehl [g/g] | Quellvermögen von Stärke [g/g] |
|---|---|---|
| oe-SSII/GWD-O.s-1 | 42,8 | 95,2 |
| oe-SSII/GWD-O.s.-2 | 41,1 | 128,3 |
| oe-SSII/GWD-O.s.-3 | 34,1 | 91,4 |
| SSII-O.s.-19 (Mutter) | 22,6 | 36,2 |
| oe-GWD-O.s.-2 (Vater 1) | 20,1 | 30,8 |
| oe-GWD-O.s.-4 (Vater 2) | 20,0 | 36,5 |
| oe-GWD-O.s.-9 (Vater 3) | 17,4 | 34,0 |
| WT | 16,3 | 27,7 |

**Patentansprüche**

1. Genetisch modifizierte Pflanzenzelle, **dadurch gekennzeichnet, dass** sie eine erhöhte Aktivität eines Proteins mit der Aktivität einer Stärkesynthase II und eine erhöhte Aktivität eines Proteins mit der Aktivität einer Glucan-Wasser-Dikinase aufweist im Vergleich zu genetisch nicht modifizierten Wildtyp-Pflanzenzellen.

EP 1 887 079 A1

**2.** Genetisch modifizierte Pflanzenzelle nach Anspruch 1, wobei die genetische Modifikation in der Einführung mindestens eines fremden Nucleinsäuremoleküls, in das Genom der Pflanze besteht.

**3.** Genetisch modifizierte Pflanzenzelle nach einem der Ansprüche 1 oder 2, die eine modifizierte Stärke synthetisiert im Vergleich zu Stärke, isoliert aus entsprechenden nicht genetisch modifizierten Wildtyp-Pflanzenzellen.

**4.** Genetisch modifizierte Pflanzenzelle nach einem der Ansprüche 1, 2 oder 3 die eine Stärke mit erhöhtem Heißwasser Quellvermögen synthetisiert.

**5.** Pflanze enthaltend genetisch modifizierte Pflanzenzellen nach einem der Ansprüche 1 bis 4.

**6.** Vermehrungsmaterial von Pflanzen nach Anspruch 5, enthaltend genetisch modifizierte Pflanzenzellen nach einem der Ansprüche 1 bis 4.

**7.** Verfahren zur Herstellung einer genetisch modifizierten Pflanze, worin

a) eine Pflanzenzelle, genetisch modifiziert wird, wobei die genetische Modifikation die folgenden Schritte i und ii in beliebiger Reihenfolge, einzeln oder gleichzeitig umfasst

i) Einführung einer genetischen Modifikation in die Pflanzenzelle, wobei die genetische Modifikation zur Erhöhung der Aktivität eines Proteins mit der enzymatischen Aktivität einer Stärkesynthase II im Vergleich zu entsprechenden nicht genetisch modifizierten Wildtyp-Pflanzenzellen führt,
ii) Einführung einer genetischen Modifikation in die Pflanzenzelle, wobei die genetische Modifikation zur Erhöhung der Aktivität eines Proteins mit der enzymatischen Aktivität einer Glucan-Wasser-Dikinase im Vergleich zu entsprechenden nicht genetisch modifizierten Wildtyp-Pflanzenzellen führt

b) aus Pflanzenzellen von Schritt a) eine Pflanze regeneriert wird;
c) gegebenenfalls weitere Pflanzen mit Hilfe der Pflanzen nach Schritt b) erzeugt werden,

wobei gegebenenfalls aus Pflanzen gemäß Schritt b) oder c) Pflanzenzellen isoliert werden und die Verfahrensschritte a) bis c) solange wiederholt werden, bis eine Pflanze erzeugt wurde, eine erhöhte Aktivität eines Proteins mit der Aktivität einer Stärkesynthase II und eine erhöhte Aktivität eines Proteins mit der Aktivität einer Glucan-Wasser Dikinase aufweist.

**8.** Verfahren zur Herstellung einer modifizierten Stärke, umfassend den Schritt der Extraktion der Stärke aus genetisch modifizierten Pflanzenzellen nach einem der Ansprüche 1 bis 4, Pflanzen nach Anspruch 5, Vermehrungsmaterial nach Anspruch 6 oder Pflanzen, erhältlich nach einem Verfahren nach Anspruch 7.

**9.** Verwendung von Pflanzen nach Anspruch 5, von Vermehrungsmaterial nach Anspruch 6 oder von Pflanzen, erhältlich nach einem Verfahren nach Anspruch 7, zur Herstellung von Stärke.

**10.** Modifizierte Stärke erhältlich nach einem Verfahren nach Anspruch 8.

**11.** Modifizierte Stärke **dadurch gekennzeichnet, dass** sie ein Heißwasser Quellvermögen von mindestens 110 g/g aufweist.

**12.** Verfahren zur Herstellung einer derivatisierten Stärke, worin modifizierte Stärke nach einem der Ansprüche 10 oder 11 nachträglich derivatisiert wird.

**13.** Derivatisierte Stärke, erhältlich nach einem Verfahren nach Anspruch 12.

**14.** Verwendung von modifizierter Stärke nach einem der Ansprüche 10 oder 11 zur Herstellung von derivatisierter Stärke.

**15.** Mehle, enthaltend eine modifizierte Stärke, erhältlich nach einem Verfahren nach Anspruch 8 oder enthaltend eine modifizierte Stärke nach einem der Ansprüche 10 oder 11.

**16.** Verfahren zur Herstellung von Mehlen, umfassend den Schritt des Mahlens von Pflanzen nach Anspruch 5, von Vermehrungsmaterial nach Anspruch 6 oder von Pflanzen, erhältlich nach einem Verfahren nach Anspruch 7.

17. Verwendung von genetisch modifizierten Pflanzenzellen nach einem der Ansprüche 1 bis 4, Pflanzen nach Anspruch 5, Vermehrungsmaterial nach Anspruch 6 oder Pflanzen, erhältlich nach einem Verfahren nach Anspruch 7 zur Herstellung von Mehlen.

## Bestimmung von SS2 Aktivität in transgenen Linien

**oe-SSIIa-O.s.-5**

WT  pur  1:2  1:4  1:8

Zweifache Erhöhung gegenüber (WT)

**oe-SSIIa-O.s.-12**

WT  pur  1:2  1.4  1:6  1:8 1:10 1:20  WT

sechsfache Erhöhung gegenüber (WT)

**oe-SSIIa-O.s.-19**

WT  pur  1:2  1:5  1:10  1:20 1:501:100  WT

zehnfache Erhöhung gegenüber (WT)

**Fig. 1**

Fig. 2

Fig. 3

**Europäisches**
**Patentamt**

**EUROPÄISCHER RECHERCHENBERICHT**

Nummer der Anmeldung

EP 06 09 0134

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | WO 2005/095617 A2 (BAYER CROPSCIENCE GMBH [DE]; FROHBERG CLAUS [DE]; KOETTING OLIVER [DE]) 13. Oktober 2005 (2005-10-13) * Zusammenfassung * ----- | 1-17 | INV. C12N5/10 C12N15/29 A01H5/00 C08B30/04 A23L1/0522 |
| A | WO 2004/056999 A (BAYER CROPSCIENCE GMBH [DE]; HOEHNE MICHAELA [CH]; FROHBERG CLAUS [DE]) 8. Juli 2004 (2004-07-08) * Zusammenfassung * ----- | 1-17 | ADD. C12N9/12 C12N9/10 |
| D,A | WO 2005/002359 A2 (SYNGENTA PARTICIPATIONS AG [CH]; LANAHAN MICHAEL B [US]; BASU SHIP S []) 13. Januar 2005 (2005-01-13) * Seite 2 * ----- | 1-17 | |
| A | BLENNOW ET AL: "Structure function relationships of transgenic starches with engineered phosphate substitution and starch branching" INTERNATIONAL JOURNAL OF BIOLOGICAL MACROMOLECULES, BUTTERWORTH & CO., GUILDFORD, GB, Bd. 36, Nr. 3, August 2005 (2005-08), Seiten 159-168, XP005001313 ISSN: 0141-8130 * Zusammenfassung * ----- | 1-17 | RECHERCHIERTE SACHGEBIETE (IPC) C12N A01H C08B |
| A | MIKKELSEN R ET AL: "Functional characterization of alpha-glucan, water dikinase, the starch phosphorylating enzyme" BIOCHEMICAL JOURNAL, THE BIOCHEMICAL SOCIETY, LONDON, GB, Bd. 377, Nr. 2, 15. Januar 2004 (2004-01-15), Seiten 525-532, XP002339213 ISSN: 0264-6021 * Zusammenfassung * ----- -/-- | 1-17 | |

Der vorliegende Recherchenbericht wurde für alle Patentansprüche erstellt

| Recherchenort | Abschlußdatum der Recherche | Prüfer |
|---|---|---|
| München | 4. Januar 2007 | Bilang, Jürg |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
........................................................
& : Mitglied der gleichen Patentfamilie, übereinstimmendes Dokument

EPO FORM 1503 03.82 (P04C03)

**Europäisches Patentamt**

**EUROPÄISCHER RECHERCHENBERICHT**

Nummer der Anmeldung

EP 06 09 0134

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| D,A | KOETTING O ET AL: "Identification of a novel enzyme required for starch metabolism in Arabidopsis leaves. The phosphoglucan, water dikinase" PLANT PHYSIOLOGY, AMERICAN SOCIETY OF PLANT PHYSIOLOGISTS, ROCKVILLE, MD, US, Bd. 137, Nr. 1, Januar 2005 (2005-01), Seiten 242-252, XP002339144 ISSN: 0032-0889 * Zusammenfassung * ----- | 1-17 | |
| A | MORELL M K ET AL: "Barley sex6 mutants lack starch synthase IIa activity and contain a starch with novel properties" PLANT JOURNAL, BLACKWELL SCIENTIFIC PUBLICATIONS, OXFORD, GB, Bd. 34, Nr. 2, April 2003 (2003-04), Seiten 173-185, XP002346241 ISSN: 0960-7412 * Zusammenfassung * ----- | 1-17 | |
| A | WADUGE ET AL: "Effect of annealing on the structure and physicochemical properties of barley starches of varying amylose content" FOOD RESEARCH INTERNATIONAL, ELSEVIER APPLIED SCIENCE, BARKING, GB, Bd. 39, Nr. 1, Januar 2006 (2006-01), Seiten 59-77, XP005115117 ISSN: 0963-9969 * Zusammenfassung * ----- | 1-17 | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 4. Januar 2007 | Bilang, Jürg |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 06 09 0134

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

04-01-2007

| Im Recherchenbericht angeführtes Patentdokument | Datum der Veröffentlichung | Mitglied(er) der Patentfamilie | Datum der Veröffentlichung |
|---|---|---|---|
| WO 2005095617 A2 | 13-10-2005 | AU 2005229361 A1<br>CA 2558079 A1 | 13-10-2005<br>13-10-2005 |
| WO 2004056999 A | 08-07-2004 | AU 2003293991 A1<br>CA 2505776 A1<br>JP 2006511235 T<br>US 2006130181 A1 | 14-07-2004<br>08-07-2004<br>06-04-2006<br>15-06-2006 |
| WO 2005002359 A2 | 13-01-2005 | BR PI0410544 A<br>CA 2526480 A1<br>CN 1826412 A<br>EP 1629102 A2 | 20-06-2006<br>13-01-2005<br>30-08-2006<br>01-03-2006 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5300145 A
- WO 0234923 A
- WO 052359 A
- WO 05095617 A
- US 4280851 A
- US 6299907 B
- WO 9711188 A
- WO 0077229 A
- US 6462256 B

- EP 120516 A
- WO 9506128 A
- EP 0513849 A
- EP 0465875 A
- EP 0292435 A
- WO 9307279 A
- WO 9745545 A
- WO 05030941 A
- WO 05095619 A


**In der Beschreibung aufgeführte Nicht-Patentliteratur**

- **BALL ; MORELL.** *Annu. Rev, Plant Biol.,* 2003, vol. 54, 207-233
- **TELTOW et al.** *J. Expt. Bot.,* 2004, vol. 55 (406), 2131-2145
- **JANE et al.** *Cereal Foods World,* 1996, vol. 41 (11), 827-832
- **TAKEDA ; HIZUKURI.** *Starch/Stärke,* 1971, vol. 23, 267-272
- **BLENNOW et al.** *Int. J. of Biological Macromolecules,* 2000, vol. 27, 211-218
- **BLENNOW et al.** *Carbohydrate Polymers,* 2000, vol. 41, 163-174
- **RITTE et al.** *PNAS,* 2002, vol. 99, 7166-7171
- **LORBERTH et al.** *Nature Biotechnology,* 1998, vol. 16, 473-477
- **KÖTTING et al.** *Plant Physiol.,* 2005, vol. 137, 2424-252
- **BAUNSGAARD et al.** *Plant Journal,* 2005, vol. 41, 595-605
- **NARAYANA ; MOORTHY.** *Starch/Stärke,* 2002, vol. 54, 559-592
- **VON LEACH et al.** *Cereal Chemistry,* 1959, vol. 36, 534-544
- **SINGH et al.** *Journal of the Science of Food and Agriculture,* 2002, vol. 82, 1376-1383
- **TAKIZAWA et al.** *Brazilian Archives of Biology and Technology,* 2004, vol. 47 (6), 921-931
- **LEACH et al.** *Cereal Chemistry,* 1959, vol. 36, 534-544
- **YAMAMORI ; QUYNH.** *Theor Appl Genet,* 2000, vol. 100, 23-38
- **YASUI et al.** *Starch/Stärke,* 2002, vol. 54, 179-184
- **SHI et al.** *J. Cereal Sci.,* 1998, vol. 27, 289-299

- **SODHI ; SINGH.** *Food Chemistry,* 2003, vol. 80, 99-108
- **CHEN et al.** *Starch/Stärke,* 2003, vol. 55, 203-212
- **VAN HUNG ; MORITA.** *Starch/Stärke,* 2005, vol. 57, 413-420
- **LIU et al.** *Starch/Stärke,* 1999, vol. 52, 249-252
- Bioanalytik, Spektrum akad. Verlag, Heidelberg, 1998
- **RITTE et al.** *Plant Journal,* 2000, vol. 21, 387-391
- Untersuchungen der Expression und Funktion der Stärkesynthase II (SSII) aus Weiten (Triticum aestivum. **WALTER.** Dissertation am Fachbereich Biologie der Universität Hamburg
- **MIKKELSEN et al.** *Biochemical Journal,* 2004, vol. 377, 525-532
- **NISHI et al.** *Plant Physiology,* 2001, vol. 127, 459-472
- **LI et al.** *Funct Integr Genomics,* 2003, vol. 3, 76-85
- **TIEN-SHIN YU et al.** *Plant Cell,* 2001, vol. 13, 1907-1918
- **MIKKELSEN ; BLENNOW.** *Biochemical Journal,* 2005, vol. 385, 355-361
- **MIKKELSEN et al.** *Biochemistry,* 2006, vol. 45, 4674-4682
- **SAMBROK et al.** Molecular Cloning, A Laboratory Manual. Cold Spring Harbour Laboratory Press, 2001
- **AUSUBEL et al.** Short Protocols in Molecular Biology. John Wiley & Sons, 2002
- *The Binary Plant Vector System,* 1985
- **FRALEY et al.** *Crit. Rev. Plant Sci.,* vol. 4, 1-46

- **AN et al.** *EMBO J.,* 1985, vol. 4, 277-287
- **ROCHA-SOSA et al.** *EMBO J.,* 1989, vol. 8, 29-33
- **CHAN et al.** *Plant Mol. Biol.,* 1993, vol. 22, 491-506
- **HIEI et al.** *Plant J.,* 1994, vol. 6, 271-282
- **DENG et al.** *Science,* 1990, vol. 33, 28-34
- **WILMINK et al.** *Plant Cell Reports,* 1992, vol. 11, 76-80
- **MAY et al.** *Bio/Technology,* 1995, vol. 13, 486-492
- **CONNER ; DOMISSE.** *Int. J. Plant Sci.,* 1992, vol. 153, 550-555
- **RITCHIE et al.** *Transgenic Res.,* 1993, vol. 2, 252-265
- **WAN ; LEMAUX.** *Plant Physiol.,* 1994, vol. 104, 37-48
- **VASIL et al.** *Bio/Technology,* 1993, vol. 11, 1553-1558
- **RITALA et al.** *Plant Mol. Biol.,* 1994, vol. 24, 317-325
- **SPENCER et al.** *Theor. Appl. Genet.,* 1990, vol. 79, 625-631
- **FROMM et al.** *Biotechnology,* 1990, vol. 8, 833-844
- **GORDON-KAMM et al.** *Plant Cell,* 1990, vol. 2, 603-618
- **KOZIEL et al.** *Biotechnology,* 1993, vol. 11, 194-200
- **MOROC.** *Theor. Appl. Genet.,* 1990, vol. 80, 721-726
- **KRENS et al.** *Nature,* 1982, vol. 296, 72-74
- **NEHRA et al.** *Plant J.,* 1994, vol. 5, 285-297
- **BECKER et al.** *Plant Journal,* 1994, vol. 5, 299-307
- Plant Cell Culture Protocols. Humana Press, 1999
- **THOMPSON et al.** *Nucleic Acids Research,* 1994, vol. 22, 4673-4680
- **ROCHA-SOSA et al.** *EMBO J.,* 1989, vol. 8, 23-29
- **STOCKHAUS et al.** *Proc. Natl. Acad. Sci. USA,* 1987, vol. 84, 7943-7947
- **STOCKHAUS et al.** *EMBO J.,* 1989, vol. 8, 2445-2451
- **PEDERSEN et al.** *Cell,* 1982, vol. 29, 1015-1026
- **QUATROCCIO et al.** *Plant Mol. Biol.,* 1990, vol. 15, 81-93
- **LEISY et al.** *Plant Mol. Biol.,* 1990, vol. 14, 41-50
- **ZHENG et al.** *Plant J.,* 1993, vol. 4, 357-366
- **YOSHIHARA et al.** *FEBS Lett.,* 1996, vol. 383, 213-218
- **NAKASE et al.** *Gene,* 1996, vol. 170 (2), 223-226
- **QU ; TAKAIWA.** *Plant Biotechnology Journal,* 2004, vol. 2 (2), 113-125
- **WERR et al.** *EMBO J.,* 1985, vol. 4, 1373-1380
- **FIEDLER et al.** *Plant Mol. Biol.,* 1993, vol. 22, 669-679
- **BÄUMLEIN et al.** *Mol. Gen. Genet.,* 1991, vol. 225, 459-467
- **GIELEN et al.** *EMBO J.,* 1989, vol. 8, 23-29
- **CALLIS et al.** *Genes Devel.,* 1987, vol. 1, 1183-1200
- **LUEHRSEN ; WALBOT.** *Mol. Gen. Genet.,* 1991, vol. 225, 81-93
- **RETHMEIER et al.** *Plant Journal.,* 1997, vol. 12 (4), 895-899
- **ROSE ; BELIAKOFF.** *Plant Physiol.,* 2000, vol. 122 (2), 535-542
- **VASIL et al.** *Plant Physiol.,* 1989, vol. 91, 1575-1579
- **XU et al.** *Science in China Series C,* 2003, vol. 46 (6), 561-569
- **KASEMUSUWAN ; JANE.** *Cereal Chemistry,* 1996, vol. 73, 702-707
- **STARCH.** Chemistry and Technology. Academic Press Inc. London Ltd, 1994, 412-468
- **VON WATSON.** Tapioca, Arrowroot und Sago Stärken: Herstellung. 469-479
- **VON CORBISHLEY ; MILLER.** Kartoffelstärke: Herstellung und Verwendungen. 479-490
- **VON MITCH.** Weizenstärke: Herstellung, Modifizierung und Verwendungen. 491-506
- **VON KNIGHT ; OSON.** Reisstärke: Herstellung und Verwendungen. 507-528
- **ECKHOFF et al.** *Cereal Chem.,* 1996, vol. 73, 54-57
- **CORN.** Chemistry and Technology. 1987, vol. 16, 479-499
- **NIELSEN et al.** *Plant Physiol.,* 1994, vol. 105, 111-117
- **AMES.** *Methods in Enzymology,* 1966, vol. VIII, 115-118
- **HIEI et al.** *Plant Journal,* 1994, vol. 6 (2), 271-282
- **ISHIDA et al.** *Nature Biotechnology,* 1996, vol. 14, 745-750
- **WANG ; WANG.** *Journal of Cereal Science,* 2004, vol. 39, 291-296
- **BRADFORD.** *Anal Biochem,* 1976, vol. 72, 248-254

SEQUENCE LISTING

<110>  Bayer CropScience GmbH

<120>  Genetisch modifizierte Pflanzen, die eine Stärke mit erhöhtem
       Quellvermögen synthetisieren

<130>  BCS 06-5009

<160>  6

<170>  PatentIn version 3.3

<210>  1
<211>  4851
<212>  DNA
<213>  Solanum tuberosum


<220>
<221>  transit_peptide
<222>  (1)..(77)

<220>
<221>  CDS
<222>  (105)..(4499)

<300>
<308>  EMBL / Y09533
<309>  1998-07-30
<313>  (1)..(4499)

<400>  1
catcttcatc gaatttctcg aagcttcttc gctaatttcc tggtttcttc actcaaaatc       60

gacgtttcta gctgaacttg agtgaattaa gccagtggga ggat atg agt aat tcc       116
                                            Met Ser Asn Ser
                                            1


tta ggg aat aac ttg ctg tac cag gga ttc cta acc tca aca gtg ttg       164
Leu Gly Asn Asn Leu Leu Tyr Gln Gly Phe Leu Thr Ser Thr Val Leu
5                   10                  15                  20

gaa cat aaa agt aga atc agt cct cct tgt gtt gga ggc aat tct ttg       212
Glu His Lys Ser Arg Ile Ser Pro Pro Cys Val Gly Gly Asn Ser Leu
                25                  30                  35

ttt caa caa caa gtg atc tcg aaa tca cct tta tca act gag ttt cga       260
Phe Gln Gln Gln Val Ile Ser Lys Ser Pro Leu Ser Thr Glu Phe Arg
            40                  45                  50

ggt aac agg tta aag gtg cag aaa aag aaa ata cct atg gaa aag aag       308
Gly Asn Arg Leu Lys Val Gln Lys Lys Lys Ile Pro Met Glu Lys Lys
        55                  60                  65

cgt gct ttt tct agt tct cct cat gct gta ctt acc act gat acc tct       356
Arg Ala Phe Ser Ser Ser Pro His Ala Val Leu Thr Thr Asp Thr Ser
        70                  75                  80

tct gag cta gca gaa aag ttc agt cta ggg ggg aat att gag cta cag       404
Ser Glu Leu Ala Glu Lys Phe Ser Leu Gly Gly Asn Ile Glu Leu Gln
85                  90                  95                  100

gtt gat gtt agg cct ccc act tca ggt gat gtg tcc ttt gtg gat ttt       452
Val Asp Val Arg Pro Pro Thr Ser Gly Asp Val Ser Phe Val Asp Phe
                105                 110                 115

caa gta aca aat ggt agt gat aaa ctg ttt ttg cac tgg ggg gca gta       500

33

```
        Gln Val Thr Asn Gly Ser Asp Lys Leu Phe Leu His Trp Gly Ala Val
                    120             125                 130

aaa ttc ggg aaa gaa aca tgg tct ctt ccg aat gat cgt cca gat ggg      548
Lys Phe Gly Lys Glu Thr Trp Ser Leu Pro Asn Asp Arg Pro Asp Gly
        135             140                 145

acc aaa gtg tac aag aac aaa gca ctt aga act cca ttt gtt aaa tct      596
Thr Lys Val Tyr Lys Asn Lys Ala Leu Arg Thr Pro Phe Val Lys Ser
    150             155                 160

ggc tct aac tcc atc ctg aga ctg gag ata cga gac act gct atc gaa      644
Gly Ser Asn Ser Ile Leu Arg Leu Glu Ile Arg Asp Thr Ala Ile Glu
165             170                 175                 180

gct att gag ttt ctc ata tac gat gaa gcc cac gat aaa tgg ata aag      692
Ala Ile Glu Phe Leu Ile Tyr Asp Glu Ala His Asp Lys Trp Ile Lys
                185                 190                 195

aat aat ggt ggt aat ttt cgt gtc aaa ttg tca aga aaa gag ata cga      740
Asn Asn Gly Gly Asn Phe Arg Val Lys Leu Ser Arg Lys Glu Ile Arg
        200                 205                 210

ggc cca gat gtt tct gtt cct gag gag ctt gta cag atc caa tca tat      788
Gly Pro Asp Val Ser Val Pro Glu Glu Leu Val Gln Ile Gln Ser Tyr
        215                 220                 225

ttg agg tgg gag agg aag gga aaa cag aat tac ccc cct gag aaa gag      836
Leu Arg Trp Glu Arg Lys Gly Lys Gln Asn Tyr Pro Pro Glu Lys Glu
    230                 235                 240

aag gag gaa tat gag gct gct cga act gtg cta cag gag gaa ata gct      884
Lys Glu Glu Tyr Glu Ala Ala Arg Thr Val Leu Gln Glu Glu Ile Ala
245                 250                 255                 260

cgt ggt gct tcc ata cag gac att cga gca agg cta aca aaa act aat      932
Arg Gly Ala Ser Ile Gln Asp Ile Arg Ala Arg Leu Thr Lys Thr Asn
                265                 270                 275

gat aaa agt caa agc aaa gaa gag cct ctt cat gta aca aag agt gat      980
Asp Lys Ser Gln Ser Lys Glu Glu Pro Leu His Val Thr Lys Ser Asp
        280                 285                 290

ata cct gat gac ctt gcc caa gca caa gct tac att agg tgg gag aaa      1028
Ile Pro Asp Asp Leu Ala Gln Ala Gln Ala Tyr Ile Arg Trp Glu Lys
        295                 300                 305

gca gga aag ccg aac tat cct cca gaa aag caa att gaa gaa ctc gaa      1076
Ala Gly Lys Pro Asn Tyr Pro Pro Glu Lys Gln Ile Glu Glu Leu Glu
        310                 315                 320

gaa gca aga aga gaa ttg caa ctt gag ctt gag aaa ggc att acc ctt      1124
Glu Ala Arg Arg Glu Leu Gln Leu Glu Leu Glu Lys Gly Ile Thr Leu
325                 330                 335                 340

gat gag ttg cgg aaa acg att aca aaa ggg gag ata aaa act aag gtg      1172
Asp Glu Leu Arg Lys Thr Ile Thr Lys Gly Glu Ile Lys Thr Lys Val
                345                 350                 355

gaa aag cac ctg aaa aga agt tct ttt gcc gtt gaa aga atc caa aga      1220
Glu Lys His Leu Lys Arg Ser Ser Phe Ala Val Glu Arg Ile Gln Arg
                360                 365                 370

aag aag aga gac ttt ggg cat ctt att aat aag tat act tcc agt cct      1268
Lys Lys Arg Asp Phe Gly His Leu Ile Asn Lys Tyr Thr Ser Ser Pro
                375                 380                 385

gca gta caa gta caa aag gtc ttg gaa gaa cca cca gcc tta tct aaa      1316
```

34

```
            Ala Val Gln Val Gln Lys Val Leu Glu Glu Pro Pro Ala Leu Ser Lys
                390                 395                 400

            att aag ctg tat gcc aag gag aag gag gag cag att gat gat ccg atc      1364
            Ile Lys Leu Tyr Ala Lys Glu Lys Glu Glu Gln Ile Asp Asp Pro Ile
            405                 410                 415                 420

            cta aat aaa aag atc ttt aag gtc gat gat ggg gag cta ctg gta ctg      1412
            Leu Asn Lys Lys Ile Phe Lys Val Asp Asp Gly Glu Leu Leu Val Leu
                        425                 430                 435

            gta gca aag tcc tct ggg aag aca aaa gta cat cta gct aca gat ctg      1460
            Val Ala Lys Ser Ser Gly Lys Thr Lys Val His Leu Ala Thr Asp Leu
                        440                 445                 450

            aat cag cca att act ctt cac tgg gca tta tcc aaa agt cct gga gag      1508
            Asn Gln Pro Ile Thr Leu His Trp Ala Leu Ser Lys Ser Pro Gly Glu
                    455                 460                 465

            tgg atg gta cca cct tca agc ata ttg cct cct ggg tca att att tta      1556
            Trp Met Val Pro Pro Ser Ser Ile Leu Pro Pro Gly Ser Ile Ile Leu
                470                 475                 480

            gac aag gct gcc gaa aca cct ttt tca gcc agt tct tct gat ggt cta      1604
            Asp Lys Ala Ala Glu Thr Pro Phe Ser Ala Ser Ser Ser Asp Gly Leu
            485                 490                 495                 500

            act tct aag gta caa tct ttg gat ata gta att gaa gat ggc aat ttt      1652
            Thr Ser Lys Val Gln Ser Leu Asp Ile Val Ile Glu Asp Gly Asn Phe
                        505                 510                 515

            gtg ggg atg cca ttt gtt ctt ttg tct ggt gaa aaa tgg att aag aac      1700
            Val Gly Met Pro Phe Val Leu Leu Ser Gly Glu Lys Trp Ile Lys Asn
                        520                 525                 530

            caa ggg tcg gat ttc tat gtt ggc ttc agt gct gca tcc aaa tta gca      1748
            Gln Gly Ser Asp Phe Tyr Val Gly Phe Ser Ala Ala Ser Lys Leu Ala
                        535                 540                 545

            ctc aag gct gct ggg gat ggc agt gga act gca aag tct tta ctg gat      1796
            Leu Lys Ala Ala Gly Asp Gly Ser Gly Thr Ala Lys Ser Leu Leu Asp
                550                 555                 560

            aaa ata gca gat atg gaa agt gag gct cag aag tca ttt atg cac cgg      1844
            Lys Ile Ala Asp Met Glu Ser Glu Ala Gln Lys Ser Phe Met His Arg
            565                 570                 575                 580

            ttt aat att gca gct gac ttg ata gaa gat gcc act agt gct ggt gaa      1892
            Phe Asn Ile Ala Ala Asp Leu Ile Glu Asp Ala Thr Ser Ala Gly Glu
                        585                 590                 595

            ctt ggt ttt gct gga att ctt gta tgg atg agg ttc atg gct aca agg      1940
            Leu Gly Phe Ala Gly Ile Leu Val Trp Met Arg Phe Met Ala Thr Arg
                        600                 605                 610

            caa ctg ata tgg aac aaa aac tat aac gta aaa cca cgt gaa ata agc      1988
            Gln Leu Ile Trp Asn Lys Asn Tyr Asn Val Lys Pro Arg Glu Ile Ser
                    615                 620                 625

            aag gct cag gac aga ctt aca gac ttg ttg cag aat gct ttc acc agt      2036
            Lys Ala Gln Asp Arg Leu Thr Asp Leu Leu Gln Asn Ala Phe Thr Ser
                630                 635                 640

            cac cct cag tac cgt gaa att ttg cgg atg att atg tca act gtt gga      2084
            His Pro Gln Tyr Arg Glu Ile Leu Arg Met Ile Met Ser Thr Val Gly
            645                 650                 655                 660

            cgt gga ggt gaa ggg gat gta gga cag cga att agg gat gaa att ttg      2132
```

```
Arg Gly Gly Glu Gly Asp Val Gly Gln Arg Ile Arg Asp Glu Ile Leu
                  665             670             675

gtc atc cag agg aac aat gac tgc aag ggt ggt atg atg caa gaa tgg    2180
Val Ile Gln Arg Asn Asn Asp Cys Lys Gly Gly Met Met Gln Glu Trp
            680             685             690

cat cag aaa ttg cat aat aat act agt cct gat gat gtt gtg atc tgt    2228
His Gln Lys Leu His Asn Asn Thr Ser Pro Asp Asp Val Val Ile Cys
            695             700             705

cag gca tta att gac tac atc aag agt gat ttt gat ctt ggt gtt tat    2276
Gln Ala Leu Ile Asp Tyr Ile Lys Ser Asp Phe Asp Leu Gly Val Tyr
        710             715             720

tgg aaa acc ctg aat gag aac gga ata aca aaa gag cgt ctt ttg agt    2324
Trp Lys Thr Leu Asn Glu Asn Gly Ile Thr Lys Glu Arg Leu Leu Ser
725             730             735             740

tat gac cgt gct atc cat tct gaa cca aat ttt aga gga gat caa aag    2372
Tyr Asp Arg Ala Ile His Ser Glu Pro Asn Phe Arg Gly Asp Gln Lys
                745             750             755

ggt ggt ctt ttg cgt gat tta ggt cac tat atg aga aca ttg aag gca    2420
Gly Gly Leu Leu Arg Asp Leu Gly His Tyr Met Arg Thr Leu Lys Ala
            760             765             770

gtt cat tca ggt gca gat ctt gag tct gct att gca aac tgc atg ggc    2468
Val His Ser Gly Ala Asp Leu Glu Ser Ala Ile Ala Asn Cys Met Gly
            775             780             785

tac aaa act gag gga gaa ggc ttt atg gtt gga gtc cag ata aat cct    2516
Tyr Lys Thr Glu Gly Glu Gly Phe Met Val Gly Val Gln Ile Asn Pro
        790             795             800

gta tca ggc ttg cca tct ggc ttt cag gac ctc ctc cat ttt gtc tta    2564
Val Ser Gly Leu Pro Ser Gly Phe Gln Asp Leu Leu His Phe Val Leu
805             810             815             820

gac cat gtg gaa gat aaa aat gtg gaa act ctt ctt gag aga ttg cta    2612
Asp His Val Glu Asp Lys Asn Val Glu Thr Leu Leu Glu Arg Leu Leu
                825             830             835

gag gct cgt gag gag ctt agg ccc ttg ctt ctc aaa cca aac aac cgt    2660
Glu Ala Arg Glu Glu Leu Arg Pro Leu Leu Leu Lys Pro Asn Asn Arg
            840             845             850

cta aag gat ctg ctg ttt ttg gac ata gca ctt gat tct aca gtt aga    2708
Leu Lys Asp Leu Leu Phe Leu Asp Ile Ala Leu Asp Ser Thr Val Arg
            855             860             865

aca gca gta gaa agg gga tat gaa gaa ttg aac aac gct aat cct gag    2756
Thr Ala Val Glu Arg Gly Tyr Glu Glu Leu Asn Asn Ala Asn Pro Glu
        870             875             880

aaa atc atg tac ttc atc tcc ctc gtt ctt gaa aat ctc gca ctc tct    2804
Lys Ile Met Tyr Phe Ile Ser Leu Val Leu Glu Asn Leu Ala Leu Ser
885             890             895             900

gtg gac gat aat gaa gat ctt gtt tat tgc ttg aag gga tgg aat caa    2852
Val Asp Asp Asn Glu Asp Leu Val Tyr Cys Leu Lys Gly Trp Asn Gln
                905             910             915

gct ctt tca atg tcc aat ggt ggg gac aac cat tgg gct tta ttt gca    2900
Ala Leu Ser Met Ser Asn Gly Gly Asp Asn His Trp Ala Leu Phe Ala
            920             925             930

aaa gct gtg ctt gac aga acc cgt ctt gca ctt gca agc aag gca gag    2948
```

```
        Lys Ala Val Leu Asp Arg Thr Arg Leu Ala Leu Ala Ser Lys Ala Glu
                    935             940             945

        tgg tac cat cac tta ttg cag cca tct gcc gaa tat cta gga tca ata    2996
        Trp Tyr His His Leu Leu Gln Pro Ser Ala Glu Tyr Leu Gly Ser Ile
            950             955             960

        ctt ggg gtg gac caa tgg gct ttg aac ata ttt act gaa gaa att ata    3044
        Leu Gly Val Asp Gln Trp Ala Leu Asn Ile Phe Thr Glu Glu Ile Ile
        965             970             975             980

        cgt gct gga tca gca gct tca tta tcc tct ctt ctt aat aga ctc gat    3092
        Arg Ala Gly Ser Ala Ala Ser Leu Ser Ser Leu Leu Asn Arg Leu Asp
                        985             990             995

        ccc gtg ctt cgg aaa act gca aat cta gga agt tgg cag att atc        3137
        Pro Val Leu Arg Lys Thr Ala Asn Leu Gly Ser Trp Gln Ile Ile
                    1000            1005            1010

        agt cca gtt gaa gcc gtt gga tat gtt gtc gtt gtg gat gag ttg        3182
        Ser Pro Val Glu Ala Val Gly Tyr Val Val Val Val Asp Glu Leu
                    1015            1020            1025

        ctt tca gtt cag aat gaa atc tac gag aag ccc acg atc tta gta        3227
        Leu Ser Val Gln Asn Glu Ile Tyr Glu Lys Pro Thr Ile Leu Val
                    1030            1035            1040

        gca aaa tct gtt aaa gga gag gag gaa att cct gat ggt gct gtt        3272
        Ala Lys Ser Val Lys Gly Glu Glu Glu Ile Pro Asp Gly Ala Val
                    1045            1050            1055

        gcc ctg ata aca cca gac atg cca gat gtt ctt tca cat gtt tct        3317
        Ala Leu Ile Thr Pro Asp Met Pro Asp Val Leu Ser His Val Ser
                    1060            1065            1070

        gtt cga gct aga aat ggg aag gtt tgc ttt gct aca tgc ttt gat        3362
        Val Arg Ala Arg Asn Gly Lys Val Cys Phe Ala Thr Cys Phe Asp
                    1075            1080            1085

        ccc aat ata ttg gct gac ctc caa gca aag gaa gga agg att ttg        3407
        Pro Asn Ile Leu Ala Asp Leu Gln Ala Lys Glu Gly Arg Ile Leu
                    1090            1095            1100

        ctc tta aag cct aca cct tca gac ata atc tat agt gag gtg aat        3452
        Leu Leu Lys Pro Thr Pro Ser Asp Ile Ile Tyr Ser Glu Val Asn
                    1105            1110            1115

        gag att gag ctc caa agt tca agt aac ttg gta gaa gct gaa act        3497
        Glu Ile Glu Leu Gln Ser Ser Ser Asn Leu Val Glu Ala Glu Thr
                    1120            1125            1130

        tca gca aca ctt aga ttg gtg aaa aag caa ttt ggt ggt tgt tac        3542
        Ser Ala Thr Leu Arg Leu Val Lys Lys Gln Phe Gly Gly Cys Tyr
                    1135            1140            1145

        gca ata tca gca gat gaa ttc aca agt gaa atg gtt gga gct aaa        3587
        Ala Ile Ser Ala Asp Glu Phe Thr Ser Glu Met Val Gly Ala Lys
                    1150            1155            1160

        tca cgt aat att gca tat ctg aaa gga aaa gtg cct tcc tcg gtg        3632
        Ser Arg Asn Ile Ala Tyr Leu Lys Gly Lys Val Pro Ser Ser Val
                    1165            1170            1175

        gga att cct acg tca gta gct ctt cca ttt gga gtc ttt gag aaa        3677
        Gly Ile Pro Thr Ser Val Ala Leu Pro Phe Gly Val Phe Glu Lys
                    1180            1185            1190

        gta ctt tca gac gac ata aat cag gga gtg gca aaa gag ttg caa        3722
```

```
Val Leu Ser Asp  Asp Ile Asn Gln Gly  Val Ala Lys Glu Leu  Gln
            1195              1200              1205

att ctg atg aaa  aaa cta tct gaa gga  gac ttc agc gct ctt  ggt   3767
Ile Leu Met Lys  Lys Leu Ser Glu Gly  Asp Phe Ser Ala Leu  Gly
            1210              1215              1220

gaa att cgc aca  acg gtt tta gat ctt  tca gca cca gct caa  ttg   3812
Glu Ile Arg Thr  Thr Val Leu Asp Leu  Ser Ala Pro Ala Gln  Leu
            1225              1230              1235

gtc aaa gag ctg  aag gag aag atg cag  ggt tct ggc atg cct  tgg   3857
Val Lys Glu Leu  Lys Glu Lys Met Gln  Gly Ser Gly Met Pro  Trp
            1240              1245              1250

cct ggt gat gaa  ggt cca aag cgg tgg  gaa caa gca tgg atg  gcc   3902
Pro Gly Asp Glu  Gly Pro Lys Arg Trp  Glu Gln Ala Trp Met  Ala
            1255              1260              1265

ata aaa aag gtg  tgg gct tca aaa tgg  aat gag aga gca tac  ttc   3947
Ile Lys Lys Val  Trp Ala Ser Lys Trp  Asn Glu Arg Ala Tyr  Phe
            1270              1275              1280

agc aca agg aag  gtg aaa ctg gat cat  gac tat ctg tgc atg  gct   3992
Ser Thr Arg Lys  Val Lys Leu Asp His  Asp Tyr Leu Cys Met  Ala
            1285              1290              1295

gtc ctt gtt caa  gaa ata ata aat gct  gat tat gca ttt gtc  att   4037
Val Leu Val Gln  Glu Ile Ile Asn Ala  Asp Tyr Ala Phe Val  Ile
            1300              1305              1310

cac aca acc aac  cca tct tcc gga gac  gac tca gaa ata tat  gcc   4082
His Thr Thr Asn  Pro Ser Ser Gly Asp  Asp Ser Glu Ile Tyr  Ala
            1315              1320              1325

gag gtg gtc agg  ggc ctt ggg gaa aca  ctt gtt gga gct tat  cca   4127
Glu Val Val Arg  Gly Leu Gly Glu Thr  Leu Val Gly Ala Tyr  Pro
            1330              1335              1340

gga cgt gct ttg  agt ttt atc tgc aag  aaa aag gat ctc aac  tct   4172
Gly Arg Ala Leu  Ser Phe Ile Cys Lys  Lys Lys Asp Leu Asn  Ser
            1345              1350              1355

cct caa gtg tta  ggt tac cca agc aaa  ccg atc ggc ctt ttc  ata   4217
Pro Gln Val Leu  Gly Tyr Pro Ser Lys  Pro Ile Gly Leu Phe  Ile
            1360              1365              1370

aaa aga tct atc  atc ttc cga tct gat  tcc aat ggg gaa gat  ttg   4262
Lys Arg Ser Ile  Ile Phe Arg Ser Asp  Ser Asn Gly Glu Asp  Leu
            1375              1380              1385

gaa ggt tat gcc  ggt gct ggc ctc tac  gac agt gta cca atg  gat   4307
Glu Gly Tyr Ala  Gly Ala Gly Leu Tyr  Asp Ser Val Pro Met  Asp
            1390              1395              1400

gag gag gaa aaa  gtt gta att gat tac  tct tcc gac cca ttg  ata   4352
Glu Glu Glu Lys  Val Val Ile Asp Tyr  Ser Ser Asp Pro Leu  Ile
            1405              1410              1415

act gat ggt aac  ttc cgc cag aca atc  ctg tcc aac att gct  cgt   4397
Thr Asp Gly Asn  Phe Arg Gln Thr Ile  Leu Ser Asn Ile Ala  Arg
            1420              1425              1430

gct gga cat gct  atc gag gag cta tat  ggc tct cct caa gac  att   4442
Ala Gly His Ala  Ile Glu Glu Leu Tyr  Gly Ser Pro Gln Asp  Ile
            1435              1440              1445

gag ggt gta gtg  agg gat gga aag att  tat gtc gtt cag aca  aga   4487
```

```
Glu Gly Val Val   Arg Asp Gly Lys Ile   Tyr Val Val Gln Thr   Arg
              1450                  1455                  1460

cca cag atg tga ttatattctc gttgtatgtt gttcagagaa gaccacagat        4539
Pro Gln Met

gtgatcatat tctcattgta tcagatctgt gaccacttac ctgatacctc ccatgaagtt   4599

acctgtatga ttatacgtga tccaaagcca tcacatcatg ttcaccttca gctattggag   4659

gagaagtgag aagtaggaat tgcaatatga ggaataataa gaaaaacttt gtaaaagcta   4719

aattagctgg gtatgatata gggagaaatg tgtaaacatt gtactatata tagtatatac   4779

acacgcatta tgtattgcat tatgcactga ataatatcgc agcatcaaag aagaaatcct   4839

ttgggtggtt tc                                                      4851


<210> 2
<211> 1464
<212> PRT
<213> Solanum tuberosum

<400> 2

Met Ser Asn Ser Leu Gly Asn Asn Leu Leu Tyr Gln Gly Phe Leu Thr
1               5                   10                  15

Ser Thr Val Leu Glu His Lys Ser Arg Ile Ser Pro Pro Cys Val Gly
             20                  25                  30

Gly Asn Ser Leu Phe Gln Gln Gln Val Ile Ser Lys Ser Pro Leu Ser
             35          .        40                  45

Thr Glu Phe Arg Gly Asn Arg Leu Lys Val Gln Lys Lys Lys Ile Pro
         50                  55                  60

Met Glu Lys Lys Arg Ala Phe Ser Ser Ser Pro His Ala Val Leu Thr
65                   70                  75                  80

Thr Asp Thr Ser Ser Glu Leu Ala Glu Lys Phe Ser Leu Gly Gly Asn
                 85                  90                  95

Ile Glu Leu Gln Val Asp Val Arg Pro Pro Thr Ser Gly Asp Val Ser
            100                 105                 110

Phe Val Asp Phe Gln Val Thr Asn Gly Ser Asp Lys Leu Phe Leu His
            115                 120                 125

Trp Gly Ala Val Lys Phe Gly Lys Glu Thr Trp Ser Leu Pro Asn Asp
        130                 135                 140

Arg Pro Asp Gly Thr Lys Val Tyr Lys Asn Lys Ala Leu Arg Thr Pro
145                 150                 155                 160

Phe Val Lys Ser Gly Ser Asn Ser Ile Leu Arg Leu Glu Ile Arg Asp
```

165　　　　　　　　　　170　　　　　　　　　　175

Thr Ala Ile Glu Ala Ile Glu Phe Leu Ile Tyr Asp Glu Ala His Asp
　　　　　　180　　　　　　　　185　　　　　　　190

Lys Trp Ile Lys Asn Asn Gly Gly Asn Phe Arg Val Lys Leu Ser Arg
　　　　195　　　　　　　200　　　　　　　205

Lys Glu Ile Arg Gly Pro Asp Val Ser Val Pro Glu Glu Leu Val Gln
　　210　　　　　　　215　　　　　　　220

Ile Gln Ser Tyr Leu Arg Trp Glu Arg Lys Gly Lys Gln Asn Tyr Pro
225　　　　　　　230　　　　　　　235　　　　　　　240

Pro Glu Lys Glu Lys Glu Glu Tyr Glu Ala Ala Arg Thr Val Leu Gln
　　　　　　245　　　　　　　250　　　　　　　255

Glu Glu Ile Ala Arg Gly Ala Ser Ile Gln Asp Ile Arg Ala Arg Leu
　　　　260　　　　　　　265　　　　　　　270

Thr Lys Thr Asn Asp Lys Ser Gln Ser Lys Glu Glu Pro Leu His Val
　　　275　　　　　　　280　　　　　　　285

Thr Lys Ser Asp Ile Pro Asp Asp Leu Ala Gln Ala Gln Ala Tyr Ile
　　290　　　　　　　295　　　　　　　300

Arg Trp Glu Lys Ala Gly Lys Pro Asn Tyr Pro Pro Glu Lys Gln Ile
305　　　　　　　310　　　　　　　315　　　　　　　320

Glu Glu Leu Glu Glu Ala Arg Arg Glu Leu Gln Leu Glu Leu Glu Lys
　　　　　　325　　　　　　　330　　　　　　　335

Gly Ile Thr Leu Asp Glu Leu Arg Lys Thr Ile Thr Lys Gly Glu Ile
　　　　340　　　　　　　345　　　　　　　350

Lys Thr Lys Val Glu Lys His Leu Lys Arg Ser Ser Phe Ala Val Glu
　　　355　　　　　　　360　　　　　　　365

Arg Ile Gln Arg Lys Lys Arg Asp Phe Gly His Leu Ile Asn Lys Tyr
　　370　　　　　　　375　　　　　　　380

Thr Ser Ser Pro Ala Val Gln Val Gln Lys Val Leu Glu Glu Pro Pro
385　　　　　　　390　　　　　　　395　　　　　　　400

Ala Leu Ser Lys Ile Lys Leu Tyr Ala Lys Glu Lys Glu Glu Gln Ile
　　　　　　405　　　　　　　410　　　　　　　415

Asp Asp Pro Ile Leu Asn Lys Lys Ile Phe Lys Val Asp Asp Gly Glu
　　　　420　　　　　　　425　　　　　　　430

Leu Leu Val Leu Val Ala Lys Ser Ser Gly Lys Thr Lys Val His Leu

|||435||||||440|||||445|||

Ala Thr Asp Leu Asn Gln Pro Ile Thr Leu His Trp Ala Leu Ser Lys
450 455 460

Ser Pro Gly Glu Trp Met Val Pro Pro Ser Ser Ile Leu Pro Pro Gly
465 470 475 480

Ser Ile Ile Leu Asp Lys Ala Ala Glu Thr Pro Phe Ser Ala Ser Ser
485 490 495

Ser Asp Gly Leu Thr Ser Lys Val Gln Ser Leu Asp Ile Val Ile Glu
500 505 510

Asp Gly Asn Phe Val Gly Met Pro Phe Val Leu Leu Ser Gly Glu Lys
515 520 525

Trp Ile Lys Asn Gln Gly Ser Asp Phe Tyr Val Gly Phe Ser Ala Ala
530 535 540

Ser Lys Leu Ala Leu Lys Ala Ala Gly Asp Gly Ser Gly Thr Ala Lys
545 550 555 560

Ser Leu Leu Asp Lys Ile Ala Asp Met Glu Ser Glu Ala Gln Lys Ser
565 570 575

Phe Met His Arg Phe Asn Ile Ala Ala Asp Leu Ile Glu Asp Ala Thr
580 585 590

Ser Ala Gly Glu Leu Gly Phe Ala Gly Ile Leu Val Trp Met Arg Phe
595 600 605

Met Ala Thr Arg Gln Leu Ile Trp Asn Lys Asn Tyr Asn Val Lys Pro
610 615 620

Arg Glu Ile Ser Lys Ala Gln Asp Arg Leu Thr Asp Leu Leu Gln Asn
625 630 635 640

Ala Phe Thr Ser His Pro Gln Tyr Arg Glu Ile Leu Arg Met Ile Met
645 650 655

Ser Thr Val Gly Arg Gly Gly Glu Gly Asp Val Gly Gln Arg Ile Arg
660 665 670

Asp Glu Ile Leu Val Ile Gln Arg Asn Asn Asp Cys Lys Gly Gly Met
675 680 685

Met Gln Glu Trp His Gln Lys Leu His Asn Asn Thr Ser Pro Asp Asp
690 695 700

Val Val Ile Cys Gln Ala Leu Ile Asp Tyr Ile Lys Ser Asp Phe Asp

705     710     715     720

Leu Gly Val Tyr Trp Lys Thr Leu Asn Glu Asn Gly Ile Thr Lys Glu
725 730 735

Arg Leu Leu Ser Tyr Asp Arg Ala Ile His Ser Glu Pro Asn Phe Arg
740 745 750

Gly Asp Gln Lys Gly Gly Leu Leu Arg Asp Leu Gly His Tyr Met Arg
755 760 765

Thr Leu Lys Ala Val His Ser Gly Ala Asp Leu Glu Ser Ala Ile Ala
770 775 780

Asn Cys Met Gly Tyr Lys Thr Glu Gly Glu Gly Phe Met Val Gly Val
785 790 795 800

Gln Ile Asn Pro Val Ser Gly Leu Pro Ser Gly Phe Gln Asp Leu Leu
805 810 815

His Phe Val Leu Asp His Val Glu Asp Lys Asn Val Glu Thr Leu Leu
820 825 830

Glu Arg Leu Leu Glu Ala Arg Glu Glu Leu Arg Pro Leu Leu Leu Lys
835 840 845

Pro Asn Asn Arg Leu Lys Asp Leu Leu Phe Leu Asp Ile Ala Leu Asp
850 855 860

Ser Thr Val Arg Thr Ala Val Glu Arg Gly Tyr Glu Glu Leu Asn Asn
865 870 875 880

Ala Asn Pro Glu Lys Ile Met Tyr Phe Ile Ser Leu Val Leu Glu Asn
885 890 895

Leu Ala Leu Ser Val Asp Asp Asn Glu Asp Leu Val Tyr Cys Leu Lys
900 905 910

Gly Trp Asn Gln Ala Leu Ser Met Ser Asn Gly Gly Asp Asn His Trp
915 920 925

Ala Leu Phe Ala Lys Ala Val Leu Asp Arg Thr Arg Leu Ala Leu Ala
930 935 940

Ser Lys Ala Glu Trp Tyr His His Leu Leu Gln Pro Ser Ala Glu Tyr
945 950 955 960

Leu Gly Ser Ile Leu Gly Val Asp Gln Trp Ala Leu Asn Ile Phe Thr
965 970 975

Glu Glu Ile Ile Arg Ala Gly Ser Ala Ala Ser Leu Ser Ser Leu Leu

```
                980                        985                        990

        Asn Arg Leu Asp Pro Val Leu Arg  Lys Thr Ala Asn Leu  Gly Ser Trp
                995                       1000                  1005

        Gln Ile  Ile Ser Pro Val Glu  Ala Val Gly Tyr Val  Val Val Val
            1010                      1015                  1020

        Asp Glu  Leu Leu Ser Val Gln  Asn Glu Ile Tyr Glu  Lys Pro Thr
            1025                      1030                  1035

        Ile Leu  Val Ala Lys Ser Val  Lys Gly Glu Glu Glu  Ile Pro Asp
            1040                      1045                  1050

        Gly Ala  Val Ala Leu Ile Thr  Pro Asp Met Pro Asp  Val Leu Ser
            1055                      1060                  1065

        His Val  Ser Val Arg Ala Arg  Asn Gly Lys Val Cys  Phe Ala Thr
            1070                      1075                  1080

        Cys Phe  Asp Pro Asn Ile Leu  Ala Asp Leu Gln Ala  Lys Glu Gly
            1085                      1090                  1095

        Arg Ile  Leu Leu Leu Lys Pro  Thr Pro Ser Asp Ile  Ile Tyr Ser
            1100                      1105                  1110

        Glu Val  Asn Glu Ile Glu Leu  Gln Ser Ser Ser Asn  Leu Val Glu
            1115                      1120                  1125

        Ala Glu  Thr Ser Ala Thr Leu  Arg Leu Val Lys Lys  Gln Phe Gly
            1130                      1135                  1140

        Gly Cys  Tyr Ala Ile Ser Ala  Asp Glu Phe Thr Ser  Glu Met Val
            1145                      1150                  1155

        Gly Ala  Lys Ser Arg Asn Ile  Ala Tyr Leu Lys Gly  Lys Val Pro
            1160                      1165                  1170

        Ser Ser  Val Gly Ile Pro Thr  Ser Val Ala Leu Pro  Phe Gly Val
            1175                      1180                  1185

        Phe Glu  Lys Val Leu Ser Asp  Asp Ile Asn Gln Gly  Val Ala Lys
            1190                      1195                  1200

        Glu Leu  Gln Ile Leu Met Lys  Lys Leu Ser Glu Gly  Asp Phe Ser
            1205                      1210                  1215

        Ala Leu  Gly Glu Ile Arg Thr  Thr Val Leu Asp Leu  Ser Ala Pro
            1220                      1225                  1230

        Ala Gln  Leu Val Lys Glu Leu  Lys Glu Lys Met Gln  Gly Ser Gly
```

1235 1240 1245

Met Pro Trp Pro Gly Asp Glu Gly Pro Lys Arg Trp Glu Gln Ala
1250 1255 1260

Trp Met Ala Ile Lys Lys Val Trp Ala Ser Lys Trp Asn Glu Arg
1265 1270 1275

Ala Tyr Phe Ser Thr Arg Lys Val Lys Leu Asp His Asp Tyr Leu
1280 1285 1290

Cys Met Ala Val Leu Val Gln Glu Ile Ile Asn Ala Asp Tyr Ala
1295 1300 1305

Phe Val Ile His Thr Thr Asn Pro Ser Ser Gly Asp Asp Ser Glu
1310 1315 1320

Ile Tyr Ala Glu Val Val Arg Gly Leu Gly Glu Thr Leu Val Gly
1325 1330 1335

Ala Tyr Pro Gly Arg Ala Leu Ser Phe Ile Cys Lys Lys Lys Asp
1340 1345 1350

Leu Asn Ser Pro Gln Val Leu Gly Tyr Pro Ser Lys Pro Ile Gly
1355 1360 1365

Leu Phe Ile Lys Arg Ser Ile Ile Phe Arg Ser Asp Ser Asn Gly
1370 1375 1380

Glu Asp Leu Glu Gly Tyr Ala Gly Ala Gly Leu Tyr Asp Ser Val
1385 1390 1395

Pro Met Asp Glu Glu Glu Lys Val Val Ile Asp Tyr Ser Ser Asp
1400 1405 1410

Pro Leu Ile Thr Asp Gly Asn Phe Arg Gln Thr Ile Leu Ser Asn
1415 1420 1425

Ile Ala Arg Ala Gly His Ala Ile Glu Glu Leu Tyr Gly Ser Pro
1430 1435 1440

Gln Asp Ile Glu Gly Val Val Arg Asp Gly Lys Ile Tyr Val Val
1445 1450 1455

Gln Thr Arg Pro Gln Met
1460

<210> 3
<211> 4443
<212> DNA
<213> Curcuma longa

44

```
<220>
<221>  CDS
<222>  (1)..(4443)

<400>  3
atg aac aat tgt gtt gga cat acc tta cct cag caa gct ctg ttt cgg      48
Met Asn Asn Cys Val Gly His Thr Leu Pro Gln Gln Ala Leu Phe Arg
1               5                   10                  15

cct tct gtt gta gaa cgc cat aat aca gct tgc caa cgt tct tct gga      96
Pro Ser Val Val Glu Arg His Asn Thr Ala Cys Gln Arg Ser Ser Gly
                20                  25                  30

aac att ttg tgc act gtt cca tca gca tca aag gca gaa gat gtg cca     144
Asn Ile Leu Cys Thr Val Pro Ser Ala Ser Lys Ala Glu Asp Val Pro
            35                  40                  45

tct ctt aaa cct ttc ctt tca agt aga ttc ctg ggg aag act ccc tat     192
Ser Leu Lys Pro Phe Leu Ser Ser Arg Phe Leu Gly Lys Thr Pro Tyr
        50                  55                  60

gca gga aaa gga aac cca tta aag aaa aat tta aga aca gtt acc atg     240
Ala Gly Lys Gly Asn Pro Leu Lys Lys Asn Leu Arg Thr Val Thr Met
65                  70                  75                  80

agc cct caa gct tta ttg gca gca gat cct gct tca gag ctt gct aga     288
Ser Pro Gln Ala Leu Leu Ala Ala Asp Pro Ala Ser Glu Leu Ala Arg
                85                  90                  95

aaa ttc aag ctg gac acc aat tcc gaa ttg gag gtt act att tgt aag     336
Lys Phe Lys Leu Asp Thr Asn Ser Glu Leu Glu Val Thr Ile Cys Lys
            100                 105                 110

ccc aca tct gag tct cct atg caa att gat ttt caa gta acc aat gtc     384
Pro Thr Ser Glu Ser Pro Met Gln Ile Asp Phe Gln Val Thr Asn Val
        115                 120                 125

agt ggt tcc ttg gtg ctt cat tgg ggt gta att ctc caa aca aga aga     432
Ser Gly Ser Leu Val Leu His Trp Gly Val Ile Leu Gln Thr Arg Arg
        130                 135                 140

gaa tgg tct ctt cct tct cat tat cct gaa gga aca aaa gta tac aaa     480
Glu Trp Ser Leu Pro Ser His Tyr Pro Glu Gly Thr Lys Val Tyr Lys
145                 150                 155                 160

aat caa gct ctc aga act cct ttt act aaa gtt ggc tcg act tgt tca     528
Asn Gln Ala Leu Arg Thr Pro Phe Thr Lys Val Gly Ser Thr Cys Ser
                165                 170                 175

ctg aga tta gag att gat gat cct gaa ata gaa ata gtt gag ttt ctt     576
Leu Arg Leu Glu Ile Asp Asp Pro Glu Ile Glu Ile Val Glu Phe Leu
            180                 185                 190

ata ctg gat gag gca gaa aac aaa tgg tac aaa cat aat ggc cag aat     624
Ile Leu Asp Glu Ala Glu Asn Lys Trp Tyr Lys His Asn Gly Gln Asn
        195                 200                 205

ttt caa gtt cat ttg ttg aaa caa ggc tat caa aat caa cat gtt tca     672
Phe Gln Val His Leu Leu Lys Gln Gly Tyr Gln Asn Gln His Val Ser
        210                 215                 220

gtc tct gga aat cca aat atc att gta cct gaa gac ctt gtg cag att     720
Val Ser Gly Asn Pro Asn Ile Ile Val Pro Glu Asp Leu Val Gln Ile
225                 230                 235                 240

caa gcc ttt ctt agg tgg gaa aga aag ggt agg cag aca tat aca cct     768
Gln Ala Phe Leu Arg Trp Glu Arg Lys Gly Arg Gln Thr Tyr Thr Pro
```

EP 1 887 079 A1

|  | 245 | | | 250 | | | 255 | |
|---|---|---|---|---|---|---|---|---|

```
                245                    250                    255
      gat caa gaa aag gag gag tat gaa gca gct aga atg gag ctg ata gaa      816
      Asp Gln Glu Lys Glu Glu Tyr Glu Ala Ala Arg Met Glu Leu Ile Glu
              260                265                270

      gaa ata agt aga ggt atg cct gta gag gag ctt cga tcc aag ttg aca      864
      Glu Ile Ser Arg Gly Met Pro Val Glu Glu Leu Arg Ser Lys Leu Thr
              275                280                285

      gag aaa cca gaa gtc aaa tct gga agt aga gaa gag aaa acc cac aga      912
      Glu Lys Pro Glu Val Lys Ser Gly Ser Arg Glu Glu Lys Thr His Arg
              290                295                300

      gta caa agt cac aaa ggt ggg atc tca gat gat ctt gtg caa ata caa      960
      Val Gln Ser His Lys Gly Gly Ile Ser Asp Asp Leu Val Gln Ile Gln
      305                310                315                320

      gca ttc atc cga tgg gag aaa gct ggg aaa cca aac tac cct cca gag      1008
      Ala Phe Ile Arg Trp Glu Lys Ala Gly Lys Pro Asn Tyr Pro Pro Glu
                  325                330                335

      aag caa ctt atg gag ttt gag gaa gca agg aaa gag ctg cag ctt gag      1056
      Lys Gln Leu Met Glu Phe Glu Glu Ala Arg Lys Glu Leu Gln Leu Glu
              340                345                350

      ttt gat aaa ggt act tct ctg gct gaa cta cgg gaa aag atc atg aag      1104
      Phe Asp Lys Gly Thr Ser Leu Ala Glu Leu Arg Glu Lys Ile Met Lys
              355                360                365

      ggg gat ata tca act aaa gtt ttg aag caa ctg aag gtt gaa aag tat      1152
      Gly Asp Ile Ser Thr Lys Val Leu Lys Gln Leu Lys Val Glu Lys Tyr
      370                375                380

      ttc agc aac aaa aga att cag cgg aag gaa agg gac atc atg gaa att      1200
      Phe Ser Asn Lys Arg Ile Gln Arg Lys Glu Arg Asp Ile Met Glu Ile
      385                390                395                400

      ttg aat aaa aaa gtt gca gaa act cta gat gaa aaa tct tct caa ata      1248
      Leu Asn Lys Lys Val Ala Glu Thr Leu Asp Glu Lys Ser Ser Gln Ile
                  405                410                415

      gtc act cct cct aca gtg cta gaa ctc ttg gct aag tct ata cat gag      1296
      Val Thr Pro Pro Thr Val Leu Glu Leu Leu Ala Lys Ser Ile His Glu
                  420                425                430

      cag gat ggt gaa tca gtt ctg cat cag aaa atc tat aag ctg gat aat      1344
      Gln Asp Gly Glu Ser Val Leu His Gln Lys Ile Tyr Lys Leu Asp Asn
              435                440                445

      aag aat ctt ctg gta cta gta acc aaa cct ttt gaa agg aca aaa gtt      1392
      Lys Asn Leu Leu Val Leu Val Thr Lys Pro Phe Glu Arg Thr Lys Val
      450                455                460

      tat ttg gct aca gat caa agt gaa cca ctt att tta cac tgg gga tta      1440
      Tyr Leu Ala Thr Asp Gln Ser Glu Pro Leu Ile Leu His Trp Gly Leu
      465                470                475                480

      tca agg aaa tca aga gag tgg atg gta ccc cct aca agt tct att cct      1488
      Ser Arg Lys Ser Arg Glu Trp Met Val Pro Pro Thr Ser Ser Ile Pro
                  485                490                495

      cca ggt tca gta ttg cta gaa gag tct tgt gaa acc cct ttt act aag      1536
      Pro Gly Ser Val Leu Leu Glu Glu Ser Cys Glu Thr Pro Phe Thr Lys
                  500                505                510

      ggt tta atg gta gat cag tat tat cag gcc att caa ata gag att gat      1584
      Gly Leu Met Val Asp Gln Tyr Tyr Gln Ala Ile Gln Ile Glu Ile Asp
```

46

<pre>
                   515                        520                            525

       ggg ggt gat tat gct gga att ccc ttc gtt ctt cgt tca gac gat aaa        1632
       Gly Gly Asp Tyr Ala Gly Ile Pro Phe Val Leu Arg Ser Asp Asp Lys
               530                     535                    540

       tgg ata aag aat agt ggt ttg gac ttt tac att gag ttg gac gat aga        1680
       Trp Ile Lys Asn Ser Gly Leu Asp Phe Tyr Ile Glu Leu Asp Asp Arg
       545                     550                     555                560

       agt att agg aag gct cct ggt gat gga agc ggc att gca aaa tca ttg        1728
       Ser Ile Arg Lys Ala Pro Gly Asp Gly Ser Gly Ile Ala Lys Ser Leu
                       565                     570                    575

       ctt gac aag att gct gac ctg gag acc gag gct caa aaa tct ttt atg        1776
       Leu Asp Lys Ile Ala Asp Leu Glu Thr Glu Ala Gln Lys Ser Phe Met
                       580                     585                    590

       cac agg ttt agt att gca gca gat ctc act gag caa gct aga ggc tct        1824
       His Arg Phe Ser Ile Ala Ala Asp Leu Thr Glu Gln Ala Arg Gly Ser
                   595                     600                     605

       ggc cat cta ggg ctt gtt ggc att ctt gtt tgg atg aga ttc atg gca        1872
       Gly His Leu Gly Leu Val Gly Ile Leu Val Trp Met Arg Phe Met Ala
           610                     615                     620

       atg aga caa ctc att tgg aat aaa aac tac aat gtc aag cca cgt gag        1920
       Met Arg Gln Leu Ile Trp Asn Lys Asn Tyr Asn Val Lys Pro Arg Glu
       625                     630                     635                640

       att agt aaa gct cag gat agg ctc aca gat ctt ctt cag gac ata tat        1968
       Ile Ser Lys Ala Gln Asp Arg Leu Thr Asp Leu Leu Gln Asp Ile Tyr
                           645                     650                    655

       aaa gac ttc ccc cag tat aga gag atc ttg agg atg atc atg gct act        2016
       Lys Asp Phe Pro Gln Tyr Arg Glu Ile Leu Arg Met Ile Met Ala Thr
                       660                     665                    670

       gtt ggt agg ggc ggt gaa ggt gat gtt ggt cag cgt atc cga gat gaa        2064
       Val Gly Arg Gly Gly Glu Gly Asp Val Gly Gln Arg Ile Arg Asp Glu
                   675                     680                     685

       ata tta gtt ata cag aga aac aat gac tgc aag gga gga atg atg gag        2112
       Ile Leu Val Ile Gln Arg Asn Asn Asp Cys Lys Gly Gly Met Met Glu
               690                     695                     700

       gaa tgg cat cag aag cta cat aac aac act agc cca gat gat gtt gtg        2160
       Glu Trp His Gln Lys Leu His Asn Asn Thr Ser Pro Asp Asp Val Val
       705                     710                     715                720

       ata tgc cag gca ctt att gat tat gtt aaa agt gat ttt gac atc agt        2208
       Ile Cys Gln Ala Leu Ile Asp Tyr Val Lys Ser Asp Phe Asp Ile Ser
                       725                     730                    735

       gtg tac tgg gac agt ttg aat aaa aat gga ata acc aag gaa cgt ttg        2256
       Val Tyr Trp Asp Ser Leu Asn Lys Asn Gly Ile Thr Lys Glu Arg Leu
                       740                     745                    750

       ttg agc tat gat cgt gct att cat tct gaa cca agt ttc agg aga gat        2304
       Leu Ser Tyr Asp Arg Ala Ile His Ser Glu Pro Ser Phe Arg Arg Asp
                   755                     760                     765

       cag aaa gaa ggt ctt tta cgt gat cta gga aac tac atg agg acg ttg        2352
       Gln Lys Glu Gly Leu Leu Arg Asp Leu Gly Asn Tyr Met Arg Thr Leu
               770                     775                     780

       aag gca gtt cac tct ggt gca gat ctc gag tct gcc att gct acg tgt        2400
       Lys Ala Val His Ser Gly Ala Asp Leu Glu Ser Ala Ile Ala Thr Cys
</pre>

```
         785                    790                    795                    800

atg ggt tac aaa tct gag cgt caa ggc ttt atg gtt ggc gtt caa ata      2448
Met Gly Tyr Lys Ser Glu Arg Gln Gly Phe Met Val Gly Val Gln Ile
                    805                    810                    815

aac ccg ata ggg gga ttg cca tct gga ttc cct ggt cta atg aaa ttc      2496
Asn Pro Ile Gly Gly Leu Pro Ser Gly Phe Pro Gly Leu Met Lys Phe
                820                    825                    830

att cta aaa cat gtt gaa gat aaa aat gtg gag cct ttg ata gag ggg      2544
Ile Leu Lys His Val Glu Asp Lys Asn Val Glu Pro Leu Ile Glu Gly
            835                    840                    845

ttg ctg gag gca cga gtg gaa ctt aga cca ttg ctt ctt agc tct cat      2592
Leu Leu Glu Ala Arg Val Glu Leu Arg Pro Leu Leu Leu Ser Ser His
        850                    855                    860

gaa cgg ctg aag gat ctt att ttt ttg gat atc gcc ctt gat tct act      2640
Glu Arg Leu Lys Asp Leu Ile Phe Leu Asp Ile Ala Leu Asp Ser Thr
865                    870                    875                    880

gtc agg aca gct gtt gag aga gga tat gag gaa ttg agt aat gcg gag      2688
Val Arg Thr Ala Val Glu Arg Gly Tyr Glu Glu Leu Ser Asn Ala Glu
                    885                    890                    895

cca gag aaa ctt att tac ctt att atg ctg ctg ctt gag aat ctt gca      2736
Pro Glu Lys Leu Ile Tyr Leu Ile Met Leu Leu Leu Glu Asn Leu Ala
                900                    905                    910

ttg tct aca gat gat aat gag gac ctc ata tat tgc ttg aag gga tgg      2784
Leu Ser Thr Asp Asp Asn Glu Asp Leu Ile Tyr Cys Leu Lys Gly Trp
            915                    920                    925

aaa cat tcg atg gag atg tgt aag caa aaa gat gat caa tgg gca cta      2832
Lys His Ser Met Glu Met Cys Lys Gln Lys Asp Asp Gln Trp Ala Leu
        930                    935                    940

ttt gct aag tca ttt ctt gac aga acc cgt ctg gct cta tca agc aag      2880
Phe Ala Lys Ser Phe Leu Asp Arg Thr Arg Leu Ala Leu Ser Ser Lys
945                    950                    955                    960

gca gaa tac tac cat caa att ttg caa cct tca gct gaa tac ctt gga      2928
Ala Glu Tyr Tyr His Gln Ile Leu Gln Pro Ser Ala Glu Tyr Leu Gly
                    965                    970                    975

tca ttg ctt gat gtt gat gca ggg gcg gta agc ata ttc aca gaa gaa      2976
Ser Leu Leu Asp Val Asp Ala Gly Ala Val Ser Ile Phe Thr Glu Glu
                980                    985                    990

atc ata cgt gct gga tca gca gct  tct tta tct gca ctt  ctt cag cga   3024
Ile Ile Arg Ala Gly Ser Ala Ala  Ser Leu Ser Ala Leu  Leu Gln Arg
            995                    1000                   1005

ctt gac  cct ctt ctt cgg aaa  gtt gca cat ttg gga  agc tgg cag      3069
Leu Asp  Pro Leu Leu Arg Lys  Val Ala His Leu Gly  Ser Trp Gln
    1010                    1015                   1020

gtc ata  agc cct gtt gaa gtt  gct gga tat gtt gaa  att gta gaa      3114
Val Ile  Ser Pro Val Glu Val  Ala Gly Tyr Val Glu  Ile Val Glu
    1025                    1030                   1035

gaa ttg  ctt gct gtc cag aat  aaa tca tat aca caa  tca aca att      3159
Glu Leu  Leu Ala Val Gln Asn  Lys Ser Tyr Thr Gln  Ser Thr Ile
    1040                    1045                   1050

ttg gtt  gca aaa cat gta agg  gga gaa gag gaa ata  cca gat ggc      3204
Leu Val  Ala Lys His Val Arg  Gly Glu Glu Glu Ile  Pro Asp Gly
```

```
                  1055                    1060                    1065
        aca gtt  gct gtt tta aca cct  gat atg cca gat gtt  cta tct cat    3249
        Thr Val  Ala Val Leu Thr Pro  Asp Met Pro Asp Val  Leu Ser His
            1070                1075                1080

        gtc tct  gtg cga gct aga aat  agc aag gta tgt ttt  gct acc tgc    3294
        Val Ser  Val Arg Ala Arg Asn  Ser Lys Val Cys Phe  Ala Thr Cys
            1085                1090                1095

        ttt gat  gac aat atc ctg gat  gag ttt cgg aga aat  gca gga aag    3339
        Phe Asp  Asp Asn Ile Leu Asp  Glu Phe Arg Arg Asn  Ala Gly Lys
            1100                1105                1110

        ctt ttt  cat cta aag ccc aca  tca gat gat att gta  tat agt aaa    3384
        Leu Phe  His Leu Lys Pro Thr  Ser Asp Asp Ile Val  Tyr Ser Lys
            1115                1120                1125

        ata gaa  aaa act gaa cct gaa  gat gtg ggt cca gtt  caa gct gga    3429
        Ile Glu  Lys Thr Glu Pro Glu  Asp Val Gly Pro Val  Gln Ala Gly
            1130                1135                1140

        gat gag  caa tca ctg cca tct  gtg aca ttg gtt agg  aag cac ttc    3474
        Asp Glu  Gln Ser Leu Pro Ser  Val Thr Leu Val Arg  Lys His Phe
            1145                1150                1155

        agc ggc  aag tac acc ata tca  gct gaa gaa ttt acc  aat gaa atg    3519
        Ser Gly  Lys Tyr Thr Ile Ser  Ala Glu Glu Phe Thr  Asn Glu Met
            1160                1165                1170

        gtt ggt  gct aaa tca cgg aat  atc tca ttt cta aaa  gga aag gtt    3564
        Val Gly  Ala Lys Ser Arg Asn  Ile Ser Phe Leu Lys  Gly Lys Val
            1175                1180                1185

        cct tca  tgg gtg ggc att ccc  aca tca gtc gct cta  cca ttt gga    3609
        Pro Ser  Trp Val Gly Ile Pro  Thr Ser Val Ala Leu  Pro Phe Gly
            1190                1195                1200

        gtt ttt  gaa gaa gtt ctg tca  aat gac ata aac aag  gaa att gcc    3654
        Val Phe  Glu Glu Val Leu Ser  Asn Asp Ile Asn Lys  Glu Ile Ala
            1205                1210                1215

        agc cag  ctg cag tta ctg aaa  gag aag ttg gct atc  gga gaa ttc    3699
        Ser Gln  Leu Gln Leu Leu Lys  Glu Lys Leu Ala Ile  Gly Glu Phe
            1220                1225                1230

        aat gca  ctt ctc gac ata aga  aag atg atc ttg cag  cta gca tct    3744
        Asn Ala  Leu Leu Asp Ile Arg  Lys Met Ile Leu Gln  Leu Ala Ser
            1235                1240                1245

        cca att  gag ttg gta caa gag  cta aag gga aaa atg  cag gca tca    3789
        Pro Ile  Glu Leu Val Gln Glu  Leu Lys Gly Lys Met  Gln Ala Ser
            1250                1255                1260

        gga atg  cca tgg cct ggt gat  gag ggt gaa gat cgg  tgg gaa ctt    3834
        Gly Met  Pro Trp Pro Gly Asp  Glu Gly Glu Asp Arg  Trp Glu Leu
            1265                1270                1275

        gct tgg  atg gca ata aaa aga  gtt tgg gct tca aag  tgg aat gag    3879
        Ala Trp  Met Ala Ile Lys Arg  Val Trp Ala Ser Lys  Trp Asn Glu
            1280                1285                1290

        aga gca  tat ttc agc aca agg  aaa gtc aag ttg gat  cat gac tat    3924
        Arg Ala  Tyr Phe Ser Thr Arg  Lys Val Lys Leu Asp  His Asp Tyr
            1295                1300                1305

        ttg tgc  atg gct gtc ttg gtt  caa gaa atc att agt  gct gat tat    3969
        Leu Cys  Met Ala Val Leu Val  Gln Glu Ile Ile Ser  Ala Asp Tyr
```

```
          1310                      1315                      1320
     gca ttt gtc atc cac act aca aac cca tca tct gga gac tca tct      4014
     Ala Phe Val Ile His Thr Thr Asn Pro Ser Ser Gly Asp Ser Ser
         1325                      1330                      1335

     gaa ata tat gcc gag gtg gtg aaa gga ctc gga gaa act ctt gtt      4059
     Glu Ile Tyr Ala Glu Val Val Lys Gly Leu Gly Glu Thr Leu Val
         1340                      1345                      1350

     gga gcc tat cca ggc cgg gca ttg agc ttc gtc tgt aat aag aac      4104
     Gly Ala Tyr Pro Gly Arg Ala Leu Ser Phe Val Cys Asn Lys Asn
         1355                      1360                      1365

     aat ctg aac tcg cca aag gta ctt ggt ttc cca agc aag cct att      4149
     Asn Leu Asn Ser Pro Lys Val Leu Gly Phe Pro Ser Lys Pro Ile
         1370                      1375                      1380

     ggc ctc ttc atc aaa cga tca att atc ttc aga tct gat tct aat      4194
     Gly Leu Phe Ile Lys Arg Ser Ile Ile Phe Arg Ser Asp Ser Asn
         1385                      1390                      1395

     ggt gaa gat tta gaa ggt tat gca ggt gct ggt ctt tat gac agt      4239
     Gly Glu Asp Leu Glu Gly Tyr Ala Gly Ala Gly Leu Tyr Asp Ser
         1400                      1405                      1410

     gtg ccc atg gat gag gaa gag aaa gtg gta ctc gac tat gta gct      4284
     Val Pro Met Asp Glu Glu Glu Lys Val Val Leu Asp Tyr Val Ala
         1415                      1420                      1425

     gac ccg tta atc atg gat aag aac ttc cgt aat tca ctg ctc tcc      4329
     Asp Pro Leu Ile Met Asp Lys Asn Phe Arg Asn Ser Leu Leu Ser
         1430                      1435                      1440

     agc att gct cga gca ggt tat gcg atc gag gag ctc tat ggc tct      4374
     Ser Ile Ala Arg Ala Gly Tyr Ala Ile Glu Glu Leu Tyr Gly Ser
         1445                      1450                      1455

     cca cag gac att gaa ggt gtt gta aag gat ggt aaa atc ttc gtc      4419
     Pro Gln Asp Ile Glu Gly Val Val Lys Asp Gly Lys Ile Phe Val
         1460                      1465                      1470

     gtc caa aca aga cca cag atg tga                                  4443
     Val Gln Thr Arg Pro Gln Met
         1475                      1480
```

<210>   4
<211>   1480
<212>   PRT
<213>   Curcuma longa

<400>   4

```
Met Asn Asn Cys Val Gly His Thr Leu Pro Gln Gln Ala Leu Phe Arg
1               5                   10                  15

Pro Ser Val Val Glu Arg His Asn Thr Ala Cys Gln Arg Ser Ser Gly
            20                  25                  30

Asn Ile Leu Cys Thr Val Pro Ser Ala Ser Lys Ala Glu Asp Val Pro
        35                  40                  45

Ser Leu Lys Pro Phe Leu Ser Ser Arg Phe Leu Gly Lys Thr Pro Tyr
    50                  55                  60
```

```
Ala Gly Lys Gly Asn Pro Leu Lys Lys Asn Leu Arg Thr Val Thr Met
65              70              75                      80

Ser Pro Gln Ala Leu Leu Ala Ala Asp Pro Ala Ser Glu Leu Ala Arg
              85              90                  95

Lys Phe Lys Leu Asp Thr Asn Ser Glu Leu Glu Val Thr Ile Cys Lys
            100             105             110

Pro Thr Ser Glu Ser Pro Met Gln Ile Asp Phe Gln Val Thr Asn Val
        115             120             125

Ser Gly Ser Leu Val Leu His Trp Gly Val Ile Leu Gln Thr Arg Arg
    130             135             140

Glu Trp Ser Leu Pro Ser His Tyr Pro Glu Gly Thr Lys Val Tyr Lys
145             150             155             160

Asn Gln Ala Leu Arg Thr Pro Phe Thr Lys Val Gly Ser Thr Cys Ser
            165             170             175

Leu Arg Leu Glu Ile Asp Asp Pro Glu Ile Glu Ile Val Glu Phe Leu
        180             185             190

Ile Leu Asp Glu Ala Glu Asn Lys Trp Tyr Lys His Asn Gly Gln Asn
        195             200             205

Phe Gln Val His Leu Leu Lys Gln Gly Tyr Gln Asn Gln His Val Ser
    210             215             220

Val Ser Gly Asn Pro Asn Ile Ile Val Pro Glu Asp Leu Val Gln Ile
225             230             235             240

Gln Ala Phe Leu Arg Trp Glu Arg Lys Gly Arg Gln Thr Tyr Thr Pro
            245             250             255

Asp Gln Glu Lys Glu Glu Tyr Glu Ala Ala Arg Met Glu Leu Ile Glu
            260             265             270

Glu Ile Ser Arg Gly Met Pro Val Glu Glu Leu Arg Ser Lys Leu Thr
            275             280             285

Glu Lys Pro Glu Val Lys Ser Gly Ser Arg Glu Glu Lys Thr His Arg
    290             295             300

Val Gln Ser His Lys Gly Gly Ile Ser Asp Asp Leu Val Gln Ile Gln
305             310             315             320

Ala Phe Ile Arg Trp Glu Lys Ala Gly Lys Pro Asn Tyr Pro Pro Glu
            325             330             335
```

Lys Gln Leu Met Glu Phe Glu Glu Ala Arg Lys Glu Leu Gln Leu Glu
340 345 350

Phe Asp Lys Gly Thr Ser Leu Ala Glu Leu Arg Glu Lys Ile Met Lys
355 360 365

Gly Asp Ile Ser Thr Lys Val Leu Lys Gln Leu Lys Val Glu Lys Tyr
370 375 380

Phe Ser Asn Lys Arg Ile Gln Arg Lys Glu Arg Asp Ile Met Glu Ile
385 390 395 400

Leu Asn Lys Lys Val Ala Glu Thr Leu Asp Glu Lys Ser Ser Gln Ile
405 410 415

Val Thr Pro Pro Thr Val Leu Glu Leu Leu Ala Lys Ser Ile His Glu
420 425 430

Gln Asp Gly Glu Ser Val Leu His Gln Lys Ile Tyr Lys Leu Asp Asn
435 440 445

Lys Asn Leu Leu Val Leu Val Thr Lys Pro Phe Glu Arg Thr Lys Val
450 455 460

Tyr Leu Ala Thr Asp Gln Ser Glu Pro Leu Ile Leu His Trp Gly Leu
465 470 475 480

Ser Arg Lys Ser Arg Glu Trp Met Val Pro Pro Thr Ser Ser Ile Pro
485 490 495

Pro Gly Ser Val Leu Leu Glu Glu Ser Cys Glu Thr Pro Phe Thr Lys
500 505 510

Gly Leu Met Val Asp Gln Tyr Tyr Gln Ala Ile Gln Ile Glu Ile Asp
515 520 525

Gly Gly Asp Tyr Ala Gly Ile Pro Phe Val Leu Arg Ser Asp Asp Lys
530 535 540

Trp Ile Lys Asn Ser Gly Leu Asp Phe Tyr Ile Glu Leu Asp Asp Arg
545 550 555 560

Ser Ile Arg Lys Ala Pro Gly Asp Gly Ser Gly Ile Ala Lys Ser Leu
565 570 575

Leu Asp Lys Ile Ala Asp Leu Glu Thr Glu Ala Gln Lys Ser Phe Met
580 585 590

His Arg Phe Ser Ile Ala Ala Asp Leu Thr Glu Gln Ala Arg Gly Ser
595 600 605

```
Gly His Leu Gly Leu Val Gly Ile Leu Val Trp Met Arg Phe Met Ala
    610             615             620

Met Arg Gln Leu Ile Trp Asn Lys Asn Tyr Asn Val Lys Pro Arg Glu
625             630             635             640

Ile Ser Lys Ala Gln Asp Arg Leu Thr Asp Leu Leu Gln Asp Ile Tyr
            645             650             655

Lys Asp Phe Pro Gln Tyr Arg Glu Ile Leu Arg Met Ile Met Ala Thr
        660             665             670

Val Gly Arg Gly Gly Glu Gly Asp Val Gly Gln Arg Ile Arg Asp Glu
        675             680             685

Ile Leu Val Ile Gln Arg Asn Asn Asp Cys Lys Gly Gly Met Met Glu
    690             695             700

Glu Trp His Gln Lys Leu His Asn Asn Thr Ser Pro Asp Asp Val Val
705             710             715             720

Ile Cys Gln Ala Leu Ile Asp Tyr Val Lys Ser Asp Phe Asp Ile Ser
            725             730             735

Val Tyr Trp Asp Ser Leu Asn Lys Asn Gly Ile Thr Lys Glu Arg Leu
            740             745             750

Leu Ser Tyr Asp Arg Ala Ile His Ser Glu Pro Ser Phe Arg Arg Asp
        755             760             765

Gln Lys Glu Gly Leu Leu Arg Asp Leu Gly Asn Tyr Met Arg Thr Leu
    770             775             780

Lys Ala Val His Ser Gly Ala Asp Leu Glu Ser Ala Ile Ala Thr Cys
785             790             795             800

Met Gly Tyr Lys Ser Glu Arg Gln Gly Phe Met Val Gly Val Gln Ile
            805             810             815

Asn Pro Ile Gly Gly Leu Pro Ser Gly Phe Pro Gly Leu Met Lys Phe
            820             825             830

Ile Leu Lys His Val Glu Asp Lys Asn Val Glu Pro Leu Ile Glu Gly
        835             840             845

Leu Leu Glu Ala Arg Val Glu Leu Arg Pro Leu Leu Leu Ser Ser His
    850             855             860

Glu Arg Leu Lys Asp Leu Ile Phe Leu Asp Ile Ala Leu Asp Ser Thr
865             870             875             880
```

```
Val Arg Thr Ala Val Glu Arg Gly Tyr Glu Glu Leu Ser Asn Ala Glu
                885                 890                 895

Pro Glu Lys Leu Ile Tyr Leu Ile Met Leu Leu Leu Glu Asn Leu Ala
            900                 905                 910

Leu Ser Thr Asp Asp Asn Glu Asp Leu Ile Tyr Cys Leu Lys Gly Trp
        915                 920                 925

Lys His Ser Met Glu Met Cys Lys Gln Lys Asp Asp Gln Trp Ala Leu
    930                 935                 940

Phe Ala Lys Ser Phe Leu Asp Arg Thr Arg Leu Ala Leu Ser Ser Lys
945                 950                 955                 960

Ala Glu Tyr Tyr His Gln Ile Leu Gln Pro Ser Ala Glu Tyr Leu Gly
                965                 970                 975

Ser Leu Leu Asp Val Asp Ala Gly Ala Val Ser Ile Phe Thr Glu Glu
            980                 985                 990

Ile Ile Arg Ala Gly Ser Ala Ala Ser Leu Ser Ala Leu Leu Gln Arg
            995                 1000                1005

Leu Asp Pro Leu Leu Arg Lys Val Ala His Leu Gly Ser Trp Gln
    1010                1015                1020

Val Ile Ser Pro Val Glu Val Ala Gly Tyr Val Glu Ile Val Glu
    1025                1030                1035

Glu Leu Leu Ala Val Gln Asn Lys Ser Tyr Thr Gln Ser Thr Ile
    1040                1045                1050

Leu Val Ala Lys His Val Arg Gly Glu Glu Glu Ile Pro Asp Gly
    1055                1060                1065

Thr Val Ala Val Leu Thr Pro Asp Met Pro Asp Val Leu Ser His
    1070                1075                1080

Val Ser Val Arg Ala Arg Asn Ser Lys Val Cys Phe Ala Thr Cys
    1085                1090                1095

Phe Asp Asp Asn Ile Leu Asp Glu Phe Arg Arg Asn Ala Gly Lys
    1100                1105                1110

Leu Phe His Leu Lys Pro Thr Ser Asp Asp Ile Val Tyr Ser Lys
    1115                1120                1125

Ile Glu Lys Thr Glu Pro Glu Asp Val Gly Pro Val Gln Ala Gly
    1130                1135                1140
```

```
Asp Glu Gln Ser Leu Pro Ser Val Thr Leu Val Arg Lys His Phe
    1145                1150               1155

Ser Gly Lys Tyr Thr Ile Ser Ala Glu Glu Phe Thr Asn Glu Met
    1160                1165               1170

Val Gly Ala Lys Ser Arg Asn Ile Ser Phe Leu Lys Gly Lys Val
    1175                1180               1185

Pro Ser Trp Val Gly Ile Pro Thr Ser Val Ala Leu Pro Phe Gly
    1190                1195               1200

Val Phe Glu Glu Val Leu Ser Asn Asp Ile Asn Lys Glu Ile Ala
    1205                1210               1215

Ser Gln Leu Gln Leu Leu Lys Glu Lys Leu Ala Ile Gly Glu Phe
    1220                1225               1230

Asn Ala Leu Leu Asp Ile Arg Lys Met Ile Leu Gln Leu Ala Ser
    1235                1240               1245

Pro Ile Glu Leu Val Gln Glu Leu Lys Gly Lys Met Gln Ala Ser
    1250                1255               1260

Gly Met Pro Trp Pro Gly Asp Glu Gly Glu Asp Arg Trp Glu Leu
    1265                1270               1275

Ala Trp Met Ala Ile Lys Arg Val Trp Ala Ser Lys Trp Asn Glu
    1280                1285               1290

Arg Ala Tyr Phe Ser Thr Arg Lys Val Lys Leu Asp His Asp Tyr
    1295                1300               1305

Leu Cys Met Ala Val Leu Val Gln Glu Ile Ile Ser Ala Asp Tyr
    1310                1315               1320

Ala Phe Val Ile His Thr Thr Asn Pro Ser Ser Gly Asp Ser Ser
    1325                1330               1335

Glu Ile Tyr Ala Glu Val Val Lys Gly Leu Gly Glu Thr Leu Val
    1340                1345               1350

Gly Ala Tyr Pro Gly Arg Ala Leu Ser Phe Val Cys Asn Lys Asn
    1355                1360               1365

Asn Leu Asn Ser Pro Lys Val Leu Gly Phe Pro Ser Lys Pro Ile
    1370                1375               1380

Gly Leu Phe Ile Lys Arg Ser Ile Ile Phe Arg Ser Asp Ser Asn
    1385                1390               1395
```

```
Gly Glu  Asp Leu Glu Gly Tyr  Ala Gly Ala Gly Leu  Tyr Asp Ser
    1400             1405             1410


Val Pro  Met Asp Glu Glu Glu  Lys Val Val Leu Asp  Tyr Val Ala
    1415             1420             1425


Asp Pro  Leu Ile Met Asp Lys  Asn Phe Arg Asn Ser  Leu Leu Ser
    1430             1435             1440


Ser Ile  Ala Arg Ala Gly Tyr  Ala Ile Glu Glu Leu  Tyr Gly Ser
    1445             1450             1455


Pro Gln  Asp Ile Glu Gly Val  Val Lys Asp Gly Lys  Ile Phe Val
    1460             1465             1470


Val Gln  Thr Arg Pro Gln Met
    1475             1480


<210>   5
<211>   3000
<212>   DNA
<213>   Triticum aestivum


<220>
<221>   CDS
<222>   (227)..(2623)

<400>   5
ctccaccgcg gtggcggccg ctctagaact agtggatccc ccgggctgca ggaattcggc      60

acgagcttcg gcctgacccc gttcgtttac ccccacacag agcacactcc agtccagtcc     120

agcccactgc caccgcgcta ctctccactc ccactgccac cacctccgcc tgcgccgcgc     180

tctgggcgga ccaacccgcg aaccgtacca tctcccgccc cgatcc atg tcg tcg       235
                                                   Met Ser Ser
                                                     1

gcg gtc gcg tcc gcc gca tcc ttc ctc gcg ctc gcg tca gcc tcc ccc      283
Ala Val Ala Ser Ala Ala Ser Phe Leu Ala Leu Ala Ser Ala Ser Pro
      5               10                  15

ggg aga tca cgc agg cgg gcg agg gtg agc gcg cag cca ccc cac gcc      331
Gly Arg Ser Arg Arg Arg Ala Arg Val Ser Ala Gln Pro Pro His Ala
20              25                  30                  35

ggg gcc ggc agg ttg cac tgg ccg ccg tgg ccg ccg cag cgc acg gct      379
Gly Ala Gly Arg Leu His Trp Pro Pro Trp Pro Pro Gln Arg Thr Ala
                40                  45                  50

cgc gac gga gct gtg gcg gcg ctc gcc gcc ggg aag aag gac gcg ggg      427
Arg Asp Gly Ala Val Ala Ala Leu Ala Ala Gly Lys Lys Asp Ala Gly
                55                  60                  65

atc gac gac gcc gcc gcg tcc gtg agg cag ccc cgc gca ctc cgc ggt      475
Ile Asp Asp Ala Ala Ala Ser Val Arg Gln Pro Arg Ala Leu Arg Gly
            70                  75                  80

ggc gcc gcc acc aag gtc gcg gag cga agg gat ccc gtc aag acg ctc      523
```

56

```
Gly Ala Ala Thr Lys Val Ala Glu Arg Arg Asp Pro Val Lys Thr Leu
    85                  90                  95

gac cgc gac gcc gcg gaa ggc ggc ggg ccg tcc ccg ccg gca gcg agg    571
Asp Arg Asp Ala Ala Glu Gly Gly Gly Pro Ser Pro Pro Ala Ala Arg
100                 105                 110                 115

cag gac gcc gcc cgt ccg ccg agt atg aac ggc atg ccg gtg aac ggc    619
Gln Asp Ala Ala Arg Pro Pro Ser Met Asn Gly Met Pro Val Asn Gly
                120                 125                 130

gag aac aaa tct acc ggc ggc ggc ggc gcg act aaa gac agc ggg ctg    667
Glu Asn Lys Ser Thr Gly Gly Gly Gly Ala Thr Lys Asp Ser Gly Leu
            135                 140                 145

ccc acg ccc gca cgc gcg ccc cat ccg tcg acc cag aac aga gca ccg    715
Pro Thr Pro Ala Arg Ala Pro His Pro Ser Thr Gln Asn Arg Ala Pro
        150                 155                 160

gtg aac ggt gaa aac aaa gct aac gtc gcc tcg ccg ccg acg agc ata    763
Val Asn Gly Glu Asn Lys Ala Asn Val Ala Ser Pro Pro Thr Ser Ile
    165                 170                 175

gcc gag gcc gcg gct tcg gat tcc gca gct acc att tcc atc agc gac    811
Ala Glu Ala Ala Ala Ser Asp Ser Ala Ala Thr Ile Ser Ile Ser Asp
180                 185                 190                 195

aag gcg ccg gag tcc gtt gtc cca gct gag aag acg ccg ccg tcg tcc    859
Lys Ala Pro Glu Ser Val Val Pro Ala Glu Lys Thr Pro Pro Ser Ser
                200                 205                 210

ggc tca aat ttc gag tcc tcg gcc tct gct ccc ggg tct gac act gtc    907
Gly Ser Asn Phe Glu Ser Ser Ala Ser Ala Pro Gly Ser Asp Thr Val
            215                 220                 225

agc gac gtg gaa caa gaa ctg aag aag ggt gcg gtc gtt gtc gaa gaa    955
Ser Asp Val Glu Gln Glu Leu Lys Lys Gly Ala Val Val Val Glu Glu
        230                 235                 240

gct cca aag cca aag gct ctt tcg ccg cct gca gcc ccc gct gta caa   1003
Ala Pro Lys Pro Lys Ala Leu Ser Pro Pro Ala Ala Pro Ala Val Gln
    245                 250                 255

gaa gac ctt tgg gat ttc aag aaa tac att ggt ttc gag gag ccc gtg   1051
Glu Asp Leu Trp Asp Phe Lys Lys Tyr Ile Gly Phe Glu Glu Pro Val
260                 265                 270                 275

gag gcc aag gat gat ggc cgg gct gtc gca gat gat gcg ggc tcc ttt   1099
Glu Ala Lys Asp Asp Gly Arg Ala Val Ala Asp Asp Ala Gly Ser Phe
                280                 285                 290

gaa cac cac cag aat cac gac tcc gga cct ttg gca ggg gag aat gtc   1147
Glu His His Gln Asn His Asp Ser Gly Pro Leu Ala Gly Glu Asn Val
            295                 300                 305

atg aac gtg gtc gtc gtg gct gct gag tgt tct ccc tgg tgc aaa aca   1195
Met Asn Val Val Val Val Ala Ala Glu Cys Ser Pro Trp Cys Lys Thr
        310                 315                 320

ggt ggt ctg gga gat gtt gcg ggt gct ctg ccc aag gct ttg gca aag   1243
Gly Gly Leu Gly Asp Val Ala Gly Ala Leu Pro Lys Ala Leu Ala Lys
    325                 330                 335

aga gga cat cgt gtt atg gtt gtg gta cca agg tat ggg gac tat gaa   1291
Arg Gly His Arg Val Met Val Val Val Pro Arg Tyr Gly Asp Tyr Glu
340                 345                 350                 355

gaa gcc tac gat gtc gga gtc cga aaa tac tac aag gct gct gga cag   1339
```

EP 1 887 079 A1

```
Glu Ala Tyr Asp Val Gly Val Arg Lys Tyr Tyr Lys Ala Ala Gly Gln
            360             365         370

gat atg gaa gtg aat tat ttc cat gct tat atc gat gga gtt gat ttt   1387
Asp Met Glu Val Asn Tyr Phe His Ala Tyr Ile Asp Gly Val Asp Phe
            375             380         385

gtg ttc att gac gct cct ctc ttc cga cac cgt cag gaa gac att tat   1435
Val Phe Ile Asp Ala Pro Leu Phe Arg His Arg Gln Glu Asp Ile Tyr
            390             395         400

ggg ggc agc aga cag gaa att atg aag cgc atg att ttg ttc tgc aag   1483
Gly Gly Ser Arg Gln Glu Ile Met Lys Arg Met Ile Leu Phe Cys Lys
            405             410         415

gcc gct gtt gag gtt cca tgg cac gtt cca tgc ggc ggt gtc cct tat   1531
Ala Ala Val Glu Val Pro Trp His Val Pro Cys Gly Gly Val Pro Tyr
420             425             430         435

ggg gat gga aat ctg gtg ttt att gca aat gat tgg cac acg gca ctc   1579
Gly Asp Gly Asn Leu Val Phe Ile Ala Asn Asp Trp His Thr Ala Leu
            440             445         450

ctg cct gtc tat ctg aaa gca tat tac agg gac cat ggt ttg atg cag   1627
Leu Pro Val Tyr Leu Lys Ala Tyr Tyr Arg Asp His Gly Leu Met Gln
            455             460         465

tac act cgg tcc att atg gtg ata cat aac atc gct cac cag ggc cgt   1675
Tyr Thr Arg Ser Ile Met Val Ile His Asn Ile Ala His Gln Gly Arg
            470             475         480

ggc cct gta gat gaa ttc ccg ttc acc gag ttg cct gag cac tac ctg   1723
Gly Pro Val Asp Glu Phe Pro Phe Thr Glu Leu Pro Glu His Tyr Leu
            485             490         495

gaa cac ttc aga ctg tac gac ccc gtg ggt ggt gaa cac gcc aac tac   1771
Glu His Phe Arg Leu Tyr Asp Pro Val Gly Gly Glu His Ala Asn Tyr
500             505             510         515

ttc gcc gcc ggc ctg aag atg gcg gac cag gtt gtc gtg gtg agc ccc   1819
Phe Ala Ala Gly Leu Lys Met Ala Asp Gln Val Val Val Val Ser Pro
            520             525         530

ggg tac ctg tgg gag ctg aag acg gtg gag ggc ggc tgg ggg ctt cac   1867
Gly Tyr Leu Trp Glu Leu Lys Thr Val Glu Gly Gly Trp Gly Leu His
            535             540         545

gac atc ata cgg cag aac gac tgg aag acc cgc ggc atc gtc aac ggc   1915
Asp Ile Ile Arg Gln Asn Asp Trp Lys Thr Arg Gly Ile Val Asn Gly
            550             555         560

atc gac aac atg gag tgg aac ccc gag gtg gac gcc cac ctc aag tcg   1963
Ile Asp Asn Met Glu Trp Asn Pro Glu Val Asp Ala His Leu Lys Ser
            565             570         575

gac ggc tac acc aac ttc tcc ctg agg acg ctg gac tcc ggc aag cgg   2011
Asp Gly Tyr Thr Asn Phe Ser Leu Arg Thr Leu Asp Ser Gly Lys Arg
580             585             590         595

cag tgc aag gag gcc ctg cag cgc gag ctg ggc ctg cag gtc cgc gcc   2059
Gln Cys Lys Glu Ala Leu Gln Arg Glu Leu Gly Leu Gln Val Arg Ala
            600             605         610

gac gtg ccg ctg ctc ggc ttc atc ggc cgc ctg gac ggg cag aag ggc   2107
Asp Val Pro Leu Leu Gly Phe Ile Gly Arg Leu Asp Gly Gln Lys Gly
            615             620         625

gtg gag atc atc gcg gac gcc atg ccc tgg atc gtg agc cag gac gtg   2155
```

58

```
Val Glu Ile Ile Ala Asp Ala Met Pro Trp Ile Val Ser Gln Asp Val
        630                 635             640

cag ctg gtg atg ctg ggc acc ggg cgc cac gac ctg gag agc atg ctg   2203
Gln Leu Val Met Leu Gly Thr Gly Arg His Asp Leu Glu Ser Met Leu
        645                 650             655

cag cac ttc gag cgg gag cac cac gac aag gtg cgc ggg tgg gtg ggg   2251
Gln His Phe Glu Arg Glu His His Asp Lys Val Arg Gly Trp Val Gly
660                 665             670                 675

ttc tcc gtg cgc ctg gcg cac cgg atc acg gcg ggg gcg gac gcg ctc   2299
Phe Ser Val Arg Leu Ala His Arg Ile Thr Ala Gly Ala Asp Ala Leu
                680             685                 690

ctc atg ccc tcc cgg ttc gag ccg tgc ggg ctg aac cag ctc tac gcc   2347
Leu Met Pro Ser Arg Phe Glu Pro Cys Gly Leu Asn Gln Leu Tyr Ala
                695             700                 705

atg gcc tac ggc acc gtc ccc gtc gtg cac gcc gtc ggc ggc ctc agg   2395
Met Ala Tyr Gly Thr Val Pro Val Val His Ala Val Gly Gly Leu Arg
            710             715                 720

gac acc gtg ccg ccg ttc gac ccc ttc aac cac tcc ggg ctc ggg tgg   2443
Asp Thr Val Pro Pro Phe Asp Pro Phe Asn His Ser Gly Leu Gly Trp
        725                 730             735

acg ttc gac cgc gcc gag gcg cac aag ctg atc gag gcg ctc ggg cac   2491
Thr Phe Asp Arg Ala Glu Ala His Lys Leu Ile Glu Ala Leu Gly His
740                 745             750                 755

tgc ctc cgc acc tac cga gac ttc aag gag agc tgg agg gcc ctc cag   2539
Cys Leu Arg Thr Tyr Arg Asp Phe Lys Glu Ser Trp Arg Ala Leu Gln
                760             765                 770

gag cgc ggc atg tcg cag gac ttc agc tgg gag cac gcc gcc aag ctc   2587
Glu Arg Gly Met Ser Gln Asp Phe Ser Trp Glu His Ala Ala Lys Leu
                775             780                 785

tac gag gac gtc ctc gtc aag gcc aag tac cag tgg tgaacgctag        2633
Tyr Glu Asp Val Leu Val Lys Ala Lys Tyr Gln Trp
                790             795

ctgctagccg ctccagcccc gcatgcgtgc atgacaggat ggaactgcat tgcgcacgca   2693

ggaaagtgcc atggagcgcc ggcatccgcg aagtacagtg acatgaggtg tgtgtggttg   2753

agacgctgat tccaatccgg cccgtagcag agtagagcgg aggtatatgg gaatcttaac   2813

ttggtattgt aatttgttat gttgtgtgca ttattacaat gttgttactt attcttgtta   2873

agtcggaggc caagggcgaa agctagctca catgtctgat ggatgcacgt gccatggttg   2933

gtttggtagc gcagtgcaaa cggcaagaat gggaagtgaa ttcctccctg cttgaaaaaa   2993

aaaaaaa                                                            3000


<210>   6
<211>   799
<212>   PRT
<213>   Triticum aestivum

<400>   6

Met Ser Ser Ala Val Ala Ser Ala Ala Ser Phe Leu Ala Leu Ala Ser
1               5                   10                  15
```

59

```
Ala Ser Pro Gly Arg Ser Arg Arg Arg Ala Arg Val Ser Ala Gln Pro
             20              25              30

Pro His Ala Gly Ala Gly Arg Leu His Trp Pro Pro Trp Pro Pro Gln
         35              40              45

Arg Thr Ala Arg Asp Gly Ala Val Ala Ala Leu Ala Ala Gly Lys Lys
     50              55              60

Asp Ala Gly Ile Asp Asp Ala Ala Ala Ser Val Arg Gln Pro Arg Ala
65              70              75              80

Leu Arg Gly Gly Ala Ala Thr Lys Val Ala Glu Arg Arg Asp Pro Val
             85              90              95

Lys Thr Leu Asp Arg Asp Ala Ala Glu Gly Gly Gly Pro Ser Pro Pro
         100             105             110

Ala Ala Arg Gln Asp Ala Ala Arg Pro Pro Ser Met Asn Gly Met Pro
         115             120             125

Val Asn Gly Glu Asn Lys Ser Thr Gly Gly Gly Ala Thr Lys Asp
     130             135             140

Ser Gly Leu Pro Thr Pro Ala Arg Ala Pro His Pro Ser Thr Gln Asn
145             150             155             160

Arg Ala Pro Val Asn Gly Glu Asn Lys Ala Asn Val Ala Ser Pro Pro
             165             170             175

Thr Ser Ile Ala Glu Ala Ala Ala Ser Asp Ser Ala Ala Thr Ile Ser
             180             185             190

Ile Ser Asp Lys Ala Pro Glu Ser Val Val Pro Ala Glu Lys Thr Pro
         195             200             205

Pro Ser Ser Gly Ser Asn Phe Glu Ser Ser Ala Ser Ala Pro Gly Ser
     210             215             220

Asp Thr Val Ser Asp Val Glu Gln Glu Leu Lys Lys Gly Ala Val Val
225             230             235             240

Val Glu Glu Ala Pro Lys Pro Lys Ala Leu Ser Pro Pro Ala Ala Pro
             245             250             255

Ala Val Gln Glu Asp Leu Trp Asp Phe Lys Lys Tyr Ile Gly Phe Glu
         260             265             270

Glu Pro Val Glu Ala Lys Asp Asp Gly Arg Ala Val Ala Asp Asp Ala
         275             280             285
```

```
Gly Ser Phe Glu His His Gln Asn His Asp Ser Gly Pro Leu Ala Gly
    290             295             300

Glu Asn Val Met Asn Val Val Val Ala Ala Glu Cys Ser Pro Trp
305             310             315             320

Cys Lys Thr Gly Gly Leu Gly Asp Val Ala Gly Ala Leu Pro Lys Ala
            325             330             335

Leu Ala Lys Arg Gly His Arg Val Met Val Val Val Pro Arg Tyr Gly
            340             345             350

Asp Tyr Glu Glu Ala Tyr Asp Val Gly Val Arg Lys Tyr Tyr Lys Ala
            355             360             365

Ala Gly Gln Asp Met Glu Val Asn Tyr Phe His Ala Tyr Ile Asp Gly
    370             375             380

Val Asp Phe Val Phe Ile Asp Ala Pro Leu Phe Arg His Arg Gln Glu
385             390             395             400

Asp Ile Tyr Gly Gly Ser Arg Gln Glu Ile Met Lys Arg Met Ile Leu
            405             410             415

Phe Cys Lys Ala Ala Val Glu Val Pro Trp His Val Pro Cys Gly Gly
            420             425             430

Val Pro Tyr Gly Asp Gly Asn Leu Val Phe Ile Ala Asn Asp Trp His
            435             440             445

Thr Ala Leu Leu Pro Val Tyr Leu Lys Ala Tyr Tyr Arg Asp His Gly
    450             455             460

Leu Met Gln Tyr Thr Arg Ser Ile Met Val Ile His Asn Ile Ala His
465             470             475             480

Gln Gly Arg Gly Pro Val Asp Glu Phe Pro Phe Thr Glu Leu Pro Glu
            485             490             495

His Tyr Leu Glu His Phe Arg Leu Tyr Asp Pro Val Gly Gly Glu His
            500             505             510

Ala Asn Tyr Phe Ala Ala Gly Leu Lys Met Ala Asp Gln Val Val Val
            515             520             525

Val Ser Pro Gly Tyr Leu Trp Glu Leu Lys Thr Val Glu Gly Gly Trp
    530             535             540

Gly Leu His Asp Ile Ile Arg Gln Asn Asp Trp Lys Thr Arg Gly Ile
545             550             555             560
```

```
Val Asn Gly Ile Asp Asn Met Glu Trp Asn Pro Glu Val Asp Ala His
            565             570             575

Leu Lys Ser Asp Gly Tyr Thr Asn Phe Ser Leu Arg Thr Leu Asp Ser
            580             585             590

Gly Lys Arg Gln Cys Lys Glu Ala Leu Gln Arg Glu Leu Gly Leu Gln
            595             600             605

Val Arg Ala Asp Val Pro Leu Leu Gly Phe Ile Gly Arg Leu Asp Gly
            610             615             620

Gln Lys Gly Val Glu Ile Ile Ala Asp Ala Met Pro Trp Ile Val Ser
    625             630             635             640

Gln Asp Val Gln Leu Val Met Leu Gly Thr Gly Arg His Asp Leu Glu
            645             650             655

Ser Met Leu Gln His Phe Glu Arg Glu His His Asp Lys Val Arg Gly
            660             665             670

Trp Val Gly Phe Ser Val Arg Leu Ala His Arg Ile Thr Ala Gly Ala
            675             680             685

Asp Ala Leu Leu Met Pro Ser Arg Phe Glu Pro Cys Gly Leu Asn Gln
    690             695             700

Leu Tyr Ala Met Ala Tyr Gly Thr Val Pro Val Val His Ala Val Gly
    705             710             715             720

Gly Leu Arg Asp Thr Val Pro Pro Phe Asp Pro Phe Asn His Ser Gly
            725             730             735

Leu Gly Trp Thr Phe Asp Arg Ala Glu Ala His Lys Leu Ile Glu Ala
            740             745             750

Leu Gly His Cys Leu Arg Thr Tyr Arg Asp Phe Lys Glu Ser Trp Arg
            755             760             765

Ala Leu Gln Glu Arg Gly Met Ser Gln Asp Phe Ser Trp Glu His Ala
    770             775             780

Ala Lys Leu Tyr Glu Asp Val Leu Val Lys Ala Lys Tyr Gln Trp
    785             790             795
```